# EUROPEAN PATENT APPLICATION

(11) **EP 4 375 281 A1**
(43) Date of publication of application: **29.05.2024**
(21) Application number: 22845193.6
(22) Date of filing: 12.07.2022
(51) Int. Cl.: C07D 413/04, C07D 491/00, C07D 405/04, C07D 493/00, C07D 307/79, C07D 401/04, A61K 31/421, A61K 31/445, A61P 35/00

(54) **HETEROARYL-3-PIPERIDINEDIONE COMPOUND AND USE THEREOF**

(30) Priority: 19.07.2021 CN 202110815503; 03.11.2021 CN 202111296262
(71) Applicant: Medshine Discovery Inc., Nanjing, Jiangsu 210032 (CN)
(72) Inventor: LEI, Maoyi, Shanghai 200131 (CN); XU, Yu, Shanghai 200131 (CN); LUO, Yunfu, Shanghai 200131 (CN); CHEN, Shuhui, Shanghai 200131 (CN)
(74) Representative: Cabinet Becker et Associés
(86) International application number: PCT/CN2022/105236
(87) International publication number: WO 2023/001028

(57) **Abstract**

A heteroaryl-3-piperidinedione compound and the use thereof. Particularly disclosed are a compound represented by formula (II) and a pharmaceutically acceptable salt thereof.

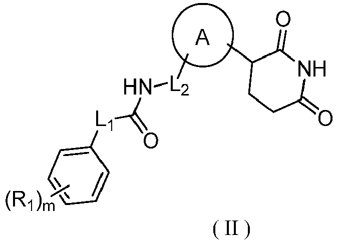

## Description

### This application claims the priority of:

CN202110815503.4, filed on July 19, 2021;
CN202111296262.3, filed on November 03, 2021.

### TECHNICAL FIELD

The present disclosure relates to heteroaryl-3-piperidinedione compounds and use thereof, specifically to a compound represented by formula (II) and a pharmaceutically acceptable salt thereof.

### BACKGROUND

By using the insert of a glutarimide ring structure into the pocket of the CRBN ubiquitin ligase, immunomodulatory drugs (IMiDs) including thalidomide, lenalidomide (Lena), and pomalidomide (Poma) recruit transcription factors Ikaros (IKZF1)/Aiolos (IKZF3) on which B cell-derived cancer cells depend for survival, and promote their degradation by ubiquitination, resulting in cytotoxic effects. In addition to mediating the degradation of IKZF1/3 by ubiquitination, lenalidomide is also capable of mediating the degradation of CKlalpha by targeting CRBN, so as to treat myelodysplastic syndrome with 5q deletion, and CC-90009 is capable of mediating the degradation of GSPT1 (G1 to S Phase Transition 1) by targeting CRBN, so as to treat acute myeloid leukemia.

As an important target of anti-tumor and immunomodulatory drugs, CRBN has been proven to have clear therapeutic effects on multiple myeloma, multiple hematological malignancies such as chronic lymphocytic leukemia, skin diseases such as leprosy and erythema nodosum, and autoimmune diseases such as systemic lupus erythematosus. "Domide" drugs all have many side effects. Currently, there is an urgent need to develop novel CRBN modulator drugs to improve clinical treatment effects.

### SUMMARY

The present disclosure provides a compound represented by formula (II) or a pharmaceutically acceptable salt thereof, 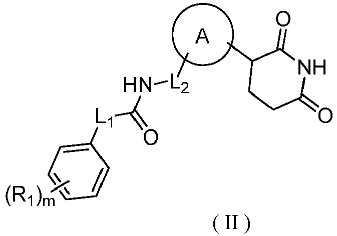 wherein
Ring A is selected from benzofuranyl, benzoisoxazolyl, benzotriazolyl, naphthofuranyl, naphthoisoxazolyl and naphthotriazolyl;
L₁ is selected from a bond, -C(Rₐ)(R_{b})-, -N(R_{c})- and -OCH₂-;
L₂ is selected from -CH₂-, -C₁₋₆ alkyl-C(=O)NH- and -C₁₋₃ alkyl-O-;
each R₁ is independently selected from F, Cl, Br, I, C₁₋₃ alkyl, C₁₋₃ alkoxy, 5-membered heteroaryl and 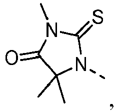 wherein the C₁₋₃ alkyl, C₁₋₃ alkoxy, 5-membered heteroaryl and 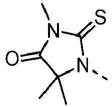 are optionally substituted by 1, 2 or 3 Ra;
m is selected from 0, 1, 2 and 3;
Rₐ and R_{b} are each independently selected from H, F, Cl, Br and I;
R_{c} is selected from H and CH₃;
each R_{d} is independently selected from F, Cl, Br, I, OCH₃, N(CH₃)₂ and morpholinyl.

In some embodiments of the present disclosure, the Ring A is selected from benzofuranyl, benzoisoxazolyl, benzotriazolyl, naphtho[2,3-*b*]furyl, naphtho[2,1-*b*]furyl, naphtho[2,3-*d*]isoxazolyl, naphtho[1,2-*d*]isoxazolyl and 1*H*-naphtho[2,3-*d*][1,2,3]triazolyl.

In some embodiments of the present disclosure, the structural moiety 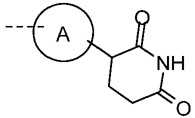 is selected from 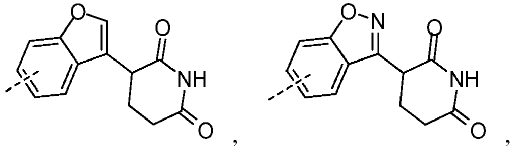 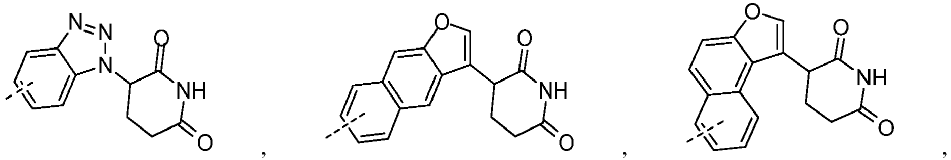 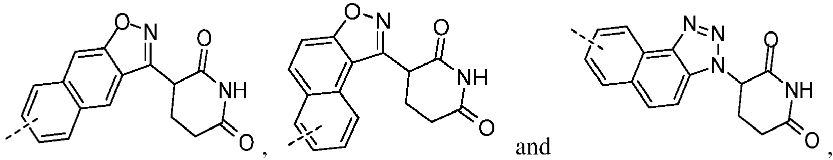 and other variables are as defined in the present disclosure.

In some embodiments of the present disclosure, the structural moiety 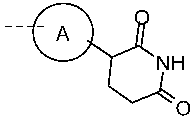 is selected from 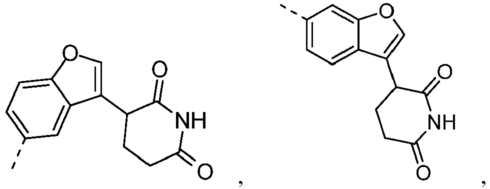 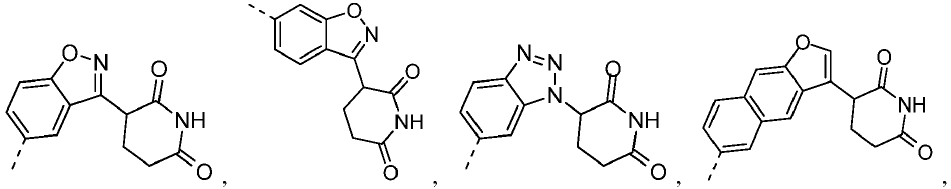 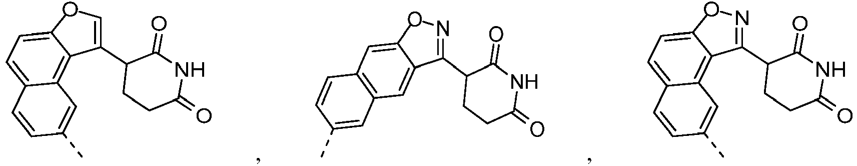 and 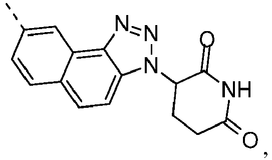 and other variables are as defined in the present disclosure.

In some embodiments of the present disclosure, the Ring A is selected from benzofuranyl and benzoisoxazolyl, and other variables are as defined in the present disclosure.

In some embodiments of the present disclosure, the Ring A is selected from 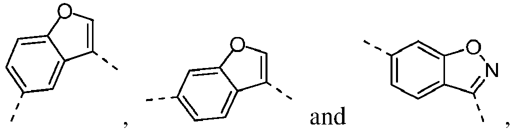 and other variables are as defined in the present disclosure.

In some embodiments of the present disclosure, the structural moiety 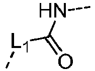 is selected from 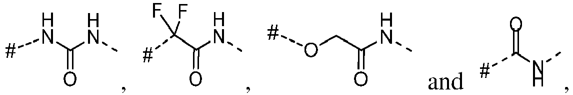 wherein the # end is connected to the phenyl group of formula (II), and other variables are as defined in the present disclosure.

In some embodiments of the present disclosure, the L₂ is selected from -CH₂-, -(CH₂)₆-C(=O)NH- and -CH₂CH₂O-, and other variables are as defined in the present disclosure.

In some embodiments of the present disclosure, the each R₁ is independently selected from F, Cl, Br, I, CH₃, OCH₃, OCH₂CH₃, thiazolyl and 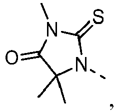 wherein the CH₃, OCH₃, OCH₂CH₃, thiazolyl and 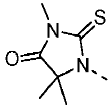 are optionally substituted by 1, 2 or 3 R_{d}, and other variables are as defined in the present disclosure.

In some embodiments of the present disclosure, the each R₁ is independently selected from F, Cl, CH₃, -OCH₂CH₂OCH₃, -OCH₂CH₂N(CH₃)₂, 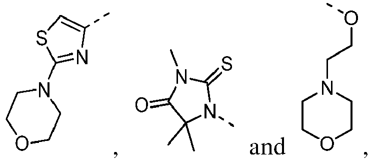 and other variables are as defined in the present disclosure.

In some embodiments of the present disclosure, the structural moiety 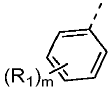 is selected from 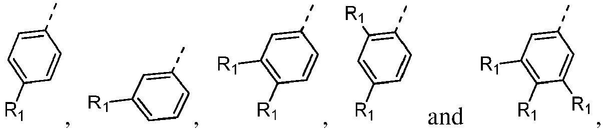 and other variables are as defined in the present disclosure.

In some embodiments of the present disclosure, the structural moiety 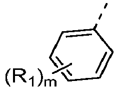 is selected from 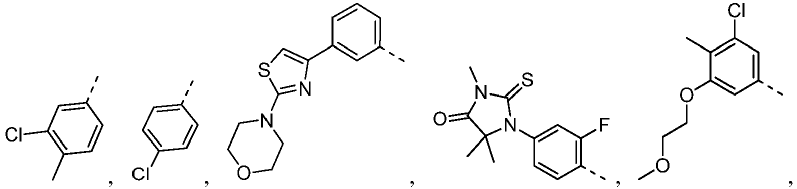 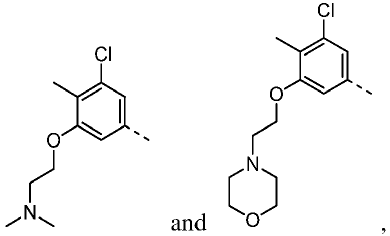 and other variables are as defined in the present disclosure.

The present disclosure provides a compound represented by formula (II) or a pharmaceutically acceptable salt thereof, 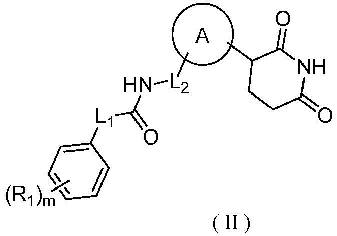 wherein
Ring A is selected from benzofuranyl, benzoisoxazolyl, benzotriazolyl, naphthofuranyl, naphthoisoxazolyl and naphthotriazolyl;
L₁ is selected from -C(Rₐ)(R_{b})-, -N(R_{c})- and -OCH₂-;
L₂ is selected from -CH₂- and -C₁₋₆ alkyl-C(=O)NH-;
each R₁ is independently selected from H, F, Cl, Br, I, C₁₋₃ alkyl, C₁₋₃ alkoxy and 5-membered heteroaryl, wherein the C₁₋₃ alkyl, C₁₋₃ alkoxy and 5-membered heteroaryl are optionally substituted by 1, 2 or 3 Ra;
m is selected from 0, 1, 2 and 3;
Rₐ, R_{b} and R_{c} are each independently selected from H, F, Cl, Br and I;
R_{d} is selected from F, Cl, Br, I and morpholinyl.

In some embodiments of the present disclosure, the Ring A is selected from benzofuranyl, benzoisoxazolyl, benzotriazolyl, naphtho[2,3-*b*]furyl, naphtho[2,1-*b*]furyl, naphtho[2,3-*d*]isoxazolyl, naphtho[1,2-*d*]isoxazolyl and 1*H*-naphtho[2,3-*d*][1,2,3]triazolyl.

In some embodiments of the present disclosure, the structural moiety 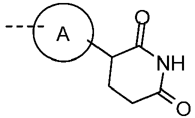 is selected from 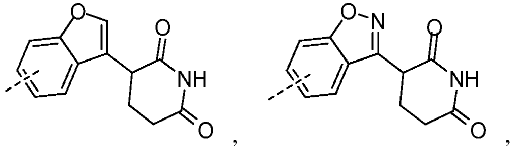 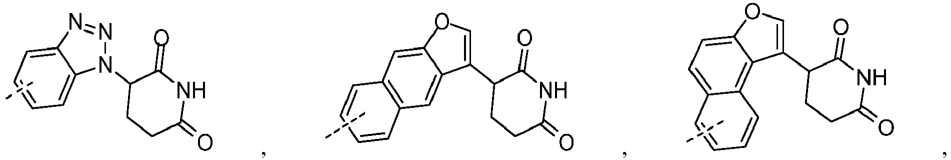 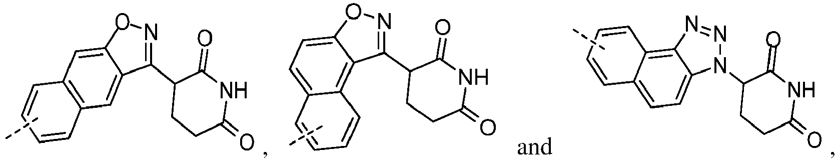 and other variables are as defined in the present disclosure.

In some embodiments of the present disclosure, the structural moiety 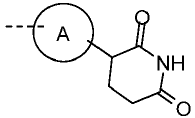 is selected from 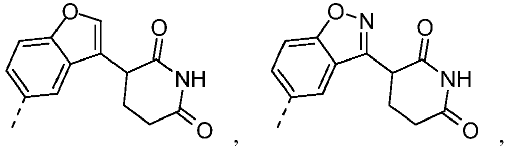 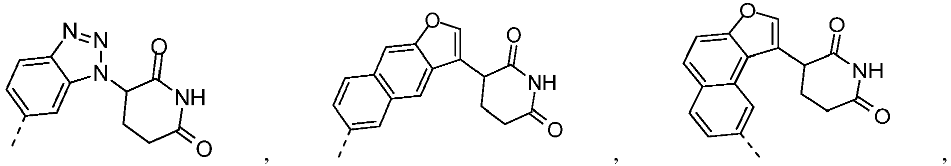 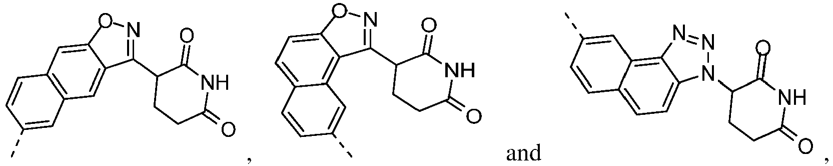 and other variables are as defined in the present disclosure.

In some embodiments of the present disclosure, the structural moiety 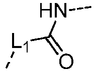 is selected from 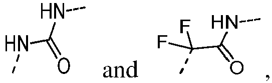 and other variables are as defined in the present disclosure.

In some embodiments of the present disclosure, the L₂ is selected from -CH₂- and - (CH₂)₆-C(=O)NH-, and other variables are as defined in the present disclosure.

In some embodiments of the present disclosure, the each R₁ is independently selected from H, F, Cl, Br, I, CH₃ and thiazolyl, wherein the CH₃ and thiazolyl are optionally substituted by 1, 2 or 3 R_{d}, and other variables are as defined in the present disclosure.

In some embodiments of the present disclosure, the each R₁ is independently selected from H, F, Cl, CH₃ and 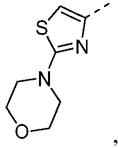 and other variables are as defined in the present disclosure.

In some embodiments of the present disclosure, the structural moiety 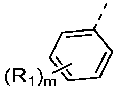 is selected from 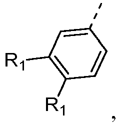 and other variables are as defined in the present disclosure.

In some embodiments of the present disclosure, the structural moiety 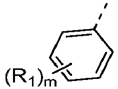 is selected from 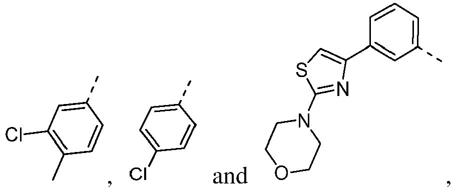 and other variables are as defined in the present disclosure.

In some embodiments of the present disclosure, a compound or a pharmaceutically acceptable salt thereof is disclosed, wherein the compound is selected from: 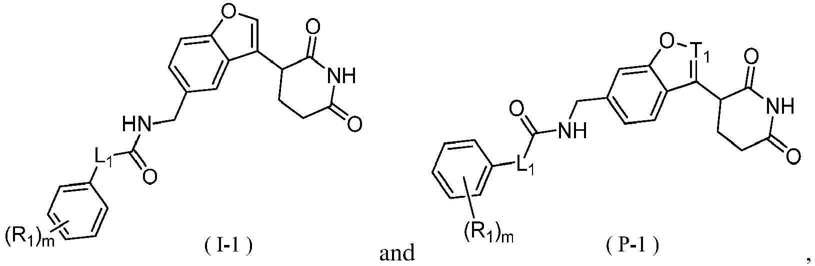 wherein
T₁ is selected from CH and N;
R₁, L₁ and m are as defined in the present disclosure.

The present disclosure also provides a compound represented by the following formula or a pharmaceutically acceptable salt thereof,

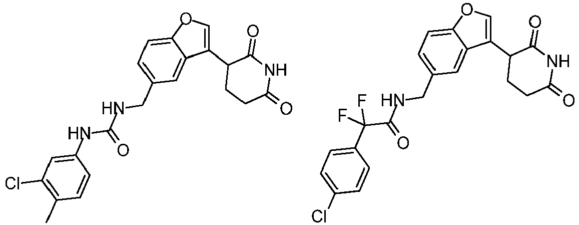

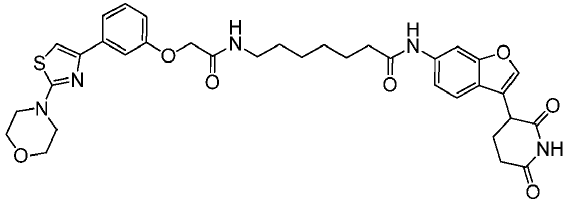

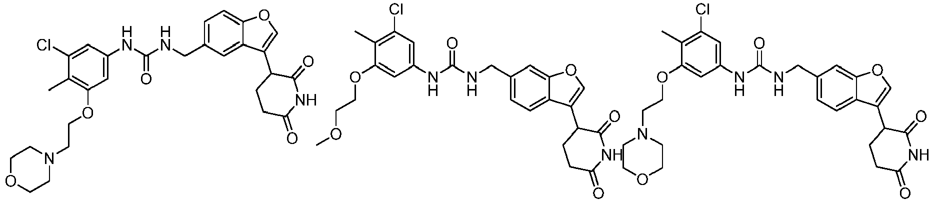

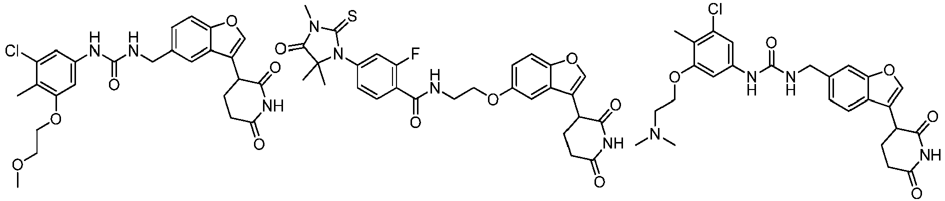

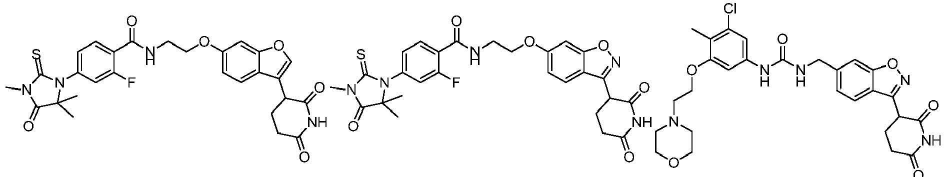

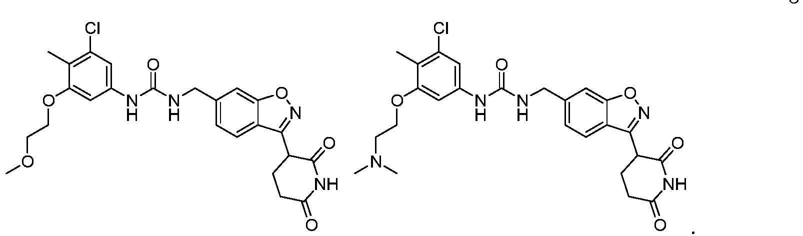

In some embodiments of the present disclosure, a compound or a pharmaceutically acceptable salt thereof is disclosed, wherein the compound is selected from:

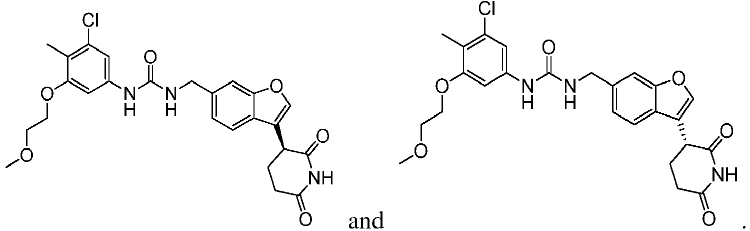

### Definition and term

Unless otherwise specified, the following terms and phrases used herein are intended to have the following meanings. A specific term or phrase should not be considered indefinite or unclear in the absence of a particular definition, but should be understood in the conventional sense. When a trade name appears herein, it is intended to refer to its corresponding commodity or active ingredient thereof.

The term "pharmaceutically acceptable" is used herein in terms of those compounds, materials, compositions, and/or dosage forms, which are suitable for use in contact with human and animal tissues within the scope of reliable medical judgment, with no excessive toxicity, irritation, allergic reaction or other problems or complications, commensurate with a reasonable benefit/risk ratio.

The term "pharmaceutically acceptable salt" refers to a salt of the compound disclosed herein that is prepared by reacting the compound having a specific substituent disclosed herein with a relatively non-toxic acid or base. When the compound disclosed herein contains a relatively acidic functional group, a base addition salt can be obtained by bringing the neutral form of the compound into contact with a sufficient amount of base in a pure solution or a suitable inert solvent. The pharmaceutically acceptable base addition salt includes a salt of sodium, potassium, calcium, ammonium, organic amine or magnesium or similar salts. When the compound disclosed herein contains a relatively basic functional group, an acid addition salt can be obtained by bringing the neutral form of the compound into contact with a sufficient amount of acid in a pure solution or a suitable inert solvent. Certain specific compounds disclosed herein contain both basic and acidic functional groups and can be converted to any base or acid addition salt.

The pharmaceutically acceptable salt disclosed herein can be prepared from the parent compound that contains an acidic or basic moiety by conventional chemical methods. Generally, such salt can be prepared by reacting the free acid or base form of the compound with a stoichiometric amount of an appropriate base or acid in water or an organic solvent or a mixture thereof.

Compounds disclosed herein may be present in a specific geometric or stereoisomeric form. The present disclosure contemplates all such compounds, including cis and trans isomers, (-)- and (+)-enantiomers, (*R*)- and (*S*)-enantiomers, diastereoisomer, (*D*)-isomer, (*L*)-isomer, and a racemic mixture and other mixtures, for example, a mixture enriched in enantiomer or diastereoisomer, all of which are encompassed within the scope disclosed herein. The substituent such as alkyl may have an additional asymmetric carbon atom. All these isomers and mixtures thereof are encompassed within the scope disclosed herein.

Unless otherwise specified, the term "enantiomer" or "optical isomer" means stereoisomers that are in a mirrored relationship with each other.

Unless otherwise specified, the term "cis-trans isomer" or "geometric isomer" is produced by the inability of a double bond or a single bond between ring-forming carbon atoms to rotate freely.

Unless otherwise specified, the term "diastereomer" means a stereoisomer in which two or more chiral centers of are contained in a molecule and is in a non-mirrored relationship between molecules.

Unless otherwise specified, "(*D*)" or "(+)" means dextroisomer, "(*L*)" or "(-)" means levoisomer, and "(*DL*)" or "(±)" means racemate.

Unless otherwise specified, a wedged solid bond ( 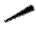 ) and a wedged dashed bond ( 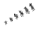 ) indicate the absolute configuration of a stereocenter; a straight solid bond ( 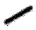 ) and a straight dashed bond ( 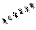 ) indicate the relative configuration of a stereocenter; a wavy line ( 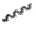 ) indicates a wedged solid bond ( 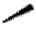 ) or a wedged dashed bond ( 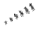 ); or a wavy line ( 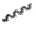 ) indicates a straight solid bond ( 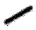 ) and a straight dashed bond ( 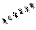 ).

The compounds of the present disclosure may exist in specific tautomers. Unless otherwise specified, the term "tautomer" or "tautomeric form" means that different functional groups in an isomer are in dynamic equilibrium and can be rapidly converted into each other at room temperature. If tautomers are possible (as in solution), a chemical equilibrium of tautomers can be achieved. For example, proton tautomers (also known as prototropic tautomers) include interconversions by proton transfer, such as keto-enol isomerization and imine-enamine isomerization. Valence tautomers include interconversions by recombination of some bonding electrons. A specific example of keto-enol tautomerization is interconversion between two tautomers pentane-2,4-dione and 4-hydroxypent-3-en-2-one.

Unless otherwise specified, the term "enriched in one isomer", "isomer enriched", "enriched in one enantiomer" or "enantiomeric enriched" means that the content of one isomer or enantiomer is less than 100%, and the content of the isomer or enantiomer is 60% or more, or 70% or more, or 80% or more, or 90% or more, or 95% or more, or 96% or more, or 97% or more, or 98% or more, or 99% or more, or 99.5% or more, or 99.6% or more, or 99.7% or more, or 99.8% or more, or 99.9% or more.

Unless otherwise specified, the term "isomer excess" or "enantiomeric excess" means the difference between the relative percentages of two isomers or two enantiomers. For example, if one isomer or enantiomer is present in an amount of 90% and the other isomer or enantiomer is present in an amount of 10%, the isomer or enantiomeric excess (ee value) is 80%.

Optically active (*R*)- and (*S*)-isomer, or *D* and *L* isomer can be prepared using chiral synthesis or chiral reagents or other conventional techniques. If one kind of enantiomer of certain compound disclosed herein is to be obtained, the pure desired enantiomer can be obtained by asymmetric synthesis or derivative action of chiral auxiliary followed by separating the resulting diastereomeric mixture and cleaving the auxiliary group. Alternatively, when the molecule contains a basic functional group (such as amino) or an acidic functional group (such as carboxyl), the compound reacts with an appropriate optically active acid or base to form a salt of the diastereomeric isomer which is then subjected to diastereomeric resolution through the conventional method in the art to afford the pure enantiomer. In addition, the enantiomer and the diastereoisomer are generally isolated through chromatography which uses a chiral stationary phase and optionally combines with a chemical derivative method (for example, carbamate generated from amine).

Compounds disclosed herein may contain an unnatural proportion of atomic isotopes at one or more of the atoms that make up the compounds. For example, a compound may be labeled with a radioisotope such as tritium (³H), iodine-125 (¹²⁵I) or C-14(¹⁴C). For another example, hydrogen can be replaced by heavy hydrogen to form a deuterated drug. The bond between deuterium and carbon is stronger than that between ordinary hydrogen and carbon. Compared with undeuterated drugs, deuterated drugs have advantages of reduced toxic side effects, increased drug stability, enhanced efficacy, and prolonged biological half-life of drugs. All changes in the isotopic composition of compounds disclosed herein, regardless of radioactivity, are included within the scope of the present disclosure.

"Optional" or "optionally" means that the subsequent event or condition may occur but not requisite, that the term includes the instance in which the event or condition occurs and the instance in which the event or condition does not occur.

The term "substituted" means one or more than one hydrogen atoms on a specific atom are substituted by a substituent, including deuterium and hydrogen variants, as long as the valence of the specific atom is normal and the substituted compound is stable. When the substituent is oxo (i.e., =O), it means two hydrogen atoms are substituted. Positions on an aromatic ring cannot be substituted by oxo. The term "optionally substituted" means an atom can be substituted by a substituent or not, unless otherwise specified, the species and number of the substituent may be arbitrary so long as being chemically achievable.

When any variable (such as R) occurs in the constitution or structure of the compound more than once, the definition of the variable at each occurrence is independent. Thus, for example, if a group is substituted by 0-2 R, the group can be optionally substituted by up to two R, wherein the definition of R at each occurrence is independent. Moreover, a combination of the substituent and/or the variant thereof is allowed only when the combination results in a stable compound.

When the number of a linking group is 0, such as -(CRR)₀-, it means that the linking group is a single bond.

When one of variables is a single bond, it means that the two groups linked by the single bond are connected directly. For example, when L in A-L-Z represents a single bond, the structure of A-L-Z is actually A-Z.

When a substituent is vacant, it means that the substituent does not exist. For example, when X is vacant in A-X, the structure of A-X is actually A. When an enumerated substituent does not indicate through which atom it is linked to the substituted group, such substituent can be bonded through any of its atoms. For example, a pyridyl group as a substituent may be linked to the substituted group through any one of carbon atoms on the pyridine ring.

When an enumerated linking group does not indicate its linking direction, its linking direction is arbitrary. For example, when the linking group L in 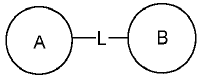 is -M-W-, the -M-W- can be linked to the ring A and the ring B in the same direction as the reading order from left to right to constitute 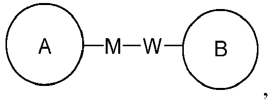 or can be linked to the ring A and the ring B in the reverse direction as the reading order from left to right to constitute 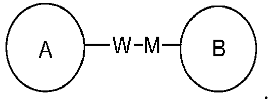 A combination of the linking groups, substituents and/or variants thereof is allowed only when such combination can result in a stable compound.

Unless otherwise specified, when a group has one or more connectable sites, any one or more sites of the group can be connected to other groups through chemical bonds. Where the connection position of the chemical bond is variable, and there is H atom(s) at a connectable site(s), when the connectable site(s) having H atom(s) is connected to the chemical bond, the number of H atom(s) at this site will correspondingly decrease as the number of the connected chemical bond increases, and the group will become a group of corresponding valence. The chemical bond between the site and other groups can be represented by a straight solid bond ( 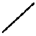 ), a straight dashed bond ( 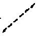 ), or a wavy line 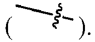 For example, the straight solid bond in -OCH₃ indicates that the group is connected to other groups through the oxygen atom in the group; the straight dashed bond in 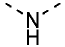 indicates that the group is connected to other groups through two ends of the nitrogen atom in the group; the wavy line in 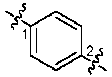 indicates that the group is connected to other groups through the 1- and 2-carbon atoms in the phenyl group; 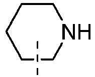 indicates that any connectable site on the piperidinyl group can be connected to other groups through one chemical bond, including at least four connection ways, 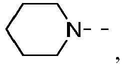 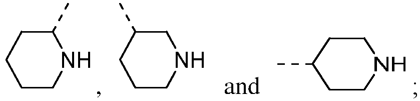 even if a H atom is drawn on -N-, 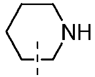 still includes the connection way of 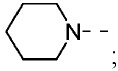 it's just that when one chemical bond is connected, the H at this site will be reduced by one, and the group will become the corresponding monovalent piperidinyl group.

Unless otherwise specified, Cₙ₋ₙ₊ₘ or Cₙ-Cₙ₊ₘ includes any specific case of n to n+m carbons, for example, C₁₋₁₂ includes C₁, C₂, C₃, C₄, C₅, C₆, C₇, C₈, C₉, C₁₀, C₁₁ and C₁₂, also includes any range from n to n+m, for example, C₁₋₁₂ includes C₁₋₃, C₁₋₆, C₁₋₉, C₃₋₆, C₃₋₉, C₃₋₁₂, C₆₋₉, C₆₋₁₂ and C₉₋₁₂, etc.; similarly, n membered to n+m membered indicates that the number of atoms on a ring is n to n+m, for example, 3-12 membered ring includes 3 membered ring, 4 membered ring, 5 membered ring, 6 membered ring, 7 membered ring, 8 membered ring, 9 membered ring, 10 membered ring, 11 membered ring, and 12 membered ring, also includes any range from n to n+m, for example, 3-12 membered ring includes 3-6 membered ring, 3-9 membered ring, 5-6 membered ring, 5-7 membered ring, 6-7 membered ring, 6-8 membered ring, 6-10 membered ring, and the like.

Unless otherwise specified, the term "C₁₋₆ alkyl" is used to indicate a linear or branched saturated hydrocarbon group consisting of 1 to 6 carbon atoms. The C₁₋₆ alkyl group includes C₁₋₅, C₁₋₄, C₁₋₃, C₁₋₂, C₂₋₆, C₂₋₄, C₆, and C₅ alkyl groups, and the like. It may be monovalent (e.g., methyl), divalent (e.g., methylene) or multivalent (e.g., methenyl). Examples of C₂₋₆ alkyl groups include, but are not limited to, methyl (Me), ethyl (Et), propyl (including n-propyl and isopropyl), butyl (including n-butyl, isobutyl, s-butyl and t-butyl), pentyl (including n-pentyl, isopentyl and neopentyl), hexyl, and the like.

Unless otherwise specified, the term "C₁₋₃ alkyl" is used to indicate a linear or branched saturated hydrocarbon group consisting of 1 to 3 carbon atoms. The C₁₋₃ alkyl group includes C₁₋₂ and C₂₋₃ alkyl groups and the like. It may be monovalent (e.g., methyl), divalent (e.g., methylene) or multivalent (e.g., methenyl). Examples of C₁₋₃ alkyl groups include, but are not limited to, methyl (Me), ethyl (Et), propyl (including n-propyl and isopropyl), and the like.

Unless otherwise specified, the term "C₁₋₃ alkoxy" refers to an alkyl group containing 1 to 3 carbon atoms and attached to the remainder of a molecule by an oxygen atom. The C₁₋₃ alkoxy group includes C₁₋₂, C₂₋₃, C₃ and C₂ alkoxy groups, and the like. Examples of C₁₋₃ alkoxy groups include, but are not limited to, methoxy, ethoxy, propoxy (including n-propoxy and isopropoxy), and the like.

Unless otherwise specified, the terms "5-membered heteroaromatic ring" and "5-membered heteroaryl" may be used interchangeably. The term "5-membered heteroaryl" means a monocyclic group having a conjugated pi electron system and composed of 5 ring atoms, in which 1, 2, 3 or 4 ring atoms are heteroatoms independently selected from O, S and N, and the remainder is carbon atoms, wherein the nitrogen atom is optionally quaternized and the nitrogen and sulfur heteroatoms may be optionally oxidized (i.e., NO and S(O)_{b}, p is 1 or 2). A 5- to 6-membered heteroaryl can be attached to the remainder of a molecule through a heteroatom or a carbon atom. Examples of 5-membered heteroaryl include, but are not limited to, pyrrolyl (including N-pyrrolyl, 2-pyrrolyl, 3-pyrrolyl, and the like), pyrazolyl (including 2-pyrazolyl and 3-pyrazolyl, and the like), imidazolyl (including N-imidazolyl, 2-imidazolyl, 4-imidazolyl, and 5-imidazolyl, and the like), oxazolyl (including 2-oxazolyl, 4-oxazolyl, and 5-oxazolyl, and the like), triazolyl (1H-1,2,3-triazolyl, 2H-1,2,3-triazolyl, 1H-1,2,4-triazolyl and 4H-1,2,4-triazolyl, and the like), tetrazolyl, isoxazolyl (3-isoxazolyl, 4-isoxazolyl and 5-isoxazolyl, and the like), thiazolyl (including 2-thiazolyl, 4-thiazolyl and 5-thiazolyl, and the like), furyl (including 2-furyl and 3-furyl, and the like), thienyl (including 2-thienyl and 3-thienyl, and the like).

Compounds disclosed herein can be prepared by a variety of synthetic methods well known to those skilled in the art, including the following enumerated embodiment, the embodiment formed by the following enumerated embodiment in combination with other chemical synthesis methods, and equivalents well known to those skilled in the art. Alternative embodiments include, but are not limited to examples disclosed herein.

Solvents used in the present disclosure are commercially available.

The structures of compounds disclosed herein can be confirmed by conventional methods well known to those skilled in the art. If the present disclosure relates to an absolute configuration of a compound, the absolute configuration can be confirmed by conventional techniques in the art, such as single crystal X-Ray diffraction (SXRD). In the single crystal X-Ray diffraction (SXRD), the diffraction intensity data of the cultivated single crystal is collected using a Bruker D8 venture diffractometer with a light source of CuKα radiation in a scanning mode of ϕ/ω scan; after collecting the relevant data, the crystal structure is further analyzed by the direct method (Shelxs97) to confirm the absolute configuration.

Compounds are named according to general naming principles in the art or by ChemDraw^{®} software, and commercially available compounds are named with their vendor directory names.

### BRIEF DESCRIPTION OF DRAWINGS

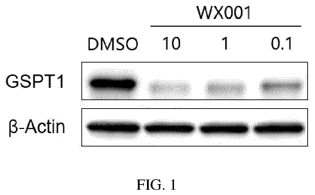

FIG. 1. Western blot diagram of degradation of GSPT1 protein.

### Technical effect

The compounds of the present disclosure have excellent degradation effects on GSPT1 protein, can effectively inhibit tumor cell proliferation, and have significant tumor shrinkage effects. In addition, the compounds of the present disclosure have excellent pharmacokinetic properties and have high plasma system exposure.

### DETAILED DESCRIPTION

The present disclosure is described in detail below by way of examples, but the examples are not intended to impose any unfavorable limitation on the present disclosure. The compounds of the present disclosure can be prepared by a variety of synthetic methods well known to those skilled in the art, including the specific embodiment listed below, embodiments formed by the specific embodiment listed below in combination with other chemical synthetic methods, and equivalents well known to those skilled in the art. Alternative embodiments include, but are not limited to, the examples of the present disclosure. It will be apparent to those skilled in the art that various changes and modifications can be made to the specific embodiments of the present disclosure without departing from the spirit and scope of the present disclosure.

### Example 1

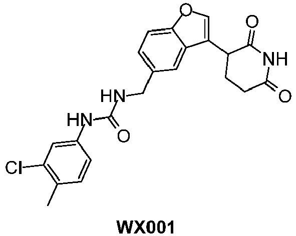

### Synthetic route:

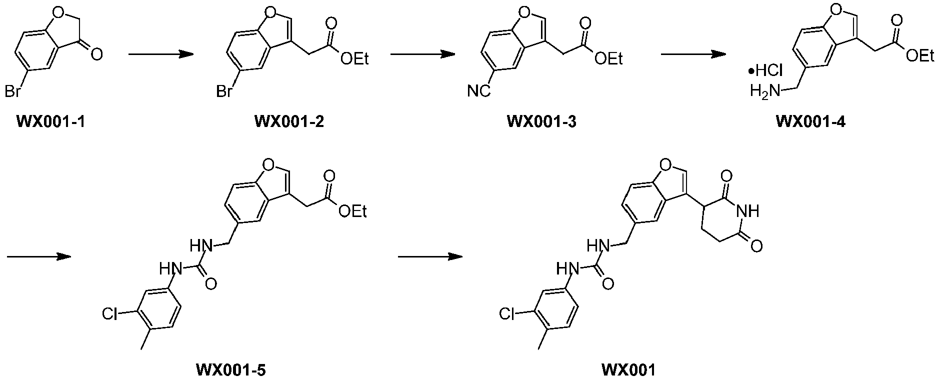

### Step 1: Synthesis of intermediate WX001-2

**WX001-1** (1 g, 4.69 mmol) was dissolved in toluene (50 mL) at room temperature under nitrogen. Then (ethoxycarbonylmethylene)triphenylphosphorane (1.96 g, 5.63 mmol) was added, and the reaction mixture was heated to 130 °C and stirred at 130 °C for 48 hours. After the reaction was completed, the mixture was cooled to room temperature and concentrated under reduced pressure to remove the solvent. The resulting residue was separated by column chromatography (eluent: petroleum ether/ethyl acetate = 20/1 - 10/1, volume ratio) to give intermediate **WX001-2.** MS-ESI *m*/*z*: 282.9 [M+H]⁺, 284.9 [M+2+H]⁺. ¹H NMR (400 MHz, CDCl₃) δ: 7.73 (d, *J*=1.6 Hz, 1H), 7.65 (s, 1H), 7.45-7.41 (m, 1H), 7.39-7.34 (m, 1H), 4.23 (q, *J*=6.8 Hz, 2H), 3.68 (s, 2H), 1.31 (t, *J*=7.0 Hz, 3H).

### Step 2: Synthesis of intermediate WX001-3

Intermediate **WX001-2** (1 g, 3.53 mmol) and zinc cyanide (497.71 mg, 4.24 mmol) were dissolved in N,N-dimethylformamide (5 mL) at room temperature under nitrogen, and then tris(dibenzylideneacetone)dipalladium (161.72 mg, 176.61 µmol) and 2-(dicyclohexylphosphino)-2,4,6-triisopropylbiphenyl (168.38 mg, 353.21 µmol) were added. The reaction mixture was heated to 80 °C and stirred to react at 80 °C for 12 hours. After the reaction was completed, the mixture was cooled to room temperature. Water (100 mL) was added to the reaction solution, and the mixture was extracted with ethyl acetate (50 mL×3). The organic phases were combined, washed with saturated brine (50 mL × 2), dried over anhydrous sodium sulfate, and filtered. The filtrate was concentrated under reduced pressure to remove the solvent. The resulting residue was separated by column chromatography (eluent: petroleum ether/ethyl acetate = 10/1 - 6/1, volume ratio) to give intermediate **WX001-3.** MS-ESI *m*/*z*: 229.9 [M+H]⁺. ¹H NMR (400 MHz, CDCl₃) δ: 7.87 (s, 1H), 7.68 (s, 1H), 7.55-7.45 (m, 2H), 4.14 (q, *J*=7.0 Hz, 2H), 3.64 (s, 2H), 1.22 (t, *J*=7.2 Hz, 3H).

### Step 3: Synthesis of intermediate WX001-4

Intermediate **WX001-3** (520 mg, 2.27 mmol) was dissolved in ethanol (20 mL) at room temperature under nitrogen, and then Raney nickel (971.75 mg, 2.27 mmol, purity: 20%) and hydrochloric acid (4 M, 1.70 mL) were added. The atmosphere was evacuated and replaced with hydrogen several times, and the reaction mixture was stirred at room temperature under hydrogen (15 psi) for 12 hours. After the reaction was completed, the mixture was filtered, and the filtrate was concentrated under reduced pressure to remove the solvent to give intermediate **WX001-4,** which was directly used in the next reaction. ¹H NMR (400 MHz, D₂O) δ: 7.65 (s, 1H), 7.54 (s, 1H), 7.49 (d, *J*=8.4 Hz, 1H), 7.30 (dd, *J*=1.2, 8.8 Hz, 1H), 4.15 (s, 2H), 4.09 (q, *J*=7.2 Hz, 2H), 3.74 (s, 2H), 1.13 (t, *J*=7.0 Hz, 3H).

### Step 4: Synthesis of intermediate WX001-5

Intermediate **WX001-4** (250 mg, 926.88 µmol) was dissolved in tetrahydrofuran (5 mL) at 0 °C under nitrogen, then triethylamine (468.95 mg, 4.63 mmol, 645.05 µL) was added. The reaction mixture was stirred at 0 °C for 0.5 hours. 3-Chloro-4-methylphenyl isocyanate (155.34 mg, 926.88 µmol) was added. The reaction mixture was gradually warmed to room temperature and stirred at room temperature for 2 hours. After the reaction was completed, water (50 mL) was added to the reaction solution, and the mixture was extracted with ethyl acetate (30 mL×3).The organic phases were combined, washed with brine (30 mL × 2), dried over anhydrous sodium sulfate, and filtered. The filtrate was concentrated under reduced pressure to remove the solvent. The resulting residue was separated by column chromatography (eluent: petroleum ether/ethyl acetate = 10/1 - 4/1, volume ratio) to give intermediate **WX001-5.** MS-ESI *m*/*z*: 422.9 [M+Na]⁺, 424.9 [M+2+Na]⁺. ¹H NMR (400 MHz, DMSO_*d₆*) δ: 8.65 (s, 1H), 7.90 (s, 1H), 7.68 (d, *J*=2.0 Hz, 1H), 7.55-7.49 (m, 2H), 7.28 (dd, *J*=1.6, 8.4 Hz, 1H), 7.18 (d, *J*=8.4 Hz, 1H), 7.11 (dd, *J*=2.0, 8.4 Hz, 1H), 6.68 (t, *J*=5.8 Hz, 1H), 4.38 (d, *J*=6.0 Hz, 2H), 4.10 (q, *J*=7.0 Hz, 2H), 3.77 (s, 2H), 2.24 (s, 3H), 1.18 (t, *J*=7.0 Hz, 3H).

### Step 5: Synthesis of compound WX001

Intermediate **WX001-5** (150 mg, 374.20 µmol) was dissolved in N,N-dimethylformamide (10 mL) at 0 °C under nitrogen, and then potassium tert-butoxide (41.99 mg, 374.20 µmol) was added. The reaction mixture was stirred at 0 °C for 1 hour. Acrylamide (26.60 mg, 374.20 µmol) was added. The reaction mixture was gradually warmed to room temperature and stirred at room temperature for 2 hours. After the reaction was completed, water (30 mL) was added, then the mixture was extracted with ethyl acetate (20 mL × 3). The organic phases were combined, washed with saturated brine (20 mL × 2), dried over anhydrous sodium sulfate, and filtered. The filtrate was concentrated under reduced pressure to remove the solvent. The resulting residue was separated by preparative HPLC (mobile phase: acetonitrile/water; acidic system: 0.05% HCl) to give the target compound **WX001.** MS-ESI *m*/*z*: 425.9 [M+H]⁺, 427.9 [M+2+H]⁺. ¹H NMR (400 MHz, DMSO_*d₆*) δ: 10.91 (s, 1H), 8.65 (s, 1H), 7.90 (s, 1H), 7.67 (d, *J*=2.4 Hz, 1H), 7.56-7.52 (m, 2H), 7.28 (dd, *J*=1.6, 8.4 Hz, 1H), 7.18 (d, *J*=8.4 Hz, 1H), 7.11 (dd, *J*=2.0, 8.4 Hz, 1H), 6.67 (t, *J*=5.8 Hz, 1H), 4.37 (d, *J*=6.0 Hz, 2H), 4.14 (dd, *J*=4.8, 12.0 Hz, 1H), 2.83-2.71 (m, 1H), 2.63-2.55 (m, 1H), 2.40-2.31 (m, 1H), 2.23 (s, 3H), 2.17-2.07 (m, 1H).

### Example 2

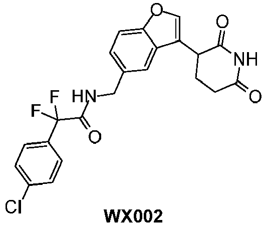

### Synthetic route:

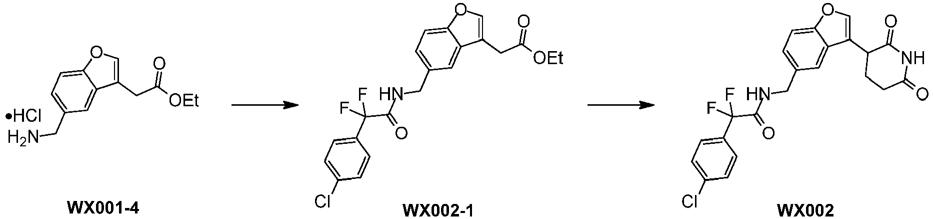

### Step 1: Synthesis of compound WX002-1

Compound **WX001-4** (200 mg, 741.50 µmol), 2-(4-chlorophenyl)-2,2-difluoroacetic acid (229.76 mg, 1.11 mmol) and O-(7-azabenzotriazol-1-yl)-*N,N,N',N'*-tetramethyluronium hexafluorophosphate (563.88 mg, 1.48 mmol) were dissolved in N,N-dimethylformamide (5 mL) at room temperature under nitrogen, then triethylamine (375.16 mg, 3.71 mmol, 516.04 µL) was added. The reaction mixture was stirred at room temperature for 12 hours. After the reaction was completed, water (50 mL) was added to the reaction mixture, and the mixture was extracted with ethyl acetate (30 mL×3). The organic phases were combined, washed with saturated brine (30 mL×2), dried over anhydrous sodium sulfate, and filtered. The solvent was removed from the filtrate under reduced pressure, and the resulting residue was separated by column chromatography (eluent: petroleum ether/ethyl acetate = 3/1, volume ratio) to give compound **WX002-1.** MS-ESI *m*/*z*: 444.0 [M+Na]⁺, 446.0 [M+Na+2]⁺. ¹H NMR (400 MHz, CDCl₃) δ: 7.58 (s, 1H), 7.50 (d, *J*=8.4 Hz, 2H), 7.43-7.33 (m, 4H), 7.14 (dd, *J*=1.8, 8.2 Hz, 1H), 6.72 (s, 1H), 4.52 (d, *J*=5.6 Hz, 2H), 4.12 (q, *J*=7.0 Hz, 2H), 3.60 (s, 2H), 1.21 (t, *J*=7.2 Hz, 3H).

### Step 2: Synthesis of compound WX002

**WX002-1** (0.120 g, 284.48 µmol) was dissolved in N,N-dimethylformamide (10 mL) at 0 °C under nitrogen, and potassium tert-butoxide (31.92 mg, 284.48 µmol) was added. The reaction mixture was stirred at 0 °C for 1 hour, and then acrylamide (20.22 mg, 284.48 µmol) was added. The reaction mixture was warmed to room temperature and stirred at room temperature for 2 hours. After the reaction was completed, water (30 mL) was added to the reaction mixture, and the mixture was extracted with ethyl acetate (20 mL×3).The organic phases were combined, washed with saturated brine (20 mL×2), dried over anhydrous sodium sulfate, and filtered. The solvent was removed from the filtrate under reduced pressure, and the resulting residue was separated by preparative HPLC (mobile phase: acetonitrile/water; acidic system: 0.05% HCl) to give the target compound **WX002.** MS-ESI *m*/*z*: 468.8 [M+Na]⁺, 470.8 [M+Na+2]⁺. ¹H NMR (400 MHz, DMSO_*d*₆) δ: 10.94 (s, 1H), 9.63 (t, *J*=6.0 Hz, 1H), 7.91 (s, 1H), 7.67-7.57 (m, 4H), 7.53 (d, *J*=8.4 Hz, 1H), 7.45 (s, 1H), 7.18 (dd, *J*=1.4, 8.2 Hz, 1H), 4.42 (d, *J*=6.0 Hz, 2H), 4.09 (dd, *J*=5.2, 12.0 Hz, 1H), 2.83-2.72 (m, 1H), 2.68-2.57 (m, 1H), 2.34-2.23 (m, 1H), 2.15-2.06 (m, 1H).

### Example 3

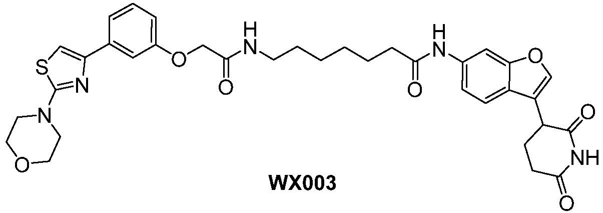

### Synthetic route:

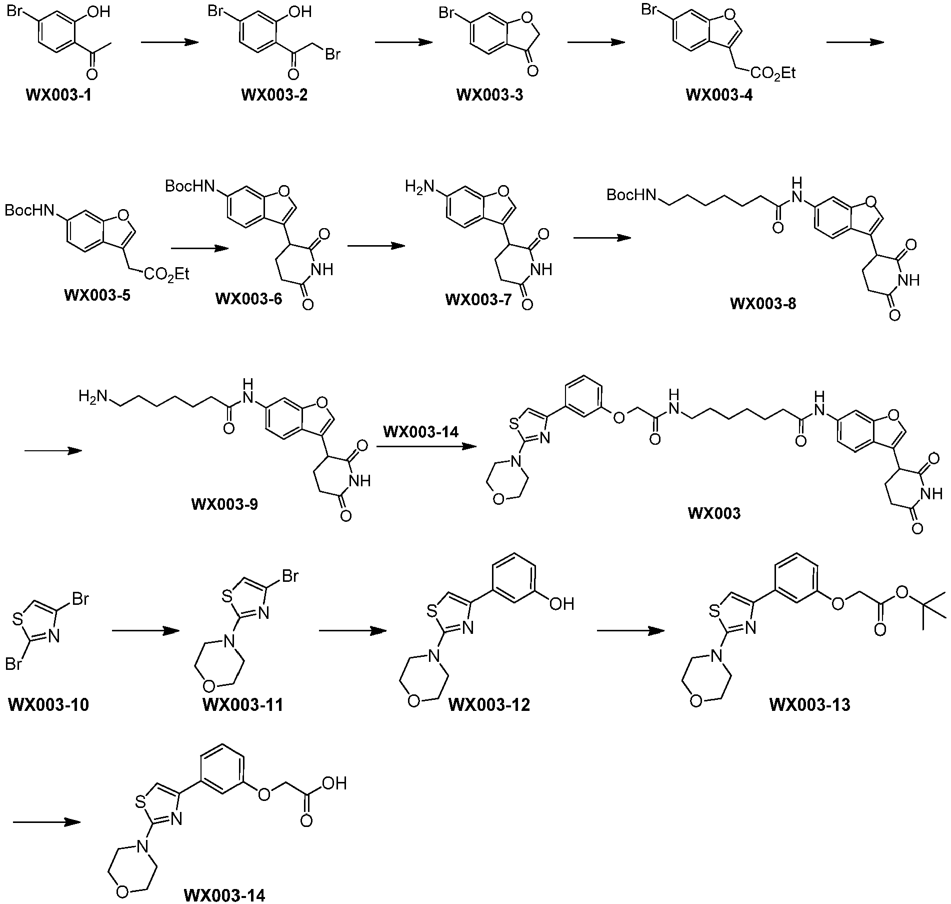

### Step 1: Synthesis of compound WX003-2

Compound **WX003-1** (100 g, 465.02 mmol) was dissolved in a mixed solvent of chloroform (500 mL) and ethyl acetate (500 mL) at room temperature, then copper bromide (207.73 g, 930.04 mmol) was added. The reaction mixture was heated to 90 °C and stirred to react for 20 hours. After the reaction was completed, the reaction solution was cooled to room temperature and filtered. The filter cake was washed with dichloromethane (200 mL × 2). The filtrate was collected to give a solution of compound **WX003-2** in dichloromethane, which was used directly in the next step.

### Step 2: Synthesis of compound WX003-3

A solution of compound **WX003-2** (136.69 g, 465.03 mmol) in dichloromethane (1.4 L) was cooled to 0 °C under nitrogen, and triethylamine (70.58 g, 697.54 mmol, 97.09 mL) was slowly added dropwise. The reaction mixture was allowed to slowly warm to 20 °C and stirred to react for 0.5 hours. After the reaction was completed, water (300 mL) was added to the system, and the layers were separated. The organic phases were collected, washed with saturated brine (1 L), dried over anhydrous sodium sulfate, and filtered. The filtrate was concentrated under reduced pressure to remove about half of the solvent, then toluene (500 mL) was added. The mixture was further concentrated under reduced pressure to remove the remaining low-boiling point solvent (carefully not to be rotary-evaporated to dryness) to give a solution of compound **WX003-3** in toluene, which was used directly in the next step. MS-ESI m/z: 212.9 [M+H]⁺, 215.0 [M+H+2]⁺.

### Step 3: Synthesis of compound WX003-4

To a solution of compound **WX003-3** (99 g, 464.73 mmol) in a mixture of dichloromethane and toluene (1 L) was added (ethoxycarbonylmethylene)triphenylphosphorane (161.90 g, 464.73 mmol) at room temperature under nitrogen. The reaction solution was heated to 130°C and stirred to react for 20 hours. After the reaction was completed, the reaction solution was cooled to room temperature, and concentrated under reduced pressure to give a residue. Methyl tert-butyl ether (800 mL) was added to the residue, and the mixture was stirred for 30 minutes, then filtered. The filter cake was rinsed with methyl tert-butyl ether (100 mL × 2), and the filtrate was concentrated under reduced pressure to give a crude product. The crude product was separated by column chromatography (eluent: petroleum ether:ethyl acetate = 100/1 - 10/1), to give compound **WX003-4.** MS-ESI *m*/*z*: 283.0 [M+H]⁺, 285.0 [M+H+2]⁺. ¹H NMR (400 MHz, CDCl₃) δ: 7.66 (d, *J*=1.2 Hz, 1H), 7.61 (s, 1H), 7.44 (d, *J*=8.4 Hz, 1H), 7.38 (dd, *J*=1.6 Hz, 8.4 Hz, 1H), 4.20 (q, *J*=7.0 Hz, 2H), 3.68 (d, *J*=1.2 Hz, 2H), 1.28 (t, *J*=7.2 Hz, 3H).

### Step 4: Synthesis of compound WX003-5

Compound **WX003-4** (5 g, 17.66 mmol), tert-butyl carbamate (2.48 g, 21.19 mmol), 2-di-tert-butylphosphino-2',4',6-triisopropylbiphenyl (1.05 g, 2.47 mmol), tris(dibenzylideneacetone)dipalladium (1.13 g, 1.24 mmol) and potassium phosphate (15.00 g, 70.64 mmol) were dissolved in toluene (100 mL) and water (20 mL) at room temperature under nitrogen. The reaction mixture was heated to 100°C and stirred to react for 12 hours. After the reaction was completed, the reaction solution was cooled to room temperature. Water (50 mL) was added, and the mixture was extracted with ethyl acetate (100 mL × 3). The organic phases were combined, washed with saturated brine (100 mL), dried over anhydrous sodium sulfate, and filtered. The filtrate was concentrated under reduced pressure to give a crude product. n-Heptane (100 mL) was added to the crude product, and the mixture was slurried, and filtered. The filter cake was washed with n-heptane (50 mL × 3) and the solid was collected to give compound **WX003-5.** ¹H NMR (400 MHz, CDCl₃) δ: 7.77 (s, 1H), 7.57 (s, 1H), 7.44 (d, *J*=8.4 Hz, 1H), 7.05 (dd, *J*=1.6 Hz, 8.4 Hz, 1H), 6.56 (s, 1H), 4.19 (q, *J*=7.2 Hz, 2H), 3.66 (d, *J*=0.8 Hz, 2H), 1.54 (s, 9H), 1.27 (t, *J*=7.2 Hz, 3H).

### Step 5: Synthesis of compound WX003-6

Compound **WX003-5** (4.4 g, 13.78 mmol) was dissolved in N,N-dimethylformamide (50 mL) at room temperature, and potassium tert-butoxide (2.78 g, 24.80 mmol) and acrylamide (1.18 g, 16.53 mmol) were added in sequence. The reaction mixture was stirred to react at room temperature for 2 hours. After the reaction was completed, the reaction solution was poured into saturated aqueous ammonium chloride solution (200 mL), and the mixture was extracted with ethyl acetate (50 mL×2). The organic phases were combined, washed with saturated brine (100 mL), dried over anhydrous sodium sulfate, and filtered. The filtrate was concentrated under reduced pressure to give a crude product. The crude product was separated by column chromatography (eluent: petroleum ether:ethyl acetate=5/1 - 1/2), to give compound**WX003-6.**

### Step 6: Synthesis of compound WX003-7

Compound **WX003-6** (2.8 g, 8.13 mmol) was dissolved in dichloromethane (80 mL) at room temperature, and hydrochloric acid/ethyl acetate (4 M, 200 mL) was added. The reaction mixture was stirred to react at room temperature for 4 hours. After the reaction was completed, the reaction solution was directly concentrated under reduced pressure to remove the solvent. The resulting residue was added to dichloromethane (500 mL). The mixture was adjusted to pH = 8~9 with saturated aqueous sodium bicarbonate solution, and extracted with dichloromethane (500 mL × 3). The organic phases were combined, washed with saturated brine (200 mL), dried over anhydrous sodium sulfate, and filtered. The filtrate was concentrated under reduced pressure to give compound **WX003-7.** MS-ESI *m*/*z*: 245.2 [M+H]⁺. ¹H NMR (400 MHz, DMSO_*d₆*) δ: 10.85 (s, 1H), 7.52 (s, 1H), 7.17 (d, *J*=8.0 Hz, 1H), 6.65 (d, *J*=1.6 Hz, 1H), 6.52 (d, *J*=1.4 Hz, 8.2 Hz, 1H), 5.16 (s, 2H), 3.98 (dd, *J*=4.8 Hz, 11.6 Hz, 1H), 2.75-2.64 (m, 1H), 2.58-2.53 (m, 1H), 2.30-2.18 (m, 1H), 2.13-2.03 (m, 1H).

### Step 7: Synthesis of compound WX003-8

7-(N-tert-butoxycarbonylamino)heptanoic acid (100.44 mg, 409.42 µmol) was dissolved in N,N-dimethylformamide (2 mL) at room temperature under nitrogen, and then 2-(7-azabenzotriazole)-N,N,N',N'-tetramethyluronium hexafluorophosphate (311.35 mg, 818.85 µmol) and N,N-diisopropylethylamine (158.74 mg, 1.23 mmol, 213.94 µL) were added. The mixture was stirred at room temperature for 30 minutes, and compound **WX003-7** (100 mg, 409.42 µmol) was added. The reaction mixture was stirred at room temperature for another 1 hour. After the reaction was completed, water (5 mL) and petroleum ether (3 mL) were added to the reaction solution. The mixture was stirred for 10 minutes, and solids were precipitated. The mixture was filtered, and the filter cake was washed with ethyl acetate (1 mL × 2). The filter cake was collected, and concentrated under reduced pressure to give compound **WX003-8.** MS-ESI *m*/*z*: 372.2 [M-99]⁺.

### Step 8: Synthesis of compound WX003-9 hydrochloride

Compound **WX003-8** (190 mg, 402.93 µmol) was dissolved in hydrochloric acid/ethyl acetate (3.61 mL, 4 M) at room temperature, and the reaction mixture was stirred to react for 0.5 hours. After the reaction was completed, the reaction solution was filtered, and the filter cake was rinsed with ethyl acetate (2 mL × 3). The filter cake was collected to give compound **WX003-9** hydrochloride. MS-ESI *m*/*z*: 372.2 [M+H]⁺. ¹H NMR (400 MHz, DMSO_*d₆*) δ: 10.88 (s, 1H), 10.16 (s, 1H), 8.07 (s, 1H), 7.90 (s, 2H), 7.81 (s, 1H), 7.47 (d, *J*=8.4 Hz, 1H), 7.31 (dd, *J*=1.2, 8.8 Hz, 1H), 4.09 (dd, *J*=4.6 Hz, 11.8 Hz, 1H), 2.81-2.65 (m, 3H), 2.60-2.53 (m, 1H), 2.35 (t, *J*=7.4 Hz, 2H), 2.32-2.23 (m, 1H), 2.15-2.05 (m, 1H), 1.65-1.52 (m, 4H), 1.40-1.26 (m, 4H).

### Step 9: Synthesis of compound WX003-11

Compound **WX003-10** (21.5 g, 88.51 mmol) was dissolved in N,N-dimethylformamide (200 mL) at room temperature under nitrogen, and triethylamine (26.87 g, 265.52 mmol, 36.96 mL) and morpholine (7.71 g, 88.51 mmol, 7.79 mL) were added. The reaction mixture was heated to 80 °C and stirred to react for 12 hours. After the reaction was completed, the reaction solution was cooled to room temperature. 10 % Saline (100 mL) was added, and the mixture was extracted with ethyl acetate (150 mL × 3). The organic phases were combined, washed with saturated brine (200 mL × 2), dried over anhydrous sodium sulfate, and filtered. The filtrate was concentrated under reduced pressure to give a crude product. The crude product was separated by column chromatography (eluent: petroleum ether:ethyl acetate = 1 / 0-1/1) to give compound **WX003-11.** MS-ESI *m*/*z*: 248.9 [M+H]⁺, 250.9 [M+H+2]⁺. ¹H NMR (400 MHz, DMSO_*d₆*) δ: 6.92 (s, 1H), 3.69 (t, *J*=5.0 Hz, 4H), 3.36 (t, *J*=5.0 Hz, 4H).

### Step 10: Synthesis of compound WX003-12

Compound **WX003-11** (8 g, 32.11 mmol) was dissolved in a mixed solvent of 1,4-dioxane (70 mL) and water (14 mL) at room temperature under nitrogen. 3-Hydroxyphenylboronic acid (6.64 g, 48.17 mmol), potassium carbonate (17.75 g, 128.45 mmol) and 1,1'-bisdiphenylphosphinoferrocene palladium dichloride (1.17 g, 1.61 mmol) were added. The reaction mixture was heated to 100 °C and stirred to react for 1 hour. After the reaction was completed, the reaction solution was cooled to room temperature, adjusted to pH of 5~6 with 1 N hydrochloric acid. Water (100 mL) was added, and the mixture was extracted with ethyl acetate (150 mL × 3). The organic phases were combined, washed with saturated brine (150 mL × 2), dried over anhydrous sodium sulfate, and filtered. The filtrate was concentrated under reduced pressure to give a crude product. The crude product was separated by column chromatography (eluent: petroleum ether:ethyl acetate = 1/0-1/1) to give compound **WX003-12.** MS-ESI *m*/*z*: 263.1 [M+H]⁺. ¹H NMR (400 MHz, DMSO_*d₆*) δ: 9.39 (s, 1H), 7.28 (d, *J*=1.6 Hz, 1H), 7.20 (s, 1H), 7.16 (t, *J*=7.6 Hz, 1H), 7.01-6.93 (m, 1H), 6.75-6.65 (m, 1H), 3.73 (t, *J*=4.8 Hz, 4H), 3.42 (t, *J*=4.8 Hz, 4H).

### Step 11: Synthesis of compound WX003-13

Compound **WX003-12** (4.3 g, 16.39 mmol) was dissolved in N,N-dimethylformamide (50 mL) at room temperature under nitrogen, and tert-butyl bromoacetate (6.39 g, 32.78 mmol, 4.84 mL) and potassium carbonate (6.80 g, 49.18 mmol) were added. The reaction solution was heated to 50 °C and stirred to react for 12 hours. After the reaction was completed, the reaction solution was cooled to room temperature. Water (50 mL) was added, and the mixture was extracted with ethyl acetate (50 mL × 2). The organic phases were combined, washed with saturated brine (50 mL), dried over anhydrous sodium sulfate, and filtered. The filtrate was concentrated under reduced pressure to give a residue. Methyl tert-butyl ether (10 mL) was added to the residue, and the mixture was stirred for 10 minutes. The mixture was filtered, and the filter cake was rinsed with methyl tert-butyl ether (3 mL). The filter cake was collected to give compound **WX003-13.** MS-ESI *m*/*z*: 377.1 [M+H]⁺. ¹H NMR (400 MHz, DMSO_*d*₆) δ:7.46 (d, *J*=7.6 Hz, 1H), 7.38 (t, *J*=2.0 Hz, 1H), 7.34 (s, 1H), 7.29 (t, *J*=8.0 Hz, 1H), 6.82 (dd, *J*=2.4 Hz, 8.0 Hz, 1H), 4.68 (s, 2H), 3.73 (t, *J*=4.8 Hz, 4H), 3.44 (t, *J*=5.0 Hz, 4H), 1.43 (s, 9H).

### Step 12: Synthesis of compound WX003-14 hydrochloride

Compound **WX003-13** (3.3 g, 8.77 mmol) was dissolved in hydrochloric acid/ethyl acetate (10 mL, 4 M) at room temperature. The reaction mixture was stirred to react at room temperature for 1 hour. After the reaction was completed, the reaction solution was filtered, and the filter cake was rinsed with ethyl acetate (5 mL × 2). The filter cake was collected and concentrated under reduced pressure to remove the residual solvent to give compound **WX003-14** hydrochloride. MS-ESI *m*/*z*: 321.1 [M+H]⁺.

### Step 13: Synthesis of compound WX003

Compound **WX003-14** hydrochloride (62.99 mg, 176.52 µmol) was dissolved in N,N-dimethylformamide (1 mL) at room temperature under nitrogen, and then O-(7-azabenzotriazol-1-yl)-*N,N,N',N'*-tetramethyluronium hexafluorophosphate (111.86 mg, 294.20 µmol) and N,N-diisopropylethylamine (114.07 mg, 882.59 µmol, 153.73 µL) were added. The mixture was stirred at room temperature for 30 minutes, then compound **WX003-9** hydrochloride (60 mg, 147.10 µmol) was added, and the mixture was stirred at room temperature for another 1 hour. After the reaction was completed, water (10 mL) was added to the reaction solution, and the mixture was extracted with ethyl acetate (20 mL × 4). The organic phases were combined, washed with saturated brine (20 mL × 3), dried over anhydrous sodium sulfate, and filtered. The filtrate was concentrated under reduced pressure to give a residue. The resulting residue was separated by preparative HPLC (mobile phase: acetonitrile / water; acidic system: 0.04% HCl) to give compound **WX003.** MS-ESI *m*/*z*: 674.3 [M+H]⁺. ¹H NMR (400 MHz, DMSO_*d₆*) δ: 10.88 (s, 1H), 10.01 (s, 1H), 8.09 (t, *J*=5.8 Hz, 1H), 8.05 (d, *J*=1.6 Hz, 1H), 7.81 (s, 1H), 7.49-7.43 (m, 3H), 7.33-7.25 (m, 3H), 6.90-6.85 (m, 1H), 4.49 (s, 2H), 4.08 (dd, *J*=5.0 Hz, 11.8 Hz, 1H), 3.73 (t, *J*=5.0 Hz, 4H), 3.43 (t, *J*=4.8 Hz, 4H), 3.12 (q, *J*=6.8 Hz, 2H), 2.78-2.65 (m, 1H), 2.60-2.53 (m, 1H), 2.35-2.25 (m, 3H), 2.15-2.07 (m, 1H), 1.62-1.52 (m, 2H), 1.48-1.40 (m, 2H), 1.32-1.23 (m, 4H).

### Example 4

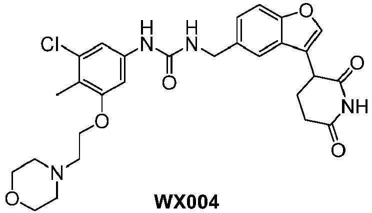

### Synthetic route:

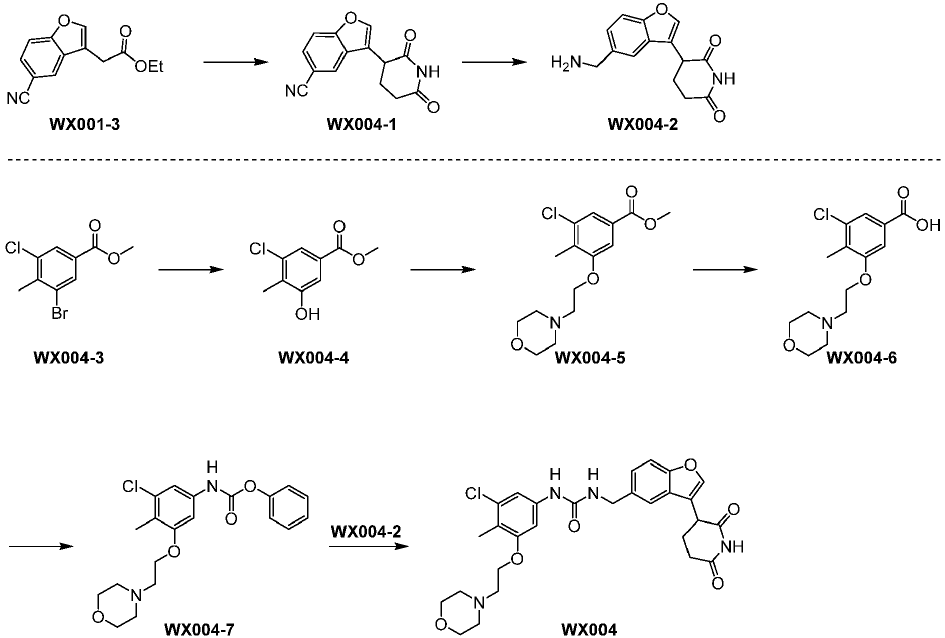

### Step 1: Synthesis of compound WX004-1

Compound **WX001-3** (3 g, 13.09 mmol) was dissolved in N,N-dimethylformamide (45 mL) at room temperature under nitrogen, and the solution was cooled to 0 °C. Acrylamide (930.21 mg, 13.09 mmol) and potassium tert-butoxide (1.47 g, 13.09 mmol) were added simultaneously. The reaction mixture was stirred at 0 °C for 1 hour. After the reaction was completed, the reaction solution was poured into a mixture solution of saturated ammonium chloride (100 mL) and ethyl acetate (50 mL), and the layers were separated. The organic phases were collected, and the aqueous phase was extracted with ethyl acetate (50 mL × 3). The organic phases were combined and washed with semi-saturated brine (50 mL × 3), dried over anhydrous sodium sulfate, and filtered. The filtrate was concentrated under reduced pressure to remove the solvent. The resulting residue was stirred with methanol (10 mL) at room temperature for 20 minutes, and solids were precipitated. The mixture was filtered. The solids were collected, and concentrated under reduced pressure to give compound **WX004-1.** ¹H NMR (400 MHz, DMSO_*d₆*) δ: 10.93 (s, 1H), 8.26 (d, *J*=0.8 Hz, 1H), 8.12 (s, 1H), 7.81 (d, *J*=8.4 Hz, 1H), 7.57 (dd, *J*=1.6, 8.8 Hz, 1H), 4.20 (dd, *J*=4.8, 12.4 Hz, 1H), 2.80-2.69 (m, 1H), 2.63-2.55 (m, 1H), 2.47-2.34 (m, 1H), 2.15-2.05 (m, 1H).

### Step 2: Synthesis of compound WX004-2

Wet palladium on carbon (4 g, purity: 10%) was added to methanol (300 mL) at room temperature, followed by compound **WX004-1** (2.1 g, 8.26 mmol) and concentrated hydrochloric acid (12 M, 6 mL). The atmosphere was replaced with hydrogen three times, and the reaction mixture was stirred at room temperature under hydrogen (15 psi) atmosphere for 12 hours. After the reaction was completed, the reaction solution was directly filtered through diatomaceous earth. The filtrate was collected, and concentrated under reduced pressure to remove the solvent. The resulting residue was separated by preparative HPLC (mobile phase: acetonitrile/water; neutral system: NH₄HCO₃), to give compound **WX004-2.** MS-ESI *m*/*z*: 257.1 [M-H]⁻. ¹H NMR (400 MHz, DMSO_*d₆*) δ: 7.87 (s, 1H), 7.58-7.43 (m, 2H), 7.32-7.20 (m, 1H), 4.21 (d, *J*=6.0 Hz, 1H), 4.11 (dd, *J*=4.8, 10.0 Hz, 1H), 3.82 (s, 1H), 2.81-2.68 (m, 1H), 2.61-2.54 (m, 1H), 2.39-2.26 (m, 1H), 2.17-2.04 (m, 1H).

### Step 3: Synthesis of compound WX004-4

Compound **WX004-3** (1 g, 3.79 mmol) was dissolved in N,N-dimethylformamide (10 mL) at room temperature under nitrogen. Then (E)-benzaldoxime (482.68 mg, 3.98 mmol), di-tert-butyl-(2,4,6-triisopropyl-3,6-dimethoxybiphenyl-2-yl)phosphine (229.92 mg, 474.36 µmol), allylpalladium chloride (II) dimer (69.42 mg, 189.74 µmol) and cesium carbonate (1.85 g, 5.69 mmol) were added. The reaction mixture was heated to 90 °C and stirred to react for 8 hours. After the reaction was completed, the reaction solution was cooled to room temperature. The reaction solution was poured into 0.5 M dilute hydrochloric acid (100 mL), and extracted with ethyl acetate (50 mL × 3). The organic phases were combined, washed with semi-saturated brine (50 mL × 3), dried over anhydrous sodium sulfate, and filtered. The filtrate was concentrated under reduced pressure to remove the solvent. The resulting residue was separated by column chromatography (eluent: petroleum ether/ethyl acetate = 1/0-19/1, volume ratio) to give compound **WX004-4.** MS-ESI *m*/*z*: 199.1 [M-H]⁻, 200.1 [M-H+1]⁻, 201.1 [M-H+2]⁻. ¹H NMR (400 MHz, DMSO_*d₆*) δ: 10.34 (s, 1H), 7.39 (d, *J*=1.6 Hz, 1H), 7.37 (d, *J*=1.6 Hz, 1H), 3.82 (s, 3H), 2.22 (s, 3H).

### Step 4: Synthesis of compound WX004-5

Compound **WX004-4** (700 mg, 3.49 mmol) was dissolved in acetonitrile (20 mL) at room temperature under nitrogen. Then potassium iodide (115.84 mg, 697.84 µmol), potassium carbonate (964.49 mg, 6.98 mmol), 4-(2-chloroethyl)morpholine (574.25 mg, 3.84 mmol) were added. The reaction mixture was heated to 80 °C and stirred to react for 12 hours. After the reaction was completed, the reaction solution was filtered directly, and the filter cake was rinsed with tetrahydrofuran. (20 mL × 3). The filtrate was collected, and concentrated under reduced pressure to remove the solvent. The resulting residue was separated by column chromatography (eluent: petroleum ether/ethyl acetate = 4/1-1/1, volume ratio) to give compound **WX004-5.** MS-ESI *m*/*z*: 314.2 [M+H]⁺, 315.2 [M+H+1]⁺, 316.2 [M+H+2]⁺. ¹H NMR (400 MHz, CDCl₃) δ: 7.68 (d, *J*=0.8 Hz, 1H), 7.40 (d, *J*=0.8 Hz, 1H), 4.18 (t, *J*=5.6 Hz, 2H), 3.91 (s, 3H), 3.74 (t, *J*=4.6 Hz, 4H), 2.86 (t, *J*=5.6 Hz, 2H), 2.61 (t, *J*=4.4 Hz, 4H), 2.32 (s, 3H).

### Step 5: Synthesis of compound WX004-6

Compound **WX004-5** (760 mg, 2.42 mmol) was dissolved in tetrahydrofuran (6 mL) and water (2 mL) at room temperature. Then lithium hydroxide monohydrate (304.92 mg, 7.27 mmol) was added. The reaction mixture was stirred to react at room temperature for 12 hours. After the reaction was completed, water (10 mL) was added to the reaction solution. The mixture was adjusted to pH of 7-8 with 6 M dilute hydrochloric acid, and extracted with 2-methyltetrahydrofuran (50 mL×10). The organic phases were combined, dried over anhydrous sodium sulfate, and filtered. The filtrate was concentrated under reduced pressure to give compound **WX004-6.** ¹H NMR (400 MHz, DMSO_*d₆*) δ: 7.51 (d, *J*=0.8 Hz, 1H), 7.43 (d, *J*=1.2 Hz, 1H), 4.17 (t, *J*=5.4 Hz, 2H), 3.57 (t, *J*=4.6 Hz, 4H), 2.74 (t, *J*=5.6 Hz, 2H), 2.53-2.51 (m, 4H), 2.25 (s, 3H).

### Step 6: Synthesis of compound WX004-7

Compound **WX004-6** (890 mg, 2.97 mmol) was dissolved in toluene (10 mL) at room temperature under nitrogen. Then triethylamine (660.98 mg, 6.53 mmol, 909.19 µL) and diphenyl phosphoryl azide (898.82 mg, 3.27 mmol, 707.73 µL) were added. The reaction mixture was stirred at room temperature for 10 minutes, and phenol (1.40 g, 14.85 mmol, 1.31 mL) was added. The reaction mixture was heated to 100 °C and stirred to react for 1 hour. After the reaction was completed, the reaction solution was directly concentrated under reduced pressure to remove the solvent, and the resulting residue was separated by column chromatography (eluent: petroleum ether/ethyl acetate = 4/1-1/1, volume ratio, wet loading), to give compound **WX004-7.** MS-ESI *m*/*z*: 391.2 [M+H]⁺, 392.2 [M+H+1]⁺, 393.2 [M+H+2]⁺. ¹H NMR (400 MHz, CDCl₃) δ: 7.45-7.38 (m, 2H), 7.29-7.26 (m, 1H), 7.25-7.16 (m, 3H), 6.93 (br s, 1H), 6.90 (d, *J*=2.0 Hz, 1H), 4.11 (t, *J*=5.8 Hz, 2H), 3.73 (t, *J*=4.8 Hz, 4H), 2.82 (t, *J*=5.6 Hz, 2H), 2.59 (t, *J*=4.6 Hz, 4H), 2.23 (s, 3H).

### Step 7: Synthesis of compound WX004

Compound **WX004-7** (80 mg, 204.68 µmol) and compound **WX004-2** (52.86 mg, 204.68 µmol) were dissolved in N,N-dimethylformamide (2 mL) at room temperature. Then triethylamine (41.42 mg, 409.35 µmol, 56.98 µL) was added. The reaction mixture was stirred to react at room temperature for 12 hours. After the reaction was completed, the reaction solution was poured into water (10 mL), and the mixture was extracted with ethyl acetate (10 mL×3). The organic phases were combined, dried over anhydrous sodium sulfate, and filtered. The filtrate was concentrated under reduced pressure to remove the solvent. The resulting residue was separated by preparative HPLC (mobile phase: acetonitrile/water; neutral system: NH₄HCO₃) to give the target compound **WX004.** MS-ESI *m*/*z*: 555.0 [M+H]⁺, 556.0 [M+H+1]⁺, 557.0 [M+H+2]⁺. ¹H NMR (400 MHz, DMSO_*d₆*) δ: 10.87 (s, 1H), 8.60 (s, 1H), 7.86 (s, 1H), 7.52-7.47 (m, 2H), 7.24 (dd, *J*=1.6, 8.4 Hz, 1H), 7.11 (d, *J*=2.0 Hz, 1H), 6.97 (d, *J*=2.0 Hz, 1H), 6.65 (t, *J*=5.6 Hz, 1H), 4.32 (d, *J*=6.0 Hz, 2H), 4.10 (dd, *J*=4.6, 12.2 Hz, 1H), 3.99 (t, *J*=5.8 Hz, 2H), 3.53 (t, *J*=4.6 Hz, 4H), 2.78-2.70 (m, 1H), 2.68 (t, *J*=5.6 Hz, 2H), 2.59-2.50 (m, 1H), 2.44-2.40 (m, 4H), 2.35-2.29 (m, 1H), 2.12-2.08 (m, 1H), 2.06 (s, 3H).

### Example 5

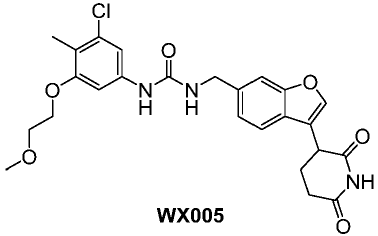

### Synthetic route:

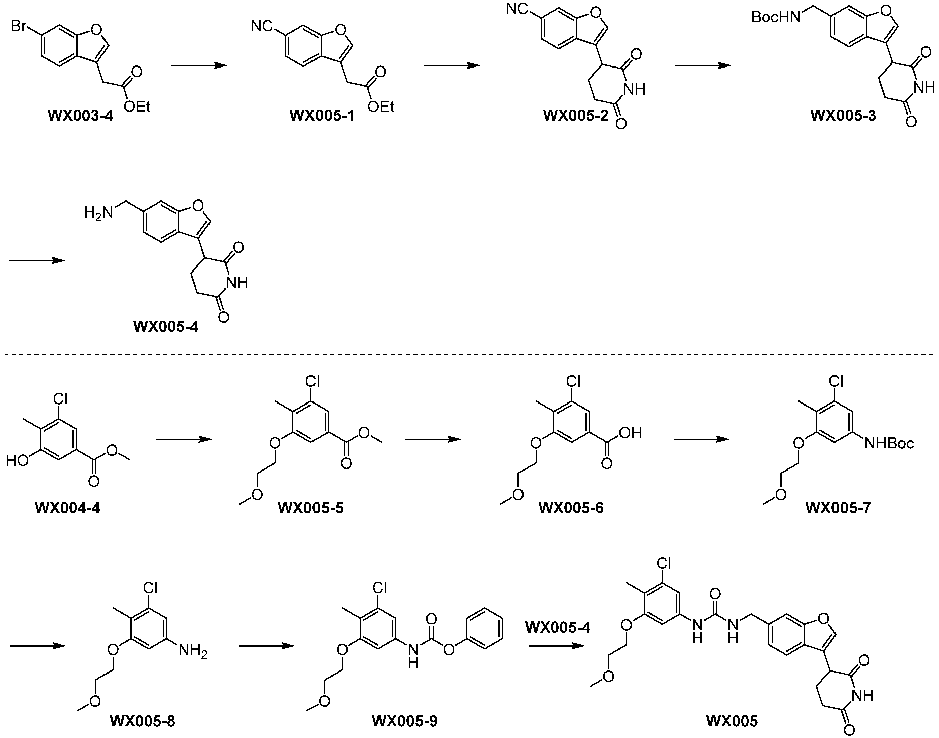

### Step 1: Synthesis of compound WX005-1

Compound **WX003-4** (5.5 g, 19.43 mmol) was dissolved in N,N-dimethylformamide (60 mL) at room temperature under nitrogen. Then zinc cyanide (2.87 g, 24.44 mmol, 1.55 mL), 2-(dicyclohexylphosphino)-2,4,6-triisopropylbiphenyl (926.10 mg, 1.94 mmol) and tris(dibenzylideneacetone)dipalladium (889.46 mg, 971.33 µmol) were added in sequence. The reaction mixture was heated to 80°C and stirred to react for 12 hours. After the reaction was completed, the reaction solution was cooled to room temperature, and water (200 mL) was added. The mixture was adjusted to pH =12-13 by adding 0.5 M aqueous sodium hydroxide solution, and extracted with ethyl acetate (100 mL×3). The organic phases were combined, washed with semi-saturated brine (50 mL×3), dried over anhydrous sodium sulfate, and filtered. The filtrate was concentrated under reduced pressure to remove the solvent. The resulting residue was separated by column chromatography (eluent: petroleum ether/ethyl acetate = 1/0-9/1, volume ratio) to give compound **WX005-1.** MS-ESI *m*/*z*: 230.2 [M+H]⁺. ¹H NMR (400 MHz, CDCl₃) δ: 7.83 (t, *J*=1.2 Hz, 1H), 7.82-2.79 (m, 1H), 7.67 (d, *J*=8.4 Hz, 1H), 7.54 (dd, *J*=1.2, 8.0 Hz, 1H), 4.21 (q, *J*=7.0 Hz, 2H), 3.72 (d, *J*=1.2 Hz, 2H), 1.28 (t, *J*=7.2 Hz, 3H).

### Step 2: Synthesis of compound WX005-2

Compound **WX005-1** (3.1 g, 13.52 mmol) was dissolved in N,N-dimethylformamide (50 mL) at room temperature under nitrogen, and the mixture was cooled to 0°C. Acrylamide (961.22 mg, 13.52 mmol) and potassium tert-butoxide (1.67 g, 14.88 mmol) were added, and the reaction mixture was stirred at 0°C to react for 0.5 hours. After the reaction was completed, the reaction solution was poured into water (100 mL), and the mixture was extracted with ethyl acetate (50 mL×3). The organic phases were combined, washed with semi-saturated brine (50 mL×3), dried over anhydrous sodium sulfate, and filtered. The filtrate was concentrated under reduced pressure to remove the solvent. The resulting residue was stirred with methanol (10 mL) at room temperature for 30 minutes, and solids were precipitated. The mixture was filtered, and the solids were collected, dried under vacuum to give compound **WX005-2.** ¹H NMR (400 MHz, DMSO_*d₆*) δ: 10.95 (s, 1H), 8.26-8.15 (m, 2H), 7.81 (d, *J*=8.4 Hz, 1H), 7.67 (dd, *J*=1.2, 8.4 Hz, 1H), 4.22 (dd, *J*=4.6, 12.6 Hz, 1H), 2.83-2.70 (m, 1H), 2.66-2.55 (m, 1H), 2.42-2.26 (m, 1H), 2.17-2.06 (m, 1H).

### Step 3: Synthesis of compound WX005-3

Wet palladium on carbon (0.3 g, purity: 10%) was added to tetrahydrofuran (30 mL) at room temperature, followed by compound **WX005-2** (750 mg, 2.95 mmol) and di-tert-butyl dicarbonate (772.60 mg, 3.54 mmol, 813.26 µL). The atmosphere was replaced with hydrogen three times, and the reaction mixture was stirred at room temperature under hydrogen atmosphere (15 psi) for 12 hours. After the reaction was completed, the reaction solution was directly filtered through diatomaceous earth. The filtrate was collected, and concentrated under reduced pressure to remove the solvent. The resulting crude product was separated by column chromatography (eluent: petroleum ether/ethyl acetate = 4/1-3/2, volume ratio) to give compound **WX005-3.** MS-ESI *m*/*z*: 357.1 [M-H]⁻.

### Step 4: Synthesis of compound WX005-4 hydrochloride

Compound **WX005-3** (700 mg, 1.95 mmol) was dissolved in hydrochloric acid/ethyl acetate (4 M, 14.00 mL) at room temperature. The reaction mixture was stirred at room temperature for 10 minutes, and solids were precipitated. After the reaction was completed, the reaction solution was directly filtered. The filter cake was collected, and dried under reduced pressure to give compound **WX005-4** hydrochloride. MS-ESI *m*/*z*: 257.0 [M-H]⁻. ¹H NMR (400 MHz, DMSO_*d₆*) δ: 10.91 (s, 1H), 8.45 (s, 3H), 7.95 (s, 1H), 7.74 (s, 1H), 7.62 (d, *J*=8.0 Hz, 1H), 7.37 (dd, *J*=1.2, 8.0 Hz, 1H), 4.20-4.08 (m, 3H), 2.83-2.70 (m, 1H), 2.63-2.53 (m, 1H), 2.41-2.27 (m, 1H), 2.16-2.05 (m, 1H). It was separated by preparative HPLC (column: Waters Xbridge Prep OBD C18 150*40mm*10 µm; mobile phase: acetonitrile/water; neutral system: NH₄HCO₃), to give compound WX005-4.

### Step 5: Synthesis of compound WX005-5

Compound **WX004-4** (2 g, 9.97 mmol) was dissolved in acetonitrile (20 mL) at room temperature under nitrogen. Then 2-bromoethyl methyl ether (1.39 g, 9.97 mmol, 936.23 µL), potassium iodide (165.49 mg, 996.92 µmol), and potassium carbonate (2.76 g, 19.94 mmol) were added. The reaction mixture was heated to 80°C and stirred to react for 12 hours. After the reaction was completed, the reaction solution was cooled to room temperature and filtered. The filter cake was rinsed with tetrahydrofuran (20 mL×3). The filtrate was collected and concentrated under reduced pressure to remove the solvent. The resulting residue was separated by column chromatography (eluent: petroleum ether/ethyl acetate = 1/0-19/1, volume ratio) to give compound **WX005-5.** ¹H NMR (400 MHz, CDCl₃) δ: 7.68 (d, *J*=1.6 Hz, 1H), 7.40 (d, *J*=0.8 Hz, 1H), 4.22-4.16 (m, 2H), 3.91 (s, 3H), 3.82-3.77 (m, 2H), 3.47 (s, 3H), 2.35 (s, 3H).

### Step 6: Synthesis of compound WX005-6

Compound **WX005-5** (2.5 g, 9.66 mmol) was dissolved in tetrahydrofuran (18 mL) and water (6 mL) at room temperature. Then lithium hydroxide monohydrate (1.62 g, 38.66 mmol) was added. The reaction mixture was stirred to react at room temperature for 12 hours. After the reaction was completed, water (20 mL) was added to the reaction solution. The mixture was adjusted to pH of 5~6 with 6 M dilute hydrochloric acid, and extracted with ethyl acetate (30 mL×3). The organic phases were combined, dried over anhydrous sodium sulfate, and filtered. The filtrate was concentrated under reduced pressure to remove the solvent to give compound **WX005-6.** ¹H NMR (400 MHz, DMSO_*d₆*) δ: 13.17 (br s, 1H), 7.53 (d, *J*=0.8 Hz, 1H), 7.41 (d, *J*=0.8 Hz, 1H), 4.22-4.16 (m, 2H), 3.73-3.67 (m, 2H), 3.33 (s, 3H), 2.26 (s, 3H).

### Step 7: Synthesis of compound WX005-7

Compound **WX005-6** (1.9 g, 7.77 mmol) was dissolved in tert-butanol (30 mL) at room temperature under nitrogen. Then diphenyl phosphoryl azide (3.21 g, 11.65 mmol, 2.52 mL) and triethylamine (1.57 g, 15.53 mmol, 2.16 mL) were added. The reaction mixture was heated to 100°C and stirred to react for 12 hours. After the reaction was completed, the reaction solution was directly concentrated under reduced pressure to remove the solvent. The resulting residue was separated by column chromatography (eluent: petroleum ether/ethyl acetate = 1/0-19/1, volume ratio) to give compound **WX005-7.** MS-ESI *m*/*z*: 260.1 [M-55]⁺, 261.1 [M+H-55]⁺, 262.1 [M+2-55]⁺. ¹H NMR (400 MHz, CDCl₃) δ: 7.01 (br s, 1H), 6.90 (d, *J*=2.0 Hz, 1H), 6.40 (br s, 1H), 4.14-4.08 (m, 2H), 3.79-3.74 (m, 2H), 3.46 (s, 3H), 2.23 (s, 3H), 1.52 (s, 9H).

### Step 8: Synthesis of compound WX005-8 hydrochloride

Compound **WX005-7** (1.65 g, 5.22 mmol) was dissolved in hydrochloric acid/ethyl acetate (4 M, 30 mL) at room temperature. The reaction mixture was stirred at room temperature for 0.5 hours, and solids were precipitated. After the reaction was completed, the reaction solution was directly filtered. The filter cake was collected, and dried in vacuum to give compound **WX005-8** hydrochloride. MS-ESI *m*/*z*: 214.1 [M-H]⁻. ¹H NMR (400 MHz, DMSO_*d₆*) δ: 6.80 (d, *J*=1.6 Hz, 1H), 6.72 (d, *J*=1.6 Hz, 1H), 4.11-4.04 (m, 2H), 3.71-3.65 (m, 2H), 3.33 (s, 3H), 2.14 (s, 3H).

### Step 9: Synthesis of compound WX005-9

Compound **WX005-8** hydrochloride (200 mg, 793.22 µmol) was dissolved in dichloromethane (5 mL) at room temperature under nitrogen. Then triethylamine (234.59 mg, 2.32 mmol, 322.68 µL) was added, and the mixture was cooled to 0°C. Phenyl chloroformate (174.23 mg, 1.11 mmol, 139.38 µL) was added dropwise. The reaction mixture was stirred to react at room temperature for 0.5 hours. After the reaction was completed, water (20 mL) was added to the reaction solution, and the mixture was extracted with dichloromethane (10 mL×3). The organic phases were combined, dried over anhydrous sodium sulfate, and filtered. The filtrate was concentrated under reduced pressure to remove the solvent. The resulting residue was separated by column chromatography (eluent: petroleum ether/ethyl acetate = 1/0-4/1, volume ratio) to give compound **WX005-9.** MS-ESI *m*/*z*: 336.2 [M+H]⁺, 337.2 [M+H+1]⁺, 338.2 [M+H+2]⁺. ¹H NMR (400 MHz, CDCl₃) δ: 7.44-7.37 (m, 2H), 7.26-7.22 (m, 1H), 7.21-7.15 (m, 3H), 6.93 (d, *J*=2.0 Hz, 1H), 6.89 (br s, 1H), 4.13-4.08 (m, 2H), 3.78-3.72 (m, 2H), 3.45 (s, 3H), 2.25 (s, 3H).

### Step 10: Synthesis of compound WX005

Compound **WX005-9** (80 mg, 238.25 µmol) was dissolved in N,N-dimethylformamide (2 mL) at room temperature. Then compound **WX005-4** hydrochloride (98.54 mg, 334.34 µmol) and triethylamine (72.32 mg, 714.75 µmol, 99.48 µL) were added in sequence. The reaction mixture was stirred to react at room temperature for 12 hours. After the reaction was completed, the reaction solution was poured into water (20 mL), and the mixture was extracted with ethyl acetate (20 mL×3). The organic phases were combined, dried over anhydrous sodium sulfate, and filtered. The filtrate was concentrated under reduced pressure to remove the solvent. The resulting residue was separated by preparative HPLC (mobile phase: acetonitrile/water; acidic system: HCl) to give the target compound **WX005.** MS-ESI *m*/*z*: 500.2 [M+H]⁺, 501.1 [M+H+1]⁺, 502.1 [M+H+2]⁺. ¹H NMR (400 MHz, DMSO_*d₆*) δ: 10.89 (s, 1H), 8.67 (s, 1H), 7.86 (s, 1H), 7.53 (d, *J*=8.0 Hz, 1H), 7.47 (s, 1H), 7.20 (dd, *J*=1.0, 8.2 Hz, 1H), 7.17 (d, *J*=2.0 Hz, 1H), 6.99 (d, *J*=2.0 Hz, 1H), 6.73 (t, *J*=6.0 Hz, 1H), 4.39 (d, *J*=5.6 Hz, 2H), 4.12 (dd, *J*=4.8, 12.0 Hz, 1H), 4.06-4.01 (m, 2H), 3.70-3.64 (m, 2H), 3.31 (s, 3H), 2.80-2.69 (m, 1H), 2.62-2.53 (m, 1H), 2.32-2.25 (m, 1H), 2.15-2.07 (m, 4H).

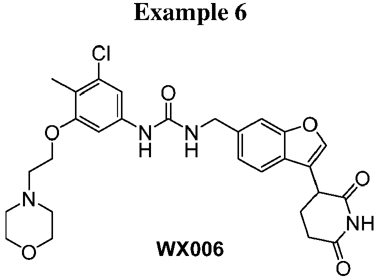

### Synthetic route:

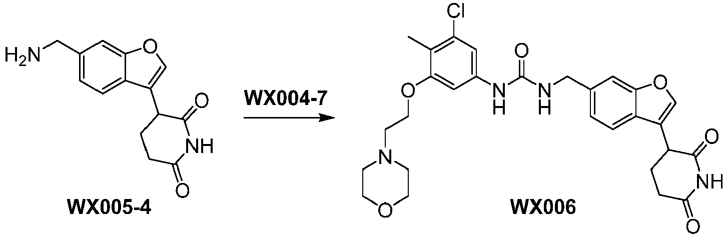

### Synthesis of compound WX006

Compound **WX004-7** (50 mg, 127.92 µmol) and compound **WX005-4** hydrochloride (36.34 mg, 140.72 µmol) were dissolved in N,N-dimethylformamide (1 mL) at room temperature. Then triethylamine (25.89 mg, 255.85 µmol, 35.61 µL) was added. The reaction mixture was stirred to react at room temperature for 12 hours. After the reaction was completed, water (10 mL) was added to the reaction solution, and the mixture was extracted with ethyl acetate (20 mL×3). The organic phases were combined, dried over anhydrous sodium sulfate, and filtered. The filtrate was concentrated under reduced pressure to remove the solvent. The resulting residue was separated by preparative HPLC (mobile phase: acetonitrile/water; neutral system: NH₄HCO₃) to give the target compound **WX006.** MS-ESI *m*/*z*: 555.3 [M+H]⁺, 556.2 [M+H+1]⁺, 557.3 [M+H+2]⁺. ¹H NMR (400 MHz, DMSO_*d₆*) δ: 10.89 (s, 1H), 8.66 (s, 1H), 7.86 (s, 1H), 7.53 (d, *J*=8.0 Hz, 1H), 7.47 (s, 1H), 7.19 (dd, *J*=1.2, 8.4 Hz, 1H), 7.15 (d, *J*=2.0 Hz, 1H), 7.01 (d, *J*=1.6 Hz, 1H), 6.74 (t, *J*=5.8 Hz, 1H), 4.38 (d, *J*=6.0 Hz, 2H), 4.12 (dd, *J*=5.0, 11.8 Hz, 1H), 4.03 (t, *J*=5.6 Hz, 2H), 3.57 (t, *J*=4.6 Hz, 4H), 2.80-2.68 (m, 3H), 2.60-2.54 (m, 1H), 2.47-2.43 (m, 4H), 2.32-2.25 (m, 1H), 2.15-2.05 (m, 4H).

### Example 7

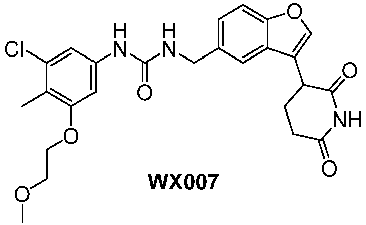

### Synthetic route:

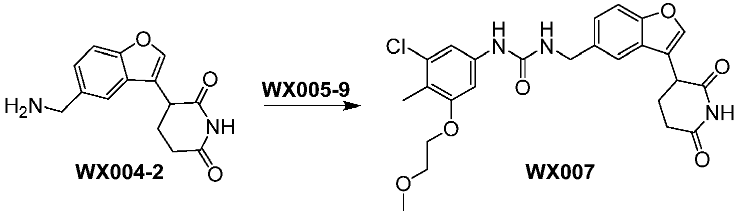

### Synthesis of compound WX007

Compound **WX005-9** (76 mg, 226.34 µmol) was dissolved in N,N-dimethylformamide (2 mL) at room temperature. Then compound **WX004-2** (70.15 mg, 271.61 µmol), triethylamine (45.81 mg, 452.68 µmol, 63.01 µL) were added in sequence. The reaction mixture was stirred to react at room temperature for 12 hours. After the reaction was completed, the reaction solution was poured into water (20 mL), and the mixture was extracted with ethyl acetate (20 mL×3). The organic phases were combined, dried over anhydrous sodium sulfate, and filtered. The filtrate was concentrated under reduced pressure to remove the solvent. The resulting residue was separated by preparative HPLC (mobile phase: acetonitrile/water; acidic system: HCl) to give the target compound **WX007.** MS-ESI *m*/*z*: 500.2 [M+H]⁺, 501.2 [M+H+1]⁺, 502.2 [M+H+2]⁺. ¹H NMR (400 MHz, DMSO_*d₆*) δ: 10.89 (s, 1H), 8.63 (s, 1H), 7.89 (s, 1H), 7.53 (d, *J*=8.8 Hz, 1H), 7.51 (d, *J*=1.2 Hz, 1H), 7.27 (dd, *J*=1.6, 8.4 Hz, 1H), 7.17 (d, *J*=2.0 Hz, 1H), 6.98 (d, *J*=1.8 Hz, 1H), 6.67 (t, *J*=5.8 Hz, 1H), 4.36 (d, *J*=5.6 Hz, 2H), 4.13 (dd, *J*=4.8, 12.0 Hz, 1H), 4.07-4.00 (m, 2H), 3.70-3.64 (m, 2H), 3.31 (s, 3H), 2.81-2.70 (m, 1H), 2.61-2.58 (m, 1H), 2.40-2.33 (m, 1H), 2.17-2.11 (m, 1H), 2.10 (s, 3H).

### Example 8

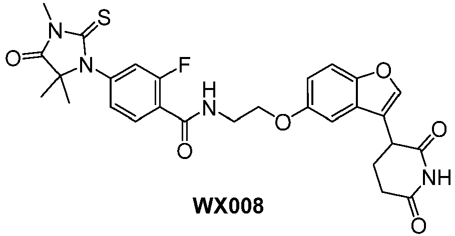

### Synthetic route:

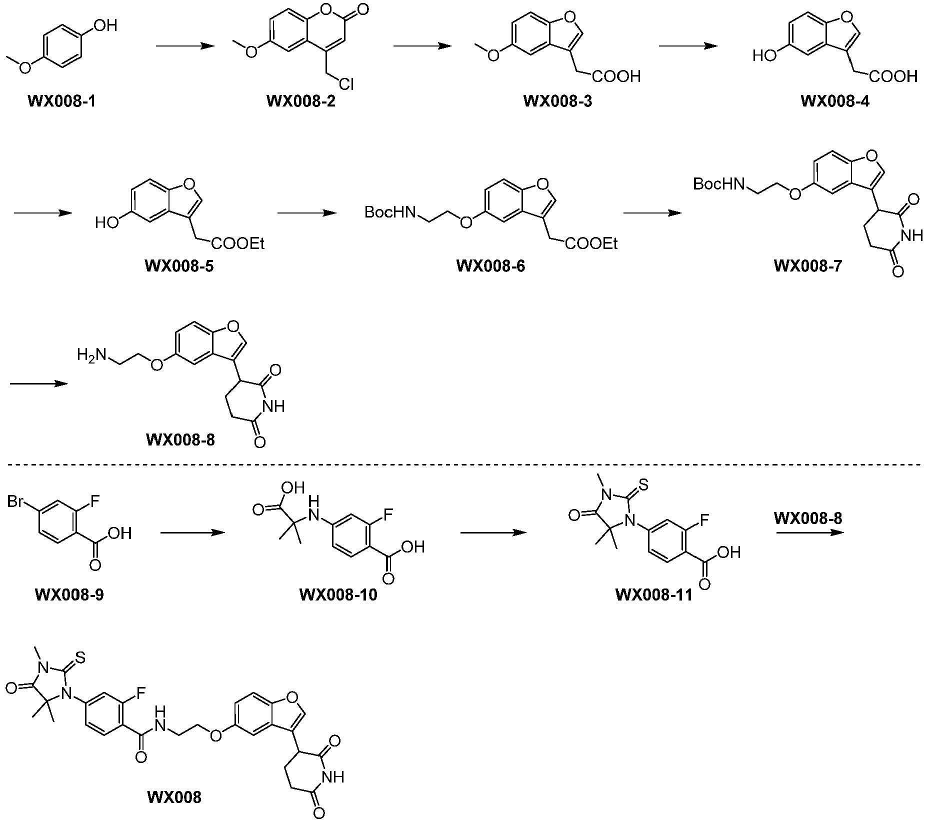

### Step 1: Synthesis of compound WX008-2

Sulfuric acid (1.22 kg, 12.19 mol, 663.00 mL, purity: 98%) was added dropwise to ice water (221 mL) at room temperature, then compound **WX008-1** (170 g, 1.37 mol) was added. The mixture was cooled to 0°C, and ethyl 4-chloroacetoacetate (247.93 g, 1.51 mol) was added dropwise. The reaction mixture was stirred to react at room temperature for 12 hours. After the reaction was completed, the reaction solution was poured into 800 mL of ice water to be quenched, and solids were precipitated. The mixture was filtered and the filter cake was collected. The filter cake was dried under reduced pressure in vacuum to give compound **WX008-2.** MS-ESI *m*/*z*: 225.1 [M+H]⁺. ¹H NMR (400 MHz, DMSO_*d₆*) δ: 7.40 (d, *J*=9.2 Hz, 1H), 7.31 (d, *J*=3.2 Hz, 1H), 7.29-7.21 (m, 1H), 6.68 (s, 1H), 5.06 (s, 2H), 3.84 (s, 3H).

### Step 2: Synthesis of compound WX008-3

Sodium hydroxide (45.45 g, 1.14 mol) was dissolved in a 2 M solution of sodium hydroxide in water (568.125 mL) at room temperature, then compound **WX008-2** (250 g, 1.11 mol) was added. The reaction mixture was heated to 80°C and stirred to react for 12 hours. After the reaction was completed, the reaction solution was cooled to room temperature. Water (1000 mL) was added, and the mixture was extracted with ethyl acetate (600 mL). The organic phase was discarded. The aqueous phase was adjusted to pH 5-6 with 12 M concentrated hydrochloric acid. Solids were precipitated. The mixture was extracted with ethyl acetate (600 mL×3). The organic phases were combined, washed with saturated brine (400 mL×3), dried over anhydrous sodium sulfate, and filtered. The filtrate was concentrated under reduced pressure to give compound **WX008-3.** MS-ESI *m*/*z*: 207.1 [M+H]⁺.

### Step 3: Synthesis of compound WX008-4

Compound **WX008-3** (68 g, 329.79 mmol) was dissolved in dichloromethane (1300 mL) at room temperature under nitrogen, and the mixture was cooled to -30°C under nitrogen. Boron tribromide (223.07 g, 890.42 mmol, 85.80 mL) was added dropwise, and the reaction mixture was slowly warmed to room temperature and stirred to react for 1 hour. After the reaction was completed, the reaction solution was poured into ice water (2000 mL) to be quenched. The layers were separated. The organic phase was collected, and the aqueous phase was extracted with ethyl acetate (800 mL×3). The organic phases were combined, washed with saturated brine (600 mL×3), dried over anhydrous sodium sulfate, and filtered. The filtrate was concentrated under reduced pressure to give compound **WX008-4.** MS-ESI *m*/*z*: 193.1 [M+H]⁺.

### Step 4: Synthesis of compound WX008-5

Compound **WX008-4** (126 g, 655.68 mmol) was dissolved in ethanol (410 mL) at room temperature under nitrogen, and concentrated sulfuric acid (12.86 g, 131.14 mmol, 6.99 mL, purity: 98%) was added. The reaction mixture was heated to 80°C and stirred to react for 12 hours. After the reaction was completed, the reaction mixture was concentrated under reduced pressure to remove the solvent. The resulting residue was separated by column chromatography (eluent: petroleum ether/ethyl acetate = 1/0-9/1, volume ratio) to give compound **WX008-5.** MS-ESI *m*/*z*: 221.1 [M+H]⁺. ¹H NMR (400 MHz, CDCl₃) δ: 7.59 (s, 1H), 7.31 (d, *J*=8.4 Hz, 1H), 6.98 (d, *J*=2.4 Hz, 1H), 6.81 (dd, *J*=2.6, 8.6 Hz, 1H), 5.70 (s, 1H), 4.20 (q, *J*=7.0 Hz, 2H), 3.64 (d, *J*=1.2 Hz, 2H), 1.27 (t, *J*=7.2 Hz, 3H).

### Step 5: Synthesis of compound WX008-6

Compound **WX008-5** (3 g, 13.62 mmol) was dissolved in tetrahydrofuran (40 mL) at room temperature under nitrogen. Then N-(tert-butoxycarbonyl)ethanolamine (3.29 g, 20.43 mmol, 3.17 mL) and azodicarbonyldipiperidine (6.87 g, 27.25 mmol) were added, and the mixture was cooled to 0°C. A solution of tributylphosphine (5.51 g, 27.25 mmol, 6.72 mL) in tetrahydrofuran (10 mL) was added dropwise, and the reaction mixture was returned to room temperature and stirred to react for 3 hours. After the reaction was completed, the reaction solution was filtered directly, and the filter cake was rinsed with tetrahydrofuran (5 mL×3). The filtrate was collected, and concentrated under reduced pressure to remove the solvent. The resulting residue was separated by column chromatography (eluent: petroleum ether/ethyl acetate = 1/0-9/1, volume ratio) to give compound **WX008-6.** MS-ESI *m*/z: 264.2 [M+H-100]⁺. ¹H NMR (400 MHz, CDCl₃) δ: 7.61 (s, 1H), 7.37 (d, *J*=8.8 Hz, 1H), 7.02 (d, *J*=2.8 Hz, 1H), 6.91 (dd, *J*=2.6, 9.0 Hz, 1H), 5.04 (br s, 1H), 4.20 (q, *J*=7.2 Hz, 2H), 4.06 (t, *J*=5.2 Hz, 2H), 3.66 (d, *J*=1.2 Hz, 2H), 3.61-3.52 (m, 2H), 1.47 (s, 9H), 1.29 (t, *J*=7.2 Hz, 3H).

### Step 6: Synthesis of compound WX008-7

Compound **WX008-6** (1.8 g, 4.95 mmol) was dissolved in tetrahydrofuran (40 mL) at room temperature under nitrogen. Then acrylamide (352.06 mg, 4.95 mmol) and potassium tert-butoxide (833.71 mg, 7.43 mmol) were added simultaneously. The reaction mixture was stirred to react at room temperature for 1 hour. After the reaction was completed, the reaction solution was poured into water (50 mL), and the mixture was extracted with ethyl acetate (30 mL×3). The organic phases were combined, dried over anhydrous sodium sulfate, and filtered. The filtrate was concentrated under reduced pressure to remove the solvent. The resulting residue was separated by column chromatography (eluent: petroleum ether/ethyl acetate = 9/1-7/3, volume ratio), to give compound **WX008-7.** ¹H NMR (400 MHz, CDCl₃) δ: 8.09 (br s, 1H), 7.56 (s, 1H), 7.40 (d, *J*=9.2 Hz, 1H), 6.97-6.95 (m, 1H), 6.95-6.91 (m, 1H), 5.02 (br s, 1H), 4.06 (t, *J*=5.0 Hz, 2H), 3.97 (t, *J*=7.6 Hz, 1H), 3.62-3.49 (m, 2H), 2.88-2.69 (m, 2H), 2.42-2.32 (m, 2H), 1.47 (s, 9H).

### Step 7: Synthesis of compound WX008-8 hydrochloride

Compound **WX008-7** (600 mg, 1.54 mmol) was dissolved in hydrochloric acid/ethyl acetate (4 M, 20 mL) at room temperature. The reaction mixture was stirred at room temperature to react for 1 hour, and solids were precipitated. After the reaction was completed, the reaction solution was directly filtered. The solid was collected, and dried under reduced pressure to give compound **WX008-8** hydrochloride. MS-ESI *m*/*z*: 287.2 [M-H]⁻. ¹H NMR (400 MHz, DMSO_*d₆*) δ: 10.89 (s, 1H), 8.25 (br s, 3H), 7.88 (s, 1H), 7.51 (d, *J*=9.2 Hz, 1H), 7.16 (d, *J*=2.8 Hz, 1H), 6.98 (dd, *J*=2.4, 8.8 Hz, 1H), 4.23-4.16 (m, 2H), 4.13 (dd, *J*=4.8, 12.0 Hz, 1H), 3.25-3.16 (m, 2H), 2.81-2.70 (m, 1H), 2.63-2.55 (m, 1H), 2.39-2.27 (m, 1H), 2.16-2.05 (m, 1H).

### Step 8: Synthesis of compound WX008-10

Compound **WX008-9** (10 g, 45.66 mmol) and compound 2-aminoisobutyric acid (14.13 g, 136.98 mmol) were added to a mixed solution of N,N-dimethylformamide (100 mL) and water (10 mL) at room temperature under nitrogen. Copper iodide (1.74 g, 9.14 mmol), copper powder (580.00 mg, 9.13 mmol), potassium carbonate (31.55 g, 228.30 mmol), N,N-dimethylglycine (2.35 g, 22.83 mmol) were slowly added to the reaction solution one by one. The atmosphere was replaced with nitrogen three times, and the reaction mixture was heated to 110°C and stirred to react for 12 hours. After the reaction was completed, the reaction solution was cooled to room temperature, and 100 mL of ice water was added. The mixture was adjusted to pH of 4-5 with 6 M hydrochloric acid, and extracted with ethyl acetate (200 mL×2). The organic phases were combined, washed with saturated brine (200 mL×2), dried over anhydrous sodium sulfate, and filtered. The filtrate was concentrated under reduced pressure to remove the solvent. The resulting crude product was added to dichloromethane (15 mL), and the mixture was stirred for 1 hour, then filtered. The filter cake was washed with dichloromethane (5 mL×2). The solids were collected, and dried under reduced pressure to give compound **WX008-10.** MS-ESI m/z: 242.2 [M+H]⁺. ¹H NMR (400 MHz, DMSO_*d₆*) δ: 12.53 (s, 1H), 7.59 (t, *J*=8.8 Hz, 1H), 6.96 (s, 1H), 6.33 (d, *J*=8.8 Hz, 1H), 6.15 (d, *J*=14.4 Hz, 1H), 1.45 (s, 6H).

### Step 9: Synthesis of compound WX008-11

Compound **WX008-10** (4 g, 16.58 mmol) and methyl isothiocyanate (1.82 g, 24.87 mmol, 1.70 mL) were dissolved in ethanol (80 mL) at room temperature, and triethylamine (5.71 g, 56.37 mmol, 7.85 mL) was slowly added. The reaction mixture was heated to 80°C and stirred to react for 12 hours. After the reaction was completed, the reaction solution was cooled to room temperature, concentrated under reduced pressure to remove the solvent. Water (20 mL) was added. The mixture was adjusted to pH of 3-4 with 1 M hydrochloric acid, extracted with ethyl acetate (20 mL×2), washed with saturated brine (20 mL×2), dried over anhydrous sodium sulfate, and filtered. The filtrate was concentrated under reduced pressure to remove the solvent. The resulting residue was separated by column chromatography (eluent: petroleum ether/ethyl acetate = 1/0-2/3, volume ratio) to give compound **WX008-11.** MS-ESI *m*/*z*: 297.1 [M+H]⁺. ¹H NMR (400 MHz, DMSO_*d₆*) δ: 13.44 (s, 1H), 7.99 (t, *J*=8.2 Hz, 1H), 7.43 (dd, *J*=1.6, 11.2 Hz, 1H), 7.30 (dd, *J*=1.6, 8.4 Hz, 1H), 3.22 (s, 3H), 1.38 (s, 6H).

### Step 10: Synthesis of compound WX008

Compound **WX008-8** hydrochloride (100 mg, 307.92 µmol) was dissolved in N,N-dimethylformamide (2 mL) at room temperature. Then compound **WX008-11** (109.49 mg, 369.50 µmol) was added, and O-(7-azabenzotriazol-1-yl)-*N,N,N',N*'-tetramethyluronium hexafluorophosphate (234.16 mg, 615.84 µmol) and triethylaine (124.63 mg, 1.23 mmol, 171.43 µL) were finally added. The reaction mixture was stirred to react at room temperature for 5 hours. After the reaction was completed, the reaction solution was poured into water (20 mL), and the mixture was extracted with ethyl acetate (20 mL×3). The organic phases were combined, dried over anhydrous sodium sulfate, and filtered. The filtrate was concentrated under reduced pressure to remove the solvent. The resulting residue was separated by preparative HPLC (mobile phase: acetonitrile/water; acidic system: HCl) to give the target compound **WX008.** MS-ESI m/z: 567.2 [M+H]⁺. ¹H NMR (400 MHz, DMSO_*d₆*) δ: 10.87 (s, 1H), 8.72 (t, *J*=4.8 Hz, 1H), 7.85 (s, 1H), 7.73 (t, *J*=8.2 Hz, 1H), 7.47 (d, *J*=8.8 Hz, 1H), 7.41 (dd, *J*=1.8, 10.6 Hz, 1H), 7.27 (dd, *J*=1.8, 8.2 Hz, 1H), 7.16 (d, *J*=2.4 Hz, 1H), 6.94 (dd, *J*=2.6, 9.0 Hz, 1H), 4.19-4.06 (m, 3H), 3.66 (q, *J*=5.6 Hz, 2H), 3.22 (s, 3H), 2.81-2.65 (m, 1H), 2.61-2.54 (m, 1H), 2.37-2.29 (m, 1H), 2.14-2.06 (m, 1H), 1.37 (s, 6H).

### Example 9

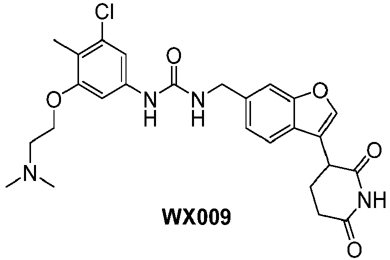

### Synthetic route:

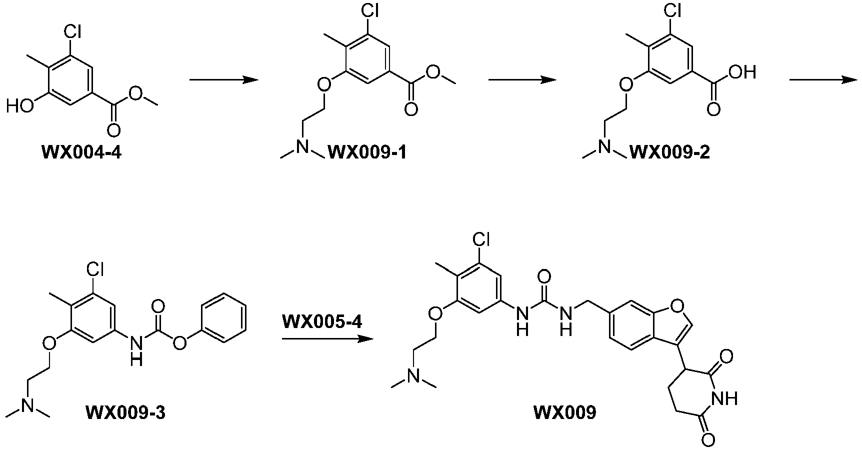

### Step 1: Synthesis of compound WX009-1

Compound **WX004-4** (1.96 g, 9.77 mmol) was dissolved in acetonitrile (30 mL) at room temperature under nitrogen. Then N,N-dimethylaminoethyl chloride hydrochloride (2.81 g, 19.54 mmol, 936.23 µL), potassium iodide (162.18 mg, 976.98 µmol), and potassium carbonate (5.40 g, 39.08 mmol) were added. The reaction mixture was heated to 80°C and stirred to react for 12 hours. After the reaction was completed, the reaction solution was cooled to room temperature and filtered. The filter cake was rinsed with tetrahydrofuran (20 mL×3). The filtrate was collected and concentrated under reduced pressure to remove the solvent. The resulting residue was separated by column chromatography (eluent: petroleum ether/ethyl acetate=4/1-2/3~dichloromethane/methanol=1/0-19/1, volume ratio) to give compound **WX009-1.** MS-ESI *m*/*z*: 272.2 [M+H]⁺, 273.2 [M+H+1]⁺, 274.2 [M+H+2]⁺.

### Step 2: Synthesis of compound WX009-2

Compound **WX009-1** (0.8 g, 2.94 mmol) was dissolved in tetrahydrofuran (9 mL) and water (3 mL) at room temperature. Then lithium hydroxide monohydrate (494.12 mg, 11.78 mmol) was added. The reaction mixture was stirred to react at room temperature for 2 hours. After the reaction was completed, the reaction solution was directly concentrated under reduced pressure to remove the solvent. The resulting residue was separated by preparative HPLC (mobile phase: acetonitrile/water; neutral system: NH₄HCO₃), to give compound **WX009-2.** MS-ESI *m*/*z*: 256.1 [M-H]⁻, 257.0 [M-H+1]⁻, 258.1 [M-H+2]⁻. ¹H NMR (400 MHz, DMSO_*d₆*) δ: 7.51 (d, *J*=1.2 Hz, 1H), 7.41 (d, *J*=0.8 Hz, 1H), 4.18 (t, *J*=5.6 Hz, 2H), 2.82 (t, *J*=5.4 Hz, 2H), 2.34 (s, 6H), 2.24 (s, 3H).

### Step 3: Synthesis of compound WX009-3

Compound **WX009-2** (240 mg, 931.27 µmol) was dissolved in toluene (2.5 mL) at room temperature under nitrogen. Then triethylamine (207.32 mg, 2.05 mmol, 285.17 µL), diphenyl phosphoryl azide (281.91 mg, 1.02 mmol, 221.98 µL) were added. The reaction mixture was heated to 100°C and stirred to react for 10 minutes, then phenol (438.21 mg, 4.66 mmol, 409.55 µL) was added. The reaction mixture was stirred at 100°C for another 0.5 hours. After the reaction was completed, the reaction solution was directly concentrated under reduced pressure to remove the solvent. The resulting residue was separated by preparative HPLC (mobile phase: acetonitrile/water; neutral system: NH₄HCO₃), to give compound **WX009-3.** MS-ESI *m*/*z*: 349.2 [M+H]⁺, 350.2 [M+H+1]⁺, 351.2 [M+H+2]⁺. ¹H NMR (400 MHz, DMSO_*d₆*) δ: 10.31 (s, 1H), 7.48-7.40 (m, 2H), 7.29-7.25 (m, 1H), 7.25-7.21 (m, 2H), 7.20-7.15 (m, 2H), 4.02 (t, *J*=5.6 Hz, 2H), 2.70 (t, *J*=5.6 Hz, 2H), 2.26 (s, 6H), 2.14 (s, 3H).

### Step 4: Synthesis of compound WX009

Compound **WX009-3** (15 mg, 43.00 µmol) was dissolved in N,N-dimethylformamide (0.4 mL) at room temperature. Then compound **WX005-4** (12.22 mg, 47.30 µmol) and triethylamine (13.05 mg, 129.01 µmol, 17.96 µL) were added in sequence. The reaction mixture was stirred to react at room temperature for 12 hours. After the reaction was completed, the reaction solution was directly separated by preparative HPLC (mobile phase: acetonitrile/water; neutral system: NH₄HCO₃), to give the target compound **WX009.** MS-ESI *m*/*z*: 513.2 [M+H]⁺, 514.3 [M+H+1]⁺, 515.2 [M+H+2]⁺. ¹H NMR (400 MHz, DMSO_d₆) δ: 10.89 (s, 1H), 8.67 (s, 1H), 7.87 (s, 1H), 7.53 (d, *J*=8.0 Hz, 1H), 7.47 (s, 1H), 7.19 (d, *J*=8.4 Hz, 1H), 7.17 (d, *J*=2.0 Hz, 1H), 6.99 (d, *J*=1.6 Hz, 1H), 6.74 (t, *J*=5.8 Hz, 1H), 4.38 (d, *J*=6.0 Hz, 2H), 4.12 (dd, *J*=4.8, 12.0 Hz, 1H), 3.99 (t, *J*=5.6 Hz, 2H), 2.79-2.69 (m, 1H), 2.65 (t, *J*=5.8 Hz, 2H), 2.61-2.56 (m, 1H), 2.32-2.26 (m, 1H), 2.22 (s, 6H), 2.15-2.06 (m, 4H).

### Example 10

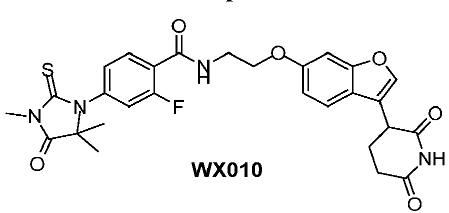

### Synthetic route:

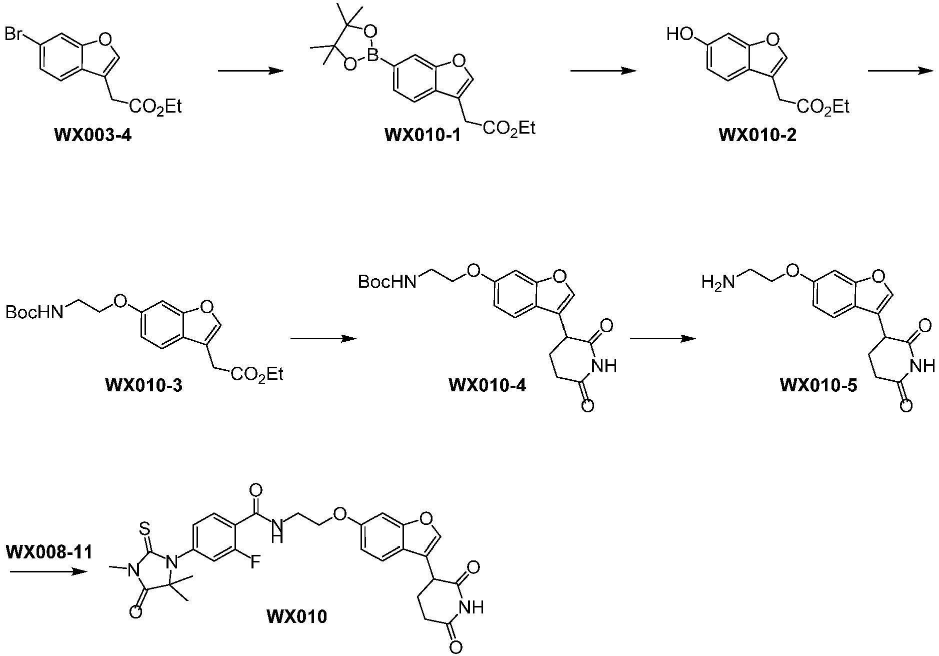

### Step 1: Synthesis of compound WX010-1

Compound **WX003-4** (3 g, 10.60 mmol), bis(pinacolato)diboron (3.23 g, 12.72 mmol), potassium acetate (4.16 g, 42.39 mmol), and [1,1-bis(diphenylphosphino)ferrocene]palladium dichloride dichloromethane (432.67 mg, 529.82 µmol) were dissolved in dioxane (50 mL) at room temperature under nitrogen. The reaction mixture was heated to 100°C and stirred to react for 5 hours. After the reaction was completed, the reaction solution was cooled to room temperature, and filtered directly through diatomaceous earth. The filter cake was rinsed with dichloromethane (30 mL×3), and the filtrate was concentrated under reduced pressure to remove the solvent. The resulting residue was separated by column chromatography (eluent: petroleum ether/ethyl acetate = 30/1, volume ratio) to give compound **WX010-1.**

### Step 2: Synthesis of compound WX010-2

Compound **WX010-1** (3.1 g, 9.39 mmol) and sodium bicarbonate (1.58 g, 18.78 mmol, 730.29 µL) were dissolved in a mixed solvent of tetrahydrofuran (40 mL) and water (20 mL) at room temperature, and the mixture was cooled to 0 °C. Hydrogen peroxide (7.15 g, 63.06 mmol, 6.06 mL, purity: 30%) was added dropwise, and the reaction mixture was stirred to react at 0 °C for 2 hours. After the reaction was completed, the reaction solution was quenched with 15% aqueous sulfurous acid solution (50 mL), stirred for 10 minutes, adjusted to pH of 5~6 with 1 N hydrochloric acid. The mixture was extracted with ethyl acetate (50 mL×3). The organic phase was washed with saturated brine (50 mL), dried over anhydrous sodium sulfate, and filtered. The filtrate was rotary evaporated to dryness. The resulting residue was separated by column chromatography (eluent: petroleum ether/ethyl acetate = 6/1, volume ratio) to give compound **WX010-2.**

### Step 3: Synthesis of compound WX010-3

Compound **WX010-2** (1 g, 4.54 mmol) was dissolved in tetrahydrofuran (20 mL) at room temperature under nitrogen. Then N-Boc-ethanolamine (1.10 g, 6.81 mmol, 1.06 mL) and azodicarbonyldipiperidine (2.29 g, 9.08 mmol) were added. The mixture was cooled to 0°C, and a solution of tributylphosphine (1.84 g, 9.08 mmol, 2.24 mL) in tetrahydrofuran (5 mL) was added dropwise. The reaction mixture was returned to room temperature and stirred to react for 3 hours. After the reaction was completed, the reaction solution was filtered directly, and the filter cake was rinsed with tetrahydrofuran (5 mL×3). The filtrate was collected, and concentrated under reduced pressure to remove the solvent. The resulting residue was separated by column chromatography (eluent: petroleum ether/ethyl acetate = 1/0-9/1, volume ratio) to give compound **WX010-3.** MS-ESI *m*/*z*: 308.1 [M-55]⁺. ¹H NMR (400 MHz, CDCl₃) δ: 7.54 (s, 1H), 7.44 (d, *J*=8.4 Hz, 1H), 7.00 (d, *J*=2.0 Hz, 1H), 6.89 (dd, *J*=2.2, 8.6 Hz, 1H), 5.03 (br s, 1H), 4.19 (q, *J*=7.0 Hz, 2H), 4.06 (t, *J*=5.0 Hz, 2H), 3.66 (d, *J*=1.2 Hz, 2H), 3.62-3.51 (m, 2H), 1.47 (s, 9H), 1.28 (t, *J*=7.0 Hz, 3H).

### Step 4: Synthesis of compound WX010-4

Compound **WX010-3** (1.1 g, 3.03 mmol) was dissolved in N,N-dimethylformamide (20 mL) at room temperature under nitrogen. Then acrylamide (215.15 mg, 3.03 mmol) and potassium tert-butoxide (509.48 mg, 4.54 mmol) were added simultaneously. The reaction mixture was stirred to react at room temperature for 1 hour. After the reaction was completed, the reaction solution was poured into water (50 mL), and the mixture was extracted with ethyl acetate (30 mL×3). The organic phases were combined, washed with semi-saturated brine (50 mL×3), dried over anhydrous sodium sulfate, and filtered. The filtrate was concentrated under reduced pressure to remove the solvent. The resulting residue was separated by column chromatography (eluent: petroleum ether/ethyl acetate = 4/1-1/1, volume ratio) to give compound **WX010-4.** ¹H NMR (400 MHz, CDCl₃) δ: 8.17 (br s, 1H), 7.49 (s, 1H), 7.36 (d, *J*=8.8 Hz, 1H), 7.02 (d, *J*=2.4 Hz, 1H), 6.89 (dd, *J*=2.2, 8.6 Hz, 1H), 5.03 (br s, 1H), 4.06 (t, *J*=5.0 Hz, 2H), 3.97 (t, *J*=7.4 Hz, 1H), 3.62-3.50 (m, 2H), 2.85-2.65 (m, 2H), 2.42-2.29 (m, 2H), 1.46 (s, 9H).

### Step 5: Synthesis of compound WX010-5 hydrochloride

Compound **WX010-4** (650 mg, 1.67 mmol) was dissolved in hydrochloric acid/ethyl acetate (4 M, 20 mL) at room temperature. The reaction mixture was stirred at room temperature for 15 minutes, and solids were precipitated. After the reaction was completed, the reaction solution was directly filtered. The filter cake was collected, and dried under reduced pressure to give compound **WX010-5** hydrochloride. MS-ESI *m*/*z*: 287.0 [M-H]⁻. ¹H NMR (400 MHz, DMSO_*d₆*) δ: 10.89 (s, 1H), 8.23 (br s, 3H), 7.81 (s, 1H), 7.49 (d, *J*=8.8 Hz, 1H), 7.25 (d, *J*=2.0 Hz, 1H), 6.94 (dd, *J*=2.2, 8.6 Hz, 1H), 4.23 (t, *J*=5.0 Hz, 2H), 4.10 (dd, *J*=4.8, 12.0 Hz, 1H), 3.27-3.17 (m, 2H), 2.81-2.66 (m, 1H), 2.62-2.52 (m, 1H), 2.37-2.23 (m, 1H), 2.16-2.05 (m, 1H).

### Step 6: Synthesis of compound WX010

Compound **WX010-5** hydrochloride (70 mg, 215.54 µmol) was dissolved in N,N-dimethylformamide (2 mL) at room temperature. Then compound **WX008-11** (70.26 mg, 237.10 µmol) was added, and O-(7-azabenzotriazol-1-yl)-*N,N,N',N*'-tetramethyluronium hexafluorophosphate (163.91 mg, 431.09 µmol) and triethylamine (87.24 mg, 862.18 µmol, 120.01 µL) were finally added. The reaction mixture was stirred to react at room temperature for 12 hours. After the reaction was completed, the reaction solution was poured into water (20 mL), and the mixture was extracted with ethyl acetate (20 mL×3). The organic phases were combined, dried over anhydrous sodium sulfate, and filtered. The filtrate was concentrated under reduced pressure to remove the solvent. The resulting residue was separated by preparative HPLC (mobile phase: acetonitrile/water; acidic system: HCl) to give the target compound **WX010.** MS-ESI *m*/*z*: 567.0 [M+H]⁺. ¹H NMR (400 MHz, DMSO_*d₆*) δ: 10.88 (s, 1H), 8.72 (t, *J*=4.8 Hz, 1H), 7.78 (s, 1H), 7.72 (t, *J*=8.0 Hz, 1H), 7.46 (d, *J*=8.4 Hz, 1H), 7.41 (dd, *J*=1.6, 10.8 Hz, 1H), 7.26 (dd, *J*=1.8, 8.2 Hz, 1H), 7.23 (d, *J*=2.0 Hz, 1H), 6.91 (dd, *J*=2.2, 8.6 Hz, 1H), 4.17 (t, *J*=5.6 Hz, 2H), 4.08 (dd, *J*=4.8, 11.6 Hz, 1H), 3.70-3.62 (m, 2H), 3.22 (s, 3H), 2.77-2.69 (m, 1H), 2.61-2.56 (m, 1H), 2.32-2.23 (m, 1H), 2.16-2.06 (m, 1H), 1.37 (s, 6H).

### Example 11

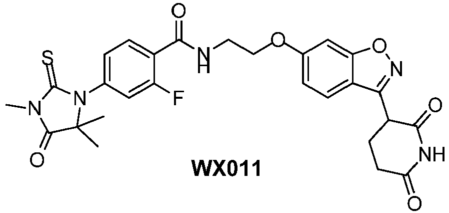

### Synthetic route:

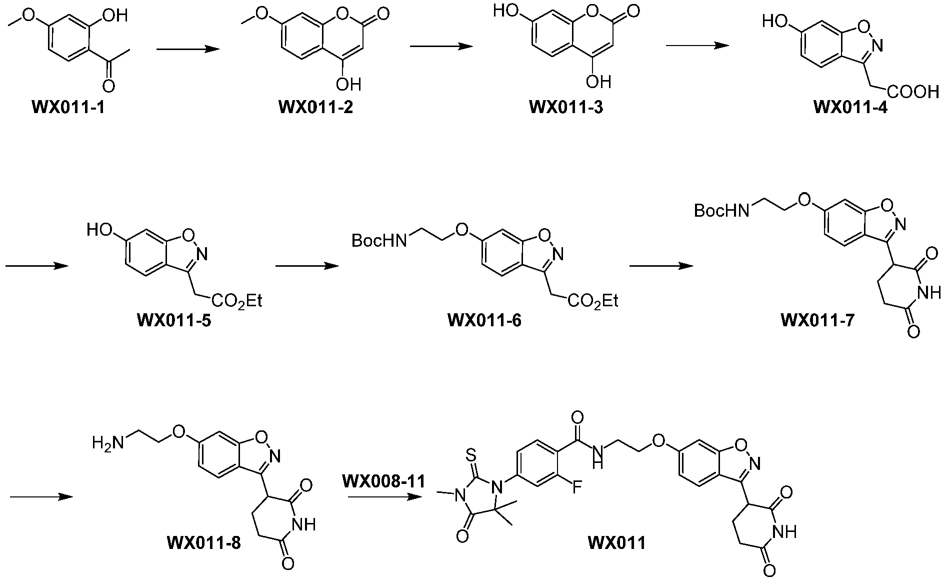

### Step 1: Synthesis of compound WX011-2

Compound **WX011-1** (10 g, 60.18 mmol) was dissolved in toluene (100 mL) at room temperature. Diethyl carbonate (48.75 g, 412.68 mmol, 50 mL) was then added. The mixture was stirred until the system was clear, and cooled to 0~5°C. Sodium hydride (12.03 g, 300.90 mmol, purity: 60%) was added in batches, and the reaction mixture was heated to 100°C and stirred to react for 12 hours. After the reaction was completed, the reaction solution was poured into water (500 mL), and the mixture was extracted with methyl tert-butyl ether (100 mL). The organic phase was discarded. The aqueous phase was adjusted to pH of 5~6 with 6 M dilute hydrochloric acid, and extracted with ethyl acetate/tetrahydrofuran (3:1) (500 mL×3). The organic phases were combined, dried over anhydrous sodium sulfate, and filtered. The filtrate was concentrated under reduced pressure to remove the solvent. The resulting residue was stirred with methyl tert-butyl ether (50 mL) at room temperature for 30 minutes, and solids were precipitated. The mixture was filtered. The solids were collected, and dried under reduced pressure to give compound **WX011-2.** MS-ESI m/z: 193.1 [M+H]⁺. ¹H NMR (400 MHz, DMSO_*d₆*) δ: 12.35 (br s, 1H), 7.71 (d, *J*=8.8 Hz, 1H), 6.97-6.89 (m, 2H), 5.44 (s, 1H), 3.85 (s, 3H).

### Step 2: Synthesis of compound WX011-3

Compound **WX011-2** (3 g, 15.61 mmol) was dissolved in dichloromethane (60 mL) at room temperature under nitrogen, and the mixture was cooled to -30°C under nitrogen. Boron tribromide (11.73 g, 46.83 mmol, 4.51 mL) was added dropwise, and the reaction mixture was returned to room temperature and stirred to react for 12 hours. After the reaction was completed, the reaction solution was slowly poured into ice water (200 mL). The mixture was concentrated under reduced pressure to remove the solvent, and extracted with (ethyl acetate/tetrahydrofuran = 3/1) (200 mL×3). The organic phases were combined, washed with saturated brine (50 mL×2), dried over anhydrous sodium sulfate, and filtered. The filtrate was concentrated under reduced pressure to remove the solvent. The resulting residue was stirred with methyl tert-butyl ether (20 mL) at room temperature for 30 minutes, and solids were precipitated. The mixture was filtered. The solids were collected, and dried under reduced pressure to give compound **WX011-3.** MS-ESI *m*/*z*: 179.0 [M+H]⁺. ¹H NMR (400 MHz, DMSO_*d₆*) δ: 12.22 (s, 1H), 10.51 (s, 1H), 7.63 (d, *J*=8.4 Hz, 1H), 6.76 (dd, *J*=2.2, 8.6 Hz, 1H), 6.66 (d, *J*=2.0 Hz, 1H), 5.38 (s, 1H).

### Step 3: Synthesis of compound WX011-4

Compound **WX011-3** (2.55 g, 14.31 mmol) was dissolved in ethanol (50 mL) at room temperature under nitrogen. Then hydroxylamine hydrochloride (3.48 g, 50.10 mmol) was added, and sodium ethoxide (3.41 g, 50.10 mmol) was finally added. The reaction mixture was heated to 80°C and stirred to react for 12 hours. After the reaction was completed, the reaction solution was poured into water (100 mL), and the mixture was adjusted to pH of 4~5 with 2 M dilute hydrochloric acid, and extracted with ethyl acetate (100 mL×3). The organic phases were combined, dried over anhydrous sodium sulfate, and filtered. The filtrate was concentrated under reduced pressure to give compound **WX011-4.** MS-ESI *m*/*z*: 194.1 [M+H]⁺. ¹H NMR (400 MHz, DMSO_*d₆*) δ: 12.77 (s, 1H), 10.34 (s, 1H), 7.58 (d, *J*=8.4 Hz, 1H), 6.93 (d, *J*=1.6 Hz, 1H), 6.85 (dd, *J*=2.0, 8.4 Hz, 1H), 3.97 (s, 2H).

### Step 4: Synthesis of compound WX011-5

Compound **WX011-4** (2.7 g, 13.98 mmol) was dissolved in ethanol (30 mL) at room temperature under nitrogen. Then concentrated sulfuric acid (920.00 mg, 9.19 mmol, 0.5 mL, purity: 98%) was added. The reaction mixture was heated to 60°C and stirred to react for 16 hours. After the reaction was completed, the reaction solution was directly concentrated under reduced pressure to remove the solvent, and the resulting residue was separated by column chromatography (eluent: petroleum ether/ethyl acetate = 1/0-4/1, volume ratio) to give compound **WX011-5.** MS-ESI *m*/*z*: 222.1 [M+H]⁺. ¹H NMR (400 MHz, CDCl₃) δ: 7.53 (d, *J*=8.8 Hz, 1H), 6.94 (d, *J*=2.0 Hz, 1H), 6.84 (dd, *J*=2.0, 8.4 Hz, 1H), 5.94 (s, 1H), 4.23 (q, *J*=7.0 Hz, 2H), 3.99 (s, 2H), 1.28 (t, *J*=7.2 Hz, 3H).

### Step 5: Synthesis of compound WX011-6

Compound **WX011-5** (1 g, 4.52 mmol) was dissolved in tetrahydrofuran (20 mL) at room temperature under nitrogen. Then N-Boc-ethanolamine (1.09 g, 6.78 mmol, 1.05 mL) and azodicarbonyldipiperidine (2.28 g, 9.04 mmol) were added. The mixture was cooled to 0°C under nitrogen. A solution of tributylphosphine (1.83 g, 9.04 mmol, 2.23 mL) in 5 mL of tetrahydrofuran was added dropwise. The reaction mixture was returned to room temperature and stirred to react for 12 hours. After the reaction was completed, the reaction solution was directly concentrated under reduced pressure to remove the solvent. The resulting residue was separated by column chromatography (eluent: petroleum ether/ethyl acetate = 1/0-9/1, volume ratio) to give compound **WX011-6.** MS-ESI *m*/*z*: 365.2 [M+H]⁺. ¹H NMR (400 MHz, CDCl₃) δ: 7.56 (d, *J*=8.8 Hz, 1H), 6.99 (d, *J*=2.0 Hz, 1H), 6.93 (dd, *J*=2.0, 8.8 Hz, 1H), 5.01 (br s, 1H), 4.21 (q, *J*=7.2 Hz, 2H), 4.09 (t, *J*=5.0 Hz, 2H), 3.98 (s, 2H), 3.64-3.55 (m, 2H), 1.46 (s, 9H), 1.26 (t, *J*=7.2 Hz, 3H).

### Step 6: Synthesis of compound WX011-7

Compound **WX011-6** (500 mg, 1.37 mmol) was dissolved in tetrahydrofuran (10 mL) at room temperature under nitrogen. Then acrylamide (97.53 mg, 1.37 mmol) and potassium tert-butoxide (230.95 mg, 2.06 mmol) were added simultaneously. The reaction mixture was stirred to react at room temperature for 1 hour. After the reaction was completed, water (50 mL) was added to the reaction solution, and the mixture was extracted with ethyl acetate (50 mL×3). The organic phases were combined, dried over anhydrous sodium sulfate, and filtered. The filtrate was concentrated under reduced pressure to remove the solvent. The resulting residue was separated by column chromatography (eluent: petroleum ether/ethyl acetate = 4/1-1/1, volume ratio) to give compound **WX011-7.** ¹H NMR (400 MHz, CDCl₃) δ: 8.11 (br s, 1H), 7.57 (d, *J*=8.8 Hz, 1H), 7.01 (d, *J*=2.0 Hz, 1H), 6.95 (dd, *J*=2.0, 8.8 Hz, 1H), 5.00 (br s, 1H), 4.28 (dd, *J*=5.2, 8.4 Hz, 1H), 4.10 (t, *J*=5.2 Hz, 2H), 3.66-3.51 (m, 2H), 3.09-2.96 (m, 1H), 2.81-2.71 (m, 1H), 2.66-2.55 (m, 1H), 2.49-2.40 (m, 1H), 1.47 (s, 9H).

### Step 7: Synthesis of compound WX011-8 hydrochloride

Compound **WX011-7** (240 mg, 616.33 µmol) was dissolved in hydrochloric acid/ethyl acetate (4 M, 4.99 mL) at room temperature. The reaction mixture was stirred at room temperature for 15 minutes, and solids were precipitated. After the reaction was completed, the reaction solution was directly filtered. The filter cake was collected, and dried under reduced pressure to give compound **WX011-8** hydrochloride. MS-ESI *m*/*z*: 290.2 [M+H]⁺. ¹H NMR (400 MHz, DMSO_*d₆*) δ: 11.08 (s, 1H), 8.27 (br s, 3H), 7.75 (d, *J*=8.8 Hz, 1H), 7.38 (d, *J*=2.0 Hz, 1H), 7.04 (dd, *J*=2.0, 8.8 Hz, 1H), 4.54 (dd, *J*=5.0, 11.8 Hz, 1H), 4.31 (t, *J*=5.0 Hz, 2H), 3.30-3.20 (m, 2H), 2.82-2.71 (m, 1H), 2.65-2.56 (m, 1H), 2.49-2.41 (m, 1H), 2.24-2.14 (m, 1H).

### Step 8: Synthesis of compound WX011

Compound **WX011-8** hydrochloride (70 mg, 214.89 µmol) was dissolved in N,N-dimethylformamide (2 mL) at room temperature. Then compound **WX008-11** (70.04 mg, 236.38 µmol) was added, and O-(7-azabenzotriazol-1-yl)-*N,N,N',N*'-tetramethyluronium hexafluorophosphate (163.42 mg, 429.78 µmol) and triethylamine (86.98 mg, 859.56 µmol, 119.64 µL) were finally added. The reaction mixture was stirred to react at room temperature for 12 hours. After the reaction was completed, the reaction solution was filtered directly, and the filtrate was separated by preparative HPLC (mobile phase: acetonitrile/water; acidic system: HCl), to give the target compound **WX011.** MS-ESI *m*/*z*: 567.9 [M+H]⁺. ¹H NMR (400 MHz, DMSO_*d₆*) δ: 11.08 (s, 1H), 8.75 (t, *J*=6.2 Hz, 1H), 7.76-7.68 (m, 2H), 7.41 (dd, *J*=1.4, 11.0 Hz, 1H), 7.36 (d, *J*=1.6 Hz, 1H), 7.27 (dd, *J*=1.8, 8.2 Hz, 1H), 7.00 (dd, *J*=2.0, 8.8 Hz, 1H), 4.52 (dd, *J*=5.2, 12.0 Hz, 1H), 4.24 (t, *J*=5.4 Hz, 2H), 3.70 (q, *J*=5.8 Hz, 2H), 3.22 (s, 3H), 2.81-2.71 (m, 1H), 2.64-2.55 (m, 1H), 2.47-2.40 (m, 1H), 2.25-2.14 (m, 1H), 1.37 (s, 6H).

### Example 12

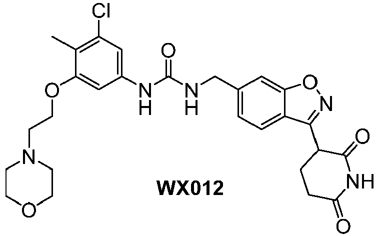

### Synthetic route:

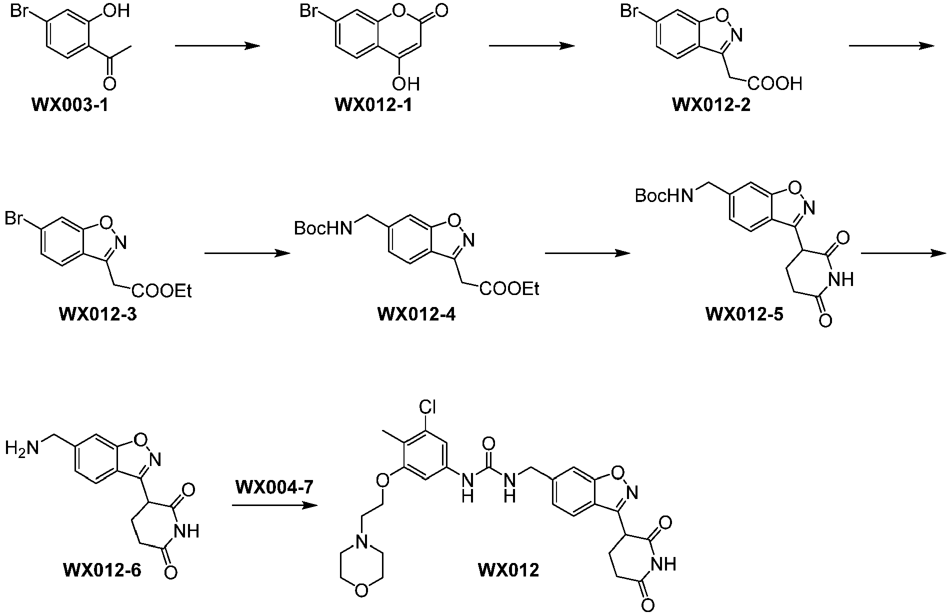

### Step 1: Synthesis of compound WX012-1

Compound **WX003-1** (25 g, 116.26 mmol) was dissolved in tetrahydrofuran (250 mL) at room temperature under nitrogen. Then dimethyl carbonate (31.42 g, 348.77 mmol, 29.36 mL) was added, and the mixture was stirred until the system was clear. The mixture was cooled to 5~15°C, and potassium tert-butoxide (78.27 g, 697.53 mmol) was added in batches. The reaction mixture was heated to 80°C and stirred to react for 12 hours. After the reaction was completed, the reaction solution was poured into water (500 mL), and the mixture was extracted with methyl tert-butyl ether (100 mL). The organic phase was discarded. The aqueous phase was adjusted to pH of 5~6 with 6 M hydrochloric acid, and solids were precipitated. The mixture was filtered. The solids were collected, and dried under reduced pressure to give compound **WX012-1.** MS-ESI *m*/*z*: 241.0 [M+H]⁺, 243.1 [M+H]⁺. ¹H NMR (400 MHz, DMSO_*d₆*) δ: 12.71 (s, 1H), 7.72 (d, *J*=8.4 Hz, 1H), 7.67 (d, *J*=2.0 Hz, 1H), 7.52 (dd, *J*=1.8, 8.6 Hz, 1H), 5.61 (s, 1H).

### Step 2: Synthesis of compound WX012-2

Compound **WX012-1** (24 g, 99.57 mmol) was dissolved in ethanol (300 mL) at room temperature. Then hydroxylamine hydrochloride (24.22 g, 348.49 mmol) was added, and sodium acetate (28.59 g, 348.49 mmol) was finally added. The reaction mixture was heated to 80°C and stirred to react for 12 hours. After the reaction was completed, the reaction solution was poured into water (1000 mL). The mixture was adjusted to pH of 4~5 with 6 M dilute hydrochloric acid, concentrated under reduced pressure to remove part of the ethanol, and extracted with ethyl acetate (500 mL×3). The organic phases were combined, dried over anhydrous sodium sulfate, and filtered. The filtrate was concentrated under reduced pressure to give compound **WX012-2.** MS-ESI *m*/*z*: 256.0 [M+H]⁺, 258.0 [M+H]⁺. ¹H NMR (400 MHz, DMSO_*d₆*) δ: 12.65 (s, 1H), 8.11 (d, *J*=1.2 Hz, 1H), 7.82 (d, *J*=8.4 Hz, 1H), 7.59 (dd, *J*=1.4, 8.6 Hz, 1H), 4.12 (s, 2H).

### Step 3: Synthesis of compound WX012-3

Compound **WX012-2** (24 g, 93.73 mmol) was dissolved in ethanol (200 mL) at room temperature under nitrogen. Then concentrated sulfuric acid (5.52 g, 55.15 mmol, 3 mL, purity: 98%) was added. The reaction mixture was heated to 60°C and stirred to react for 12 hours. After the reaction was completed, the reaction solution was concentrated under reduced pressure to remove the solvent. The resulting residue was separated by column chromatography (eluent: petroleum ether/ethyl acetate = 1/0-17/3, volume ratio) to give compound **WX012-3.** MS-ESI *m*/*z*: 284.0 [M+H]⁺, 286.0 [M+H]⁺. ¹H NMR (400 MHz, CDCl₃) δ: 7.78 (d, *J*=1.2 Hz, 1H), 7.59 (d, *J*=8.4 Hz, 1H), 7.46 (dd, *J*=1.6, 8.4 Hz, 1H), 4.22 (q, *J*=7.0 Hz, 2H), 4.03 (s, 2H), 1.27 (t, *J*=7.2 Hz, 3H).

### Step 4: Synthesis of compound WX012-4

Compound **WX012-3** (0.5 g, 1.76 mmol) was dissolved in water (2 mL) and toluene (10 mL) at room temperature under nitrogen. Then N-Boc-aminomethyl potassium trifluoroborate (1.25 g, 5.28 mmol), cesium carbonate (1.72 g, 5.28 mmol), [(bis(1-adamantyl)-N-butylphosphino)-2-(2-aminobiphenyl)palladium(II) chloride (117.67 mg, 175.99 µmol) were added in sequence. The reaction mixture was heated to 105°C and stirred to react for 12 hours. After the reaction was completed, the reaction solution was poured into water (20 mL), and the mixture was extracted with ethyl acetate (30 mL×3). The organic phases were combined, dried over anhydrous sodium sulfate, and filtered. The filtrate was concentrated under reduced pressure to remove the solvent. The resulting residue was separated by column chromatography (eluent: petroleum ether/ethyl acetate = 1/0-41/9, volume ratio) to give compound **WX012-4.** MS-ESI *m*/*z*: 335.2 [M+H]⁺. ¹H NMR (400 MHz, CDCl₃) δ: 7.66 (d, *J*=8.0 Hz, 1H), 7.50 (s, 1H), 7.27 (d, *J*=7.6 Hz, 1H), 5.01 (s, 1H), 4.48 (d, *J*=6.0 Hz, 2H), 4.22 (q, *J*=7.2 Hz, 2H), 4.03 (s, 2H), 1.48 (s, 9H), 1.26 (t, *J*=7.0 Hz, 3H).

### Step 5: Synthesis of compound WX012-5

Compound **WX012-4** (600 mg, 1.79 mmol) was dissolved in tetrahydrofuran (12 mL) at room temperature under nitrogen. Then acrylamide (127.54 mg, 1.79 mmol) and potassium tert-butoxide (201.36 mg, 1.79 mmol) were added simultaneously. The reaction mixture was stirred to react at room temperature for 0.5 hours. After the reaction was completed, the reaction solution was poured into a mixture solution of water (20 mL) and ethyl acetate (20 mL). The layers were separated. The organic phases were collected, and the aqueous phase was extracted with ethyl acetate (20 mL×3). The organic phases were combined, washed with saturated brine (50 mL), dried over anhydrous sodium sulfate, and filtered. The filtrate was concentrated under reduced pressure to remove the solvent. The resulting residue was separated by column chromatography (eluent: petroleum ether/ethyl acetate = 4/1-1/1, volume ratio) to give compound **WX012-5.** ¹H NMR (400 MHz, CDCl₃) δ: 8.14 (s, 1H), 7.66 (d, *J*=8.0 Hz, 1H), 7.53 (s, 1H), 7.26 (d, *J*=7.6 Hz 1H), 5.03 (s, 1H), 4.48 (d, *J*=5.6 Hz, 2H), 4.32 (dd, *J*=5.2, 8.8 Hz, 1H), 3.07-2.95 (m, 1H), 2.82-2.71 (m, 1H), 2.68-2.56 (m, 1H), 2.51-2.40 (m, 1H), 1.48 (s, 9H).

### Step 6: Synthesis of compound WX012-6 hydrochloride

Compound **WX012-5** (390 mg, 1.09 mmol) was dissolved in hydrochloric acid/ethyl acetate (4 M, 15 mL) at room temperature, and the reaction mixture was stirred at room temperature to react for 15 minutes. After the reaction was completed, the reaction solution was directly filtered. The filter cake was collected, and dried under reduced pressure to give compound **WX012-6** hydrochloride. MS-ESI *m*/*z*: 260.2 [M+H]⁺. ¹H NMR (400 MHz, DMSO_*d₆*) δ: 11.11 (s, 1H), 8.55 (br s, 3H), 7.93-7.88 (m, 2H), 7.52 (d, *J*=8.4 Hz, 1H), 4.63 (dd, *J*=5.2, 12.0 Hz, 1H), 4.26-4.17 (m, 2H), 2.85-2.73 (m, 1H), 2.66-2.53 (m, 2H), 2.26-2.16 (m, 1H).

### Step 7: Synthesis of compound WX012

Compound **WX004-7** (50 mg, 127.92 µmol) and compound **WX012-6** hydrochloride (41.61 mg, 140.71 µmol) were dissolved in N,N-dimethylformamide (1 mL) at room temperature. Then triethylamine (38.83 mg, 383.76 µmol, 53.41 µL) was added. The reaction mixture was stirred to react at room temperature for 12 hours. After the reaction was completed, the reaction solution was directly filtered. The resulting filtrate was separated by preparative HPLC (mobile phase: acetonitrile/water; neutral system: NH₄HCO₃), to give the target compound **WX012.** MS-ESI *m*/*z*: 556.0 [M+H]⁺, 557.0 [M+H+1]⁺, 558.0 [M+H+2]⁺. ¹H NMR (400 MHz, DMSO_*d₆*) δ: 11.10 (s, 1H), 8.77 (s, 1H), 7.80 (d, *J*=8.8 Hz, 1H), 7.61 (s, 1H), 7.33 (dd, *J*=1.2, 9.2 Hz, 1H), 7.16 (d, *J*=2.0 Hz, 1H), 7.02 (d, *J*=2.0 Hz, 1H), 6.86 (t, *J*=6.2 Hz, 1H), 4.58 (dd, *J*=5.0, 12.2 Hz, 1H), 4.45 (d, *J*=5.6 Hz, 2H), 4.03 (t, *J*=5.6 Hz, 2H), 3.59-3.55 (m, 4H), 2.83-2.73 (m, 1H), 2.71 (t, *J*=5.6 Hz, 2H), 2.65-2.56 (m, 1H), 2.48-2.44 (m, 4H), 2.24-2.15 (m, 2H), 2.10 (s, 3H).

### Example 13

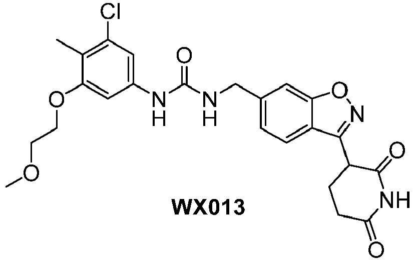

### Synthetic route:

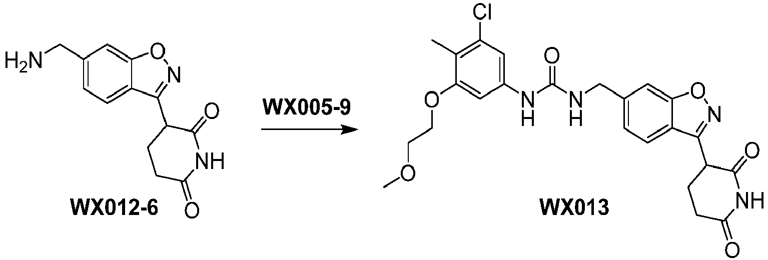

### Synthesis of compound WX013

Compound **WX005-9** (50 mg, 148.91 µmol) and compound **WX012-6** hydrochloride (48.44 mg, 163.80 µmol) were dissolved in N,N-dimethylformamide (1 mL) at room temperature. Then triethylamine (45.20 mg, 446.72 µmol, 62.18 µL) was added. The reaction mixture was stirred to react at room temperature for 2 hours. After the reaction was completed, the reaction solution was directly filtered, and the resulting filtrate was separated by preparative HPLC (mobile phase: acetonitrile/water; acidic system: HCl), to give the target compound **WX013.** MS-ESI *m*/*z*: 501.0 [M+H]⁺, 501.9 [M+H+1]⁺, 503.0 [M+H+2]⁺. ¹H NMR (400 MHz, DMSO_*d₆*) δ: 11.10 (s, 1H), 8.78 (s, 1H), 7.80 (d, *J*=8.4 Hz, 1H), 7.61 (s, 1H), 7.33 (d, *J*=8.4 Hz, 1H), 7.18 (d, *J*=1.6 Hz, 1H), 7.00 (d, *J*=1.6 Hz, 1H), 6.86 (t, *J*=5.8 Hz, 1H), 4.58 (dd, *J*=4.8, 12.0 Hz, 1H), 4.45 (d, *J*=6.0 Hz, 2H), 4.03 (t, *J*=4.6 Hz, 2H), 3.67 (t, *J*=4.6 Hz, 2H), 3.32 (s, 3H), 2.83-2.72 (m, 1H), 2.65-2.52 (m, 2H), 2.23-2.15 (m, 1H), 2.11 (s, 3H).

### Example 14

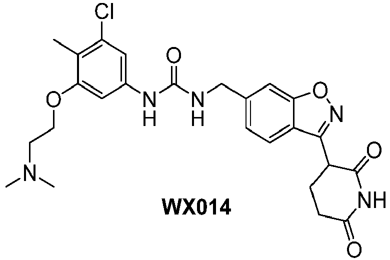

### Synthetic route:

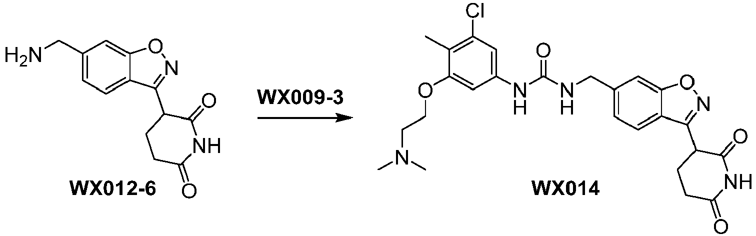

### Synthesis of compound WX014

Compound **WX009-3** (30 mg, 86.00 µmol) and compound **WX012-6** hydrochloride (27.98 mg, 94.60 µmol) were dissolved in N,N-dimethylformamide (1 mL) at room temperature. Then triethylamine (26.11 mg, 258.01 µmol, 35.91 µL) was added. The reaction mixture was stirred to react at room temperature for 2 hours. After the reaction was completed, the reaction solution was directly filtered, and the filtrate was collected. The resulting filtrate was separated by preparative HPLC (mobile phase: acetonitrile/water; neutral system: NH₄HCO₃), to give the target compound **WX014.** MS-ESI *m*/*z*: 514.0 [M+H]⁺, 515.0 [M+H+1]⁺, 516.0 [M+H+2]⁺. ¹H NMR (400 MHz, DMSO_*d₆*) δ: 11.10 (s, 1H), 8.77 (s, 1H), 7.80 (d, *J*=8.0 Hz, 1H), 7.61 (s, 1H), 7.33 (d, *J*=8.4 Hz, 1H), 7.17 (s, 1H), 7.00 (s, 1H), 6.85 (t, *J*=5.8 Hz, 1H), 4.58 (dd, *J*=4.8, 12.0 Hz, 1H), 4.45 (d, *J*=6.0 Hz, 2H), 3.99 (t, *J*=5.6 Hz, 2H), 2.85-2.72 (m, 1H), 2.70-2.60 (m, 4H), 2.22 (s, 6H), 2.19-2.15 (m, 1H), 2.10 (s, 3H).

### Example 15 and Example 16

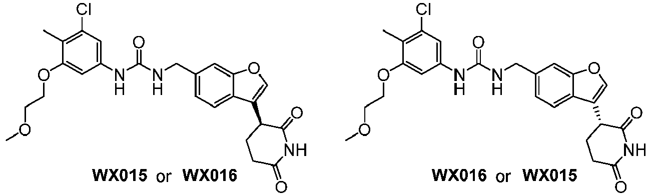

### Synthetic route:

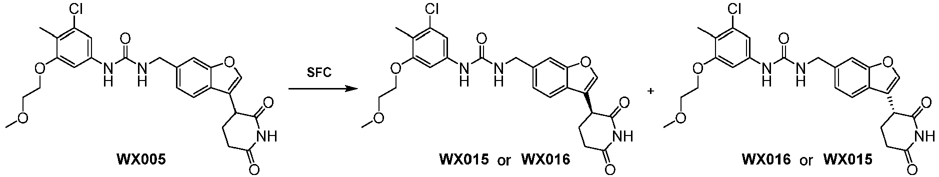

### Synthesis of compound WX015 or WX016

Compound **WX005** (100 mg, 200.02 µmol) was separated by supercritical fluid chromatography (Separation conditions, column: DAICEL CHIRALPAK AD (250 mm*30 mm, 10 µm); mobile phase: A: CO₂; B: IPA; B%: 55%-55%, 8 min). The sample with a retention time of 1.711 min was collected and separated by preparative HPLC (mobile phase: acetonitrile/water; acidic system: HCl) to give the target compound **WX015** (ee%: 98.68%). The sample with a retention time of 2.135 min was collected and separated by preparative HPLC (mobile phase: acetonitrile/water; acidic system: HCl) to give the target compound **WX016** (ee%: 100.0%). SFC analysis method, column: Chiralpak AD-3, 50×4.6 mm ID, 3 µm; mobile phase: A: CO₂; B: IPA (0.1%IPAm, v/v).

Compound **WX015.** MS-ESI *m*/*z*: 500.1 [M+H]⁺, 501.2 [M+H+1]⁺, 502.2 [M+H+2]⁺. ¹H NMR (400 MHz, DMSO_*d₆*) δ: 10.89 (s, 1H), 8.67 (s, 1H), 7.86 (s, 1H), 7.53 (d, *J*=4.0 Hz, 1H), 7.47 (s, 1H), 7.20 (dd, *J*=1.2, 8.4 Hz, 1H), 7.17 (d, *J*=2.0 Hz, 1H), 6.99 (d, *J*=1.6 Hz, 1H), 6.73 (t, *J*=6.0 Hz, 1H), 4.39 (d, *J*=6.0 Hz, 2H), 4.12 (dd, *J*=4.8, 12.0 Hz, 1H), 4.06-4.01 (m, 2H), 3.70-3.65 (m, 2H), 3.32 (s, 3H), 2.81-2.69 (m, 1H), 2.61-2.53 (m, 1H), 2.38-2.27 (m, 1H), 2.16-2.05 (m, 4H).

Compound **WX016.** MS-ESI *m*/*z*: 500.2 [M+H]⁺, 501.1 [M+H+1]⁺, 502.1 [M+H+2]⁺. ¹H NMR (400 MHz, DMSO_*d₆*) δ: 10.90 (s, 1H), 8.68 (s, 1H), 7.87 (s, 1H), 7.53 (d, *J*=8.0 Hz, 1H), 7.48 (s, 1H), 7.23-7.15 (m, 2H), 6.99 (s, 1H), 6.74 (t, *J*=5.8 Hz, 1H), 4.38 (d, *J*=5.6 Hz, 2H), 4.12 (dd, *J*=4.8, 12.0 Hz, 1H), 4.06-4.00 (m, 2H), 3.71-3.63 (m, 2H), 3.32 (s, 3H), 2.82-2.68 (m, 1H), 2.61-2.54 (m, 1H), 2.37-2.25 (m, 1H), 2.15-2.05 (m, 4H).

### Biological assays

### Assay example 1: Effect of degrading GSPT1 protein

BxPC-3 cells were treated with 10 µM, 1 µM and 0.1 µM of the test compound respectively for 24 hours. The cells were collected and lysed, denatured at 100 °C for 20 minutes, and then subjected to Western blot analysis. β-actin was used as the internal reference protein to detect the protein level of GSPT1. The main steps included: 1) Electrophoresis: Biorad precast gel was used to separate proteins using SDS-PAGE electrophoresis; 2) Transfer to membrane: Biorad rapid transfer to membrane system was used to transfer the proteins on the gel to PVDF membrane; 3) Blocking: the PVDF membrane with Western blotting was placed into an antibody incubation box, and blocking solution was added to completely cover the PVDF membrane for incubation at room temperature for 1 hour; 4) Incubation of the primary antibody: the above PVDF membrane was covered with GSPT1 antibody diluent, and incubated on a shaker at 4°C overnight, 5) Incubation of the secondary antibody: the membrane was washed three times with Wash buffer, and secondary antibody diluent (Anti-rabbit IgG, HRP-linked) was added for incubation on a shaker at room temperature for 1 hour; 5) Exposure: the membrane was washed three times with Wash buffer, and the band on the membrane was detected using chemiluminescence method (Clarity Western ECL Substrate).

### Instruments, assay materials and buffer formulations

### (1) Cells

See Table 1 for details.

**Table 1. Cells**

| Cell name | Category | Cell source | Item No. |
|---|---|---|---|
| BxPC-3 | Human pancreatic cancer tumor cells | ATCC | CRL-1687 |

### (2) Reagents

See Table 2 for details.

**Table 2. Reagents**

| Reagent name | Source | Item No. |
|---|---|---|
| 10x Tris/Glycine/SDS buffer | Biorad | 1610732 |
| 10× TBS | Biorad | 1706435 |
| Criterion TGX Precast Gel | Biorad | 5671085 |
| Trans-Blot Turbo RTA Midi LF PVDF Transfer Kit | Biorad | 1704275 |
| Precision Plus Protei Dual Color Standards | Biorad | 1610374 |
| Clarity Western ECL Substrate | Biorad | 1705060 |
| Tween-20 | Sigma | P1379-500ML |
| Pierce BCA Protein Assay Kit | Thermo | 23225 |
| DTT | Roche | 10708984001 |
| 4× Laemmli loaing buffer | Biorad | 1610747 |
| BSA | absin | abs49001012b |
| Methanol | OKA | 10014191 |

Protein loading buffer: 4 × Laemmli loading buffer was diluted 4 times with deionized water and DTT was added;
Electrophoresis solution: 10x Tris/Glycine/SDS buffer was diluted 10 times with deionized water;
Solution for transfer to membrane: Prepared by diluting Trans-Blot Turbo RTA Midi LF PVDF Transfer Kit with methanol;
Blocking solution: 5% BSA.
Wash buffer: 0.2% Tween-20 TBS buffer.

The primary and secondary antibody dilutions were prepared by diluting with blocking solution.

### (3) Antibodies

See Table 3 for details.

**Table 3. Antibodies**

| Reagent Name | Species | Dilution Ratio | Source |
|---|---|---|---|
| Anti-GSPT1 | R | 1:2000 | Abcam, ab49878 |
| Anti-β-actin | R | 1:2000 | CST, #4970 |
| Anti-rabbit IgG, HRP-linked | - | 1:2000 | CST, # 7074 |

### (4) Instruments

Biorad PowerPac Basic Power Supply electrophoresis system
Biorad Mini-PROTEAN^{®} Tetra Cell small vertical electrophoresis tank
BioRad Trans-Blot Turbo rapid transfer system
ChemiDoc Imaging System imager
The detailed results of screening are shown in FIG. **1****.**

**Conclusion:** The compound WX001 of the present disclosure has a significant effect of promoting the degradation of GSPT1 protein in BxPC-3 cells.

### Assay example 2: Evaluation of target protein degradation in HEK293T-LgBiT_GSPT1-nHiBiT cells

**Assay purpose:** This assay was performed to detect the degradation effect of the test compound on the target protein GSPT1 in HEK293T-LgBiT cells.

### Assay Materials:

### 1. Cells and culture media

Cell: HEK293T-LgBiT_GSPT1-nHiBiT polyclone
Medium: DMEM + 10% FBS + 2mM GlutaMax + 1mM sodium pyruvate + 1X penicillin/streptomycin
Positive control: 1000 nM; Negative control: 0.1 % DMSO

### 2. Reagents and consumables

See Table 4 for details.

**Table 4. Reagents and consumables**

| Reagent or consumable | Manufacturer | Item No. |
|---|---|---|
| DMEM | Gibco | 31331093 |
| Serum (FBS) | Biosera | #FB-1058/500 |
| P/S double antibody | Biosera | #LM-A4118 |
| DPBS | Invitrogen | #14190 |
| 0.25% Trypsin-EDTA | Invitrogen | #25200 |
| Glutamine | Invitrogen | Cat# 35050061 |
| Sodium pyruvate | Invitrogen | Cat# 11360070 |
| Nano-Glo^{®} HiBiT Assay Kit | Promega | promega# N3040 |
| 384-well whiteboard, flat bottom | Corning | Cat# 3570 |
| 384_LDV compound plate | Labcyte | Cat# LP-0200 |

### 3. Instruments

See Table 5 for details.

**Table 5. Instruments**

| Name | Manufacturer | Instrument model |
|---|---|---|
| ECHO pipette | Labcyte | Echo 550 |
| Bravo automatic liquid workstation | Agilent | 16050-101 |
| Envision plate reader | Perkin Elmer | 2104 |
| Automatic sampler | Thermofisher | Multidrop Combi |
| Cell counter | Thermo | Countess II FL |

### Assay program:

### Day 1

### 1. Preparation of compounds

(1). Dissolving the powder of the test compound with DMSO to 10 mM as the storage concentration, and using a pipette to manually draw 9 µL of 10 mM or 3 mM test compound to columns 1 and 13 of a LDV plate;
(2). Using multidrop Combi to add 6 µL of DMSO to columns 2-12 and 14-24;
(3). Using Bravo to dilute the test compound 3 times (3 µL+6 µL) from columns 1 to 11 and 13 to 23;
(4). According to the layout of the plate, using Echo to transfer 25 nL of compound solution (columns 1-24 of the LDV plate) to the assay plate;
(5). Using Echo to transfer 25 nL of 1 mM positive control solution to the assay plate as a 100% degradation control (i.e. LC, HPE), and to transfer 25 nL of DMSO to the assay plate as a 0% control (i.e. HC, ZPE).

### 2. Cell plating

(1). Discarding the cell culture medium, and washing the cells once with DPBS; then the cells were trypsinized, counted, and used to prepare a cell suspension of 4×10⁵ cells/mL;
(2). Using MultiDrop Combi to add 25 µL/well of cell suspension at medium speed to the assay plate containing the test compound;
(3). Putting the assay plate containing cells back into the incubator and culturing it at 37 °C and 5% CO₂ for 16 to 18 hours.

### Day 2

(1). Using MultiDrop Combi to add 25 µL/well of detection reagent (NanoGlo lysis solution + substrate + LgBit protein) to the assay plate at high speed, and shaking for 10 minutes;
(2). Centrifuging at 2000 rpm × 1 min to remove bubbles;
(3). Reading the plate using Envision, US Luminescence detection method.

### 3. Data analysis

The following formula was used to calculate the degradation rate (DR) of the test compound: DR (%) = (RLU of vehicle control - RLU of compound) / (RLU of vehicle control - RLU of positive control) * 100%, wherein the vehicle control was a blank control. The degradation rates of compounds at different concentrations were calculated in Excel, and then XLFit software was used to obtain inhibition curves and to calculate relevant parameters, including the minimum degradation rate, maximum degradation rate and DC₅₀.

The test results are shown in Table 6.

**Table 6 Target protein degradation effect of compounds of the present disclosure in HEK293T-LgBiT_GSPT1-nHiBiT cells**

| Compound | DC₅₀ (nm) | Max. DR(%) |
|---|---|---|
| **WX005** | 2.51 | 100.32% |
| **WX006** | 1.94 | 99.92% |
| **WX012** | 1.18 | 99.85% |
| **WX013** | 1.07 | 100.36% |

**Conclusion:** The compounds of the present disclosure exhibit excellent target protein degradation in HEK293T-LgBiT_GSPT1-nHiBiT cells.

### Assay example 3: Evaluation of anti-proliferative effects in tumor cell lines MV4-11 and MDA-MB-231

**Assay purpose:** This assay studied the inhibitory effect of the compounds on cell proliferation by detecting the effect of the test compounds on the in vitro activity of tumor cells **MV4-11** and **MDA-MB-231.**

### Assay Materials:

### 1. Cell lines and culture methods

See Table 7 for details.

**Table 7. Cell lines and culture methods**

| Cell line | Tumor type | Growth characteristics | Culture method |
|---|---|---|---|
| MV4-11 | Leukemia | Suspending | RPMI1640 + 10% FBS |
| MDA-MB-231 | Breast cancer | Adhering to the wall | Leibovitz's L-15+10% FBS (0% CO₂) |

### 2. Culture media and reagents

See Table 8 for details.

**Table 8. Culture media and reagents**

| Culture medium or reagent | Manufacturer | Item No. |
|---|---|---|
| RPMI 1640 | GIBCO | 22400-089 |
| Leibovitz's L-15 | SIGMA | L1518 |
| Dulbecco's PBS | Hyclone | SH30256.01 |
| FBS | Hyclone | SY30087.03 |
| Antibiotic-antimycotic | GIBCO | 15240-062 |
| 0.25% Trypsin | GIBCO | 25200072 |
| DMSO | SIGMA | D2650 |

### 3. Multi-well plate

Greiner CELLSTAR^{®} 96-well plate, flat bottom black plate (with lid and transparent bottom), # 655090.

### 4. Reagents and instruments used in the cell viability assay

(1) Promega CellTiter-Glo Luminescence Cell Viability Assay Kit (Promega-G7573).
(2) 2104 EnVision^{®} plate reader, PerkinElmer.

### Assay program:

### 1. Cell culture

The tumor cell lines were cultured in an incubator at 37 °C, 5% CO₂ or 0% CO₂ according to the above culture conditions. The cells were periodically passaged, and cells in logarithmic growth phase were taken for plating.

### 2. Cell plating

(1). Cells were stained with trypan blue and viable cells were counted.
(2). The cell concentration was adjusted to an appropriate concentration, as shown in Table 9.

**Table 9. Cell lines and densities**

| **Cell line** | **Density (per 96-well)** |
|---|---|
| MDA-MB-231 | 5000 |
| MV4-11 | 6000 |

(3). 90 µL of cell suspension was added to each well of the culture plate, and culture medium without cells was added to the blank control well.
(4). The plate was incubated overnight in an incubator at 37 °C, 5% CO₂ or 0% CO₂, and 100% relative humidity.

### 3. Preparation of a compound storage plate

Preparation of a 400X compound storage plate: The compounds were diluted with DMSO in a gradient from the highest concentration to the lowest concentration. The plates were prepared freshly at each time for use.

### 4. Preparation of 10X compound working solution and treatment of cells with compounds

(1). Preparation of 10X compound working solution: compound or DMSO was added, and the well content was mixed to uniform by aspiration and expelling with pipette. 78 µL of cell culture medium was added to a 96-well plate with a V-shaped bottom. 2 µL of the compound from the 400X compound storage plate was added to the cell culture medium in the 96-well plate. 2 µL of DMSO was added to the vehicle control and the blank control. After adding compound or DMSO, the well content was mixed to uniform by aspiration and expelling with pipette.
(2). Dosing: 10 µL of 10X compound working solution was added to the cell culture plate. 10 µL of DMSO-cell culture mix solution was added to the vehicle control and blank control.
(3). The 96-well cell plate was placed back into the incubator and cultured for 3 days.

### 5. CellTiter-Glo luminescence cell viability detection

The following steps were performed according to the instruction of Promega CellTiter-Glo luminescence cell viability detection kit (Promega-G7573).
(1). The CellTiter-Glo buffer was thawed and allowed to stand to reach room temperature;
(2). CellTiter-Glo substrate was allowed to stand to reach room temperature;
(3). 10 mL of CellTiter-Glo buffer was added to CellTiter-Glo substrate in a bottle to dissolve the substrate to formulate CellTiter-Glo working solution;
(4). The working solution was vortexed slowly for fully dissolution;
(5). The cell culture plates were taken out and allowed to stand for 30 minutes to equilibrate to room temperature;
(6). 50 µL (equal to half the volume of cell culture solution in each well) of Cell Titer-Glo working solution was added into each well. The cell plate was wrapped with aluminum foil to protect the cell plate from light;
(7). The culture plates were shaken on an orbital shaker for 2 minutes to induce cell lysis;
(8). The culture plates were left at room temperature for 10 minutes to stabilize the luminescence signal;
(9). The luminous signal was detected on the 2104 EnVision plate reader.

### 6. Data analysis

The following formula was used to calculate the inhibition rate (IR) of the test compounds: IR (%) = (RLU of the vehicle control - RLU of the compound)/(RLU of the vehicle control - RLU of the blank control)*100%. The inhibition rates of the compounds at different concentrations were calculated in Excel, and then GraphPad Prism software was used to graph the inhibition curves and calculate the relevant parameters, including the minimum inhibition rate, maximum inhibition rate and IC₅₀.

**Test results:** The test results are shown in Table 10.

**Table 10 Inhibitory effect of the compounds of the present disclosure on cell proliferation in MV4-11 and MDA-MB-231 cell lines**

| Compound | MV4-11 | | MDA-MB-231 | |
|---|---|---|---|---|
| | IC₅₀ (nM) | Max. IR (%) | IC₅₀ (nM) | Max. IR (%) |
| **WX001** | 30.5 | 99.95 | 57.2 | 69.41 |
| **WX003** | 6.0 | 100.15 | 52.4 | 65.29 |
| **WX005** | / | / | 4.7 | 80.7 |
| **WX006** | / | / | 4.7 | 80.3 |

| | | | | |
|---|---|---|---|---|
| "/" means not detected. | | | | |

### Conclusion:

The compounds of the present disclosure exhibit excellent inhibitory effects on cell proliferation in tumor cell lines MV4-11 and MDA-MB-231.

### Assay example 4: Evaluation of anti-proliferative effects in tumor cell lines NCI-H1581, DMS114, NCI-H69 and NCI-H526

**Assay purpose:** This assay studied the inhibitory effect of the compounds on cell proliferation by detecting the effect of the test compounds on the in vitro activity of tumor cells **NCI-H1581, DMS114, NCI-H69 and NCI-H526.**

### Assay Materials:

### 1. Cell lines and culture methods

See Table 11 for details.

**Table 11. Cell lines and culture methods**

| Cell line | Tumor type | Growth characteristics | Culture method |
|---|---|---|---|
| NCI-H1581 | Non-small cell lung cancer | Adhering to the wall | RPMI 1640+10%FBS(5% CO₂) |
| DMS114 | Small cell lung cancer | Adhering to the wall | RPMI 1640+10%FBS(5% CO₂) |
| NCI-H69 | Small cell lung cancer | Suspending | RPMI 1640+10%FBS(5% CO₂) |
| NCI-H526 | Small cell lung cancer | Suspending | RPMI 1640+10%FBS(5% CO₂) |

### 2. Culture media and reagents

See Table 12 for details.

**Table 12. Culture media and reagents**

| Culture medium or reagent | Manufacturer | Item No. |
|---|---|---|
| RPMI 1640 | GIBCO | 22400-089 |
| Leibovitz's L-15 | SIGMA | L1518 |
| Dulbecco's PBS | GIBCO | 2160-051 |
| FBS | GIBCO | 10091-148 |
| Antibiotic-antimycotic | GIBCO | 15240-062 |
| 0.25% Trypsin | GIBCO | 25200072 |
| DMSO | SIGMA | D2650 |

### 3. Multi-well plate

Corning Costar 96-well plate, flat bottom white plate (with lid and transparent bottom), # 3599.

### 4. Reagents and instruments used in the cell viability assay

(3) Promega CellTiter-Glo Luminescence Cell Viability Assay Kit (Promega-G7573).
(4) 2104 EnVision^{®} plate reader, PerkinElmer.

### Assay program:

### 1. Cell culture

The tumor cell lines were cultured in an incubator at 37 °C, 5% CO₂ or 0% CO₂ according to the above culture conditions. The cells were periodically passaged, and cells in logarithmic growth phase were taken for plating.

### 2. Cell plating

(1). Cells were stained with trypan blue and viable cells were counted.
(2). The cell concentration was adjusted to an appropriate concentration, as shown in Table 13.

**Table 13. Cell lines and densities**

| **Cell line** | **Density (per 96-well)** |
|---|---|
| NCI-H1581 | 12000 |
| DMS114 | 5000 |
| NCI-H69 | 20000 |
| NCI-H526 | 10000 |

(3). 100 µL of cell suspension was added to each well of the culture plate, and culture medium without cells was added to the blank control well.
(4). The plate was incubated overnight in an incubator at 37 °C, 5% CO₂ or 0% CO₂, and 100% relative humidity.

### 3. Preparation of compound stock solution

Preparation of 1000 X compound stock solution: Compound was dissolved in DMSO to 10 mM, then a portion was taken out and diluted to 1000 times the highest acting concentration. The diluted stock solution was dispensed for later use.

4. Preparation of 5X compound working solution and treatment of cells with compounds. The working solutions were prepared freshly at each time for use.
(1). Preparation of 5X compound working solution: 119.4 µL of cell culture medium was added to a 96-well plate with a V-shaped bottom. 96 µL of DMSO-cell culture medium mix solution (with DMSO content of 0.5%) was added to subsequent gradient wells. 0.6 µL of the compound from the 1000X compound stock solution was added to the cell culture medium in the 96-well plate, and mixed to uniform with a pipette. A pipette was used to draw 24 µL of the compound-cell culture medium mixture into subsequent wells, and the mixture was mixed well by a pipette each time.
(2). Dosing: 25 µL of 5X compound working solution was added to the cell culture plate. 25 µL of DMSO-cell culture medium mix solution (with DMSO content of 0.5%) was added to the vehicle control and blank control.
(3). The 96-well cell plate was placed back into the incubator and cultured for 3 days.

### 5. CellTiter-Glo luminescence cell viability detection

The following steps were performed according to the instruction of Promega CellTiter-Glo luminescence cell viability detection kit (Promega-G7573).
(1). The CellTiter-Glo buffer was thawed and allowed to stand to reach room temperature;
(2). CellTiter-Glo substrate was allowed to stand to reach room temperature;
(3). 10 mL of CellTiter-Glo buffer was added to CellTiter-Glo substrate in a bottle to dissolve the substrate to formulate CellTiter-Glo working solution;
(4). The working solution was vortexed slowly for fully dissolution;
(5). The cell culture plates were taken out and allowed to stand for 30 minutes to equilibrate to room temperature;
(6). 50 µL (equal to half the volume of cell culture solution in each well) of Cell Titer-Glo working solution was added into each well. The cell plate was wrapped with aluminum foil to protect the cell plate from light;
(7). The culture plates were shaken on an orbital shaker for 2 minutes to induce cell lysis;
(8). The culture plates were left at room temperature for 10 minutes to stabilize the luminescence signal;
(9). The luminous signal was detected on the 2104 EnVision plate reader.

### 6. Data analysis

The following formula was used to calculate the inhibition rate (IR) of the test compounds: IR (%) = (RLU of the vehicle control - RLU of the compound)/(RLU of the vehicle control - RLU of the blank control)* 100%. The inhibition rates of the compounds at different concentrations were calculated in Excel, and then GraphPad Prism software was used to graph the inhibition curves and calculate the relevant parameters, including the minimum inhibition rate, maximum inhibition rate and IC₅₀.

**Test results:** The test results are shown in Table 14.

**Table 14 Inhibitory effect of the compounds of the present disclosure on cell proliferation in NCI-H1581, DMS114, NCI-H69 and NCI-H526 cell lines**

| Compound | NCI-H1581 | | DMS114 | | NCI-H69 | | NCI-H526 | |
|---|---|---|---|---|---|---|---|---|
| | IC₅₀ (nM) | Max. IR (%) | IC₅₀ (nM) | Max. IR (%) | IC₅₀ (nM) | Max. IR (%) | IC₅₀ (nM) | Max. IR (%) |
| **WX005** | 5.2 | 92.19 | 6.1 | 89.45 | 6.2 | 68.41 | 0.5 | 99.17 |
| **WX013** | 1.3 | 88.62 | 2.1 | 87.56 | 1.3 | 70.53 | 0.1 | 99.37 |
| **WX015** | 5.0 | 91.87 | 5.6 | 90.22 | 6.9 | 67.66 | 0.5 | 99.44 |
| **WX016** | 5.9 | 92.76 | 5.8 | 91.27 | 6.5 | 69.31 | 0.1 | 99.15 |

### Conclusion:

The compounds of the present disclosure exhibit excellent inhibitory effects on cell proliferation in tumor cell lines NCI-H1581, DMS114, NCI-H69 and NCI-H526.

### Assay example 5: Evaluation of pharmacokinetic of compounds in mice

### Assay purpose:

In this study, C57BL/6N male mice were selected as the test animals, and the LC/MS/MS method was used to quantitatively determine the drug concentration in the plasma of mice intravenously or orally administered the test compounds, so as to evaluate the pharmacokinetic profile of the test compounds in mice.

### Assay Materials:

C57BL/6N mice (male, 20-30g, 7-10 weeks old, Beijing Vital River).

### Procedure:

A clear solution or suspension of the test compound was intravenously injected into C57BL/6N mice (fasted overnight) via tail or given to C57BL/6N mice (fasted overnight) by gavage. For intravenous injection administration, blood was collected from the cheek at 0 h (before administration) and 0.083, 0.25, 0.5, 1, 2, 4, 6, 8, and 24 h after administration. Each sample was collected about 0.05 mL. Heparin sodium was added to anti-coagulate. Plasma samples were collected and placed on wet ice, and centrifuged within 1 hour to separate the plasma (centrifugation conditions: 6000g, 3 minutes, 2-8°C). Blood samples were stored in a - 80°C refrigerator before analysis; For oral gavage administration, blood was collected from the cheek at 0 h (before administration) and 0.083, 0.25, 0.5, 1, 2, 4, 6, 8, 24 h after administration. Each sample was collected about 0.05 mL. Heparin sodium was added to anti-coagulate. Plasma samples were collected and placed on wet ice, and centrifuged within 1 hour to separate the plasma (centrifugation conditions: 6000g, 3 minutes, 2-8°C). Blood samples were stored in a -80°C refrigerator before analysis. Through blood drug concentration data at different time points, Phoenix WinNonlin8.2.0 was used to calculate pharmacokinetic parameters, providing parameters such as AUC₀₋ₜ, AUC_{0-∞}, MRT_{0-∞}, Cₘₐₓ, Tₘₐₓ, and T_{1/2}, etc. and their mean and standard deviation.

The test results are shown in Table 15.

**Table 15 Pharmacokinetic parameters of the compounds of the present disclosure in mice**

| Pharmacok inetic parameters in mice / dosage | Intravenous injection | | | | Administration by gavage | | | |
|---|---|---|---|---|---|---|---|---|
| | Plasma clearance (mL/min/k g) | Elimi natio n half-life (h) | Appare nt volume of distribu tion (L/kg) | Area under the plasma concentr ation-time curve (0-inf,µM.h ) | Peak conce ntrati on (µM) | Tim e to peak (h) | Area under the plasma concentr ation-time curve (0-inf,µM.h ) | Bioav ailabil ity F (%) |
| **WX005** (IV_1mg/k g; PO_10mg/ kg) | 2.3 | 2.71 | 0.35 | 14.34 | 23.27 | 1.00 | 108.89 | 75.9 % |
| **WX006** (IV_1mg/k g; PO_10mg/ kg) | 15.8 | 3.62 | 2.3 | 1.90 | 1.76 | 0.50 | 2.71 | 14.2 % |
| **WX012** (IV_2mg/k g; PO_10mg/ kg) | 9.5 | 1.85 | 0.41 | 6.45 | 0.69 | 1.08 | 1.99 | 6.18 % |
| **WX013** (IV_2mg/k g; PO_10mg/ kg) | 1.84 | 5.11 | 0.44 | 37.03 | 3.06 | 2.67 | 40.90 | 22.09 % |

**Conclusion:** The oral plasma systemic exposures (AUC_{0-inf}) of the compounds of the present disclosure are high. In rodents, mice, the pharmacokinetic properties of the compounds of the present disclosure are superior.

### Assay example 6: Evaluation of pharmacokinetic of the compound in rats

### Assay purpose:

In this study, SD male rats were selected as the test animals, and the LC/MS/MS method was used to quantitatively determine the drug concentration at different time points in the plasma of rats intravenously or orally administered the test compound, so as to evaluate the pharmacokinetic profile of the test compound in rats.

### Assay Materials:

Sprague Dawley (SD) rats (male, 200-300g, 7-10 weeks old, Beijing Vital River).

### Procedure:

A clear solution or suspension of the test compound was intravenously injected into SD rats (fasted overnight) via tail or given to SD rats (fasted overnight) by gavage. For intravenous injection administration, blood was collected from the jugular vein at 0 h (before administration) and 0.083, 0.25, 0.5, 1, 2, 4, 6, 8, and 24 h after administration. Each sample was collected about 0.25 mL. Heparin sodium was added to anti-coagulate. Blood samples were collected and placed on wet ice, and centrifuged within 1 hour to separate the plasma (centrifugation conditions: 6000g, 3 minutes, 2-8°C). Plasma samples were stored in a -80°C refrigerator before analysis; For oral gavage administration, blood was collected from the jugular vein at 0 h (before administration) and 0.083, 0.25, 0.5, 1, 2, 4, 6, 8, and 24 h after administration. Each sample was collected about 0.25 mL. Heparin sodium was added to anti-coagulate. Blood samples were collected and placed on wet ice, and centrifuged within 1 hour to separate the plasma (centrifugation conditions: 6000g, 3 minutes, 2-8°C). Plasma samples were stored in a -80°C refrigerator before analysis. Through blood drug concentration data at different time points, Phoenix WinNonlin8.2.0 was used to calculate pharmacokinetic parameters, providing parameters such as AUC₀₋ₜ, AUC_{0-∞}, MRT_{0-∞}, Cₘₐₓ, Tₘₐₓ, and T_{1/2}, etc. and their mean and standard deviation.

The test results are shown in Table 16.

**Table 16 Pharmacokinetic parameters of the compound of the present disclosure in rats**

| Pharmaco kinetic parameter s in rats | Intravenous injection (2 mg/kg) | | | | Administration by gavage (10 mg/kg) | | | |
|---|---|---|---|---|---|---|---|---|
| | Plasma clearan ce (mL/mi n/kg) | Elimin ation half-life (h) | Appar ent volum e of distrib ution (L/kg) | Area under the plasma concentr ation-time curve (0-inf,µM.h ) | Peak concentr ation (µM) | Ti me to pe ak (h) | Area under the plasma concentr ation-time curve (0-inf,µM.h ) | Bioavail ability F (%) |
| **WX005** | 0.145 | 11.1 | 0.121 | 464.10 | 14.4 | 5.3 3 | 264.34 | 11.39 |

**Conclusion:** The oral plasma systemic exposure (AUC_{0-inf}) of the compound of the present disclosure is high. In rodents, rats, the pharmacokinetic properties of the compound of the present disclosure are superior.

### Assay example 7: In vivo pharmacodynamic study of compounds in subcutaneous xenograft tumor Balb/c nude mouse model of human triple-negative breast cancer MDA-MB-231 cells

Cell culture: Human triple-negative breast cancer MDA-MB-231 cells (ATCC-HTB-26) were cultured in monolayer in vitro. The culture conditions were RPMI-1640 medium with 10% fetal bovine serum in an incubator at 37° C and 5% CO₂. Conventional digestion with trypsin-EDTA was performed once every two days for passage. When the cell saturation was 80%-90% and the number met the requirement, the cells were collected, counted, and inoculated.

Assay animals: Balb/c nude mice, female, 6 weeks old upon arrival, purchased from Shanghai Bikai Experimental Animal Co., Ltd.

### Assay program:

Cell inoculation: 0.2 mL (5×10⁶ cells) of MDA-MB-231 cells (with matrigel, v/v 1:1) were subcutaneously inoculated into the right back of each mouse. The grouping for administration was started when the average tumor volume reached 110 mm³. One dosing cycle was seven days, with administration once a day and an interval of 24 hours. The test compound was administered orally for a total of four cycles. The dosages of test compound WX005 were 10 mg/kg, 30 mg/kg and 100 mg/kg respectively, and the dosage of WX013 was 30 mg/kg respectively. Tumor diameter was measured twice a week with a vernier caliper. Tumor volume was calculated by the following formula: V = 0.5a × b², where a and b are the long and short diameters of the tumor, respectively. The anti-tumor efficacy of compounds was evaluated by TGI (%) or relative tumor proliferation rate T/C (%). Relative tumor proliferation rate T/C (%) = T_{RTV} / C_{RTV} × 100% (T_{RTV}: average RTV of the treatment group; C_{RTV}: average RTV of the negative control group). The relative tumor volume (RTV) was calculated based on the results of tumor measurement by the calculation formula: RTV = Vₜ/ V₀, where V₀ is the tumor volume measured at grouping for administration (i.e., D0), and Vₜ is the tumor volume at a certain measurement, and T_{RTV} and C_{RTV} are taken on the same day.

### Test results:

The test results are shown in Table 17.

**Table 17 Test results of the compounds of the present disclosure in the Balb/c nude mouse model of subcutaneous xenograft tumor of human triple-negative breast cancer MDA-MB-231 cells**

| Group | Dosage | Tumor volume (mm³) (Day 0) | Tumor volume (mm³) (Day 27) | Relative tumor proliferation rate T/C (%) (Day 27) | TGI (%) (Day 27) |
|---|---|---|---|---|---|
| Vehicle control | 0 mg/kg | 107.54 | 833.65 | 805.28 | / |
| **WX005** | 10 mg/kg | 108.29 | 0 | 0 | 114.91 |
| **WX013** | 30 mg/kg | 108.69 | 0 | 0 | 114.97 |

Vehicle: 1%DMSO/20%PEG400/0.2%Tween80/78.8%H₂O.

TGI: Tumor Growth Inhibition. TGI (%) = [1 - (Average tumor volume at the end of administration in a certain treatment group - Average tumor volume at the administration in this treatment group)/(Average tumor volume at the end of treatment in the vehicle control group - Average tumor volume at the beginning of treatment in the vehicle control group)] ×100%.

### Conclusion:

The in vivo efficacy of the compounds **WX005** and **WX013** of the present disclosure on the xenograft tumor model of human triple-negative breast cancer MDA-MB-231 exhibit significant tumor shrinkage effect.

## Claims

1. A compound represented by formula (II) or a pharmaceutically acceptable salt thereof, 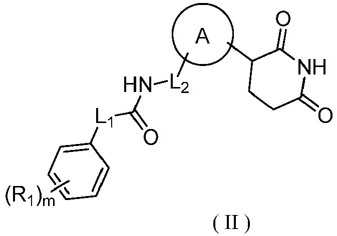 wherein
Ring A is selected from benzofuranyl, benzoisoxazolyl, benzotriazolyl, naphthofuranyl, naphthoisoxazolyl and naphthotriazolyl;
L₁ is selected from a bond, -C(Rₐ)(R_{b})-, -N(R_{c})- and -OCH₂-;
L₂ is selected from -CH₂-, -C₁₋₆ alkyl-C(=O)NH- and -C₁₋₃ alkyl-O-;
each R₁ is independently selected from F, Cl, Br, I, C₁₋₃ alkyl, C₁₋₃ alkoxy, 5-membered heteroaryl and 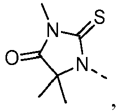 wherein the C₁₋₃ alkyl, C₁₋₃ alkoxy, 5-membered heteroaryl and 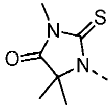 are optionally substituted by 1, 2 or 3 R_{d};
m is selected from 0, 1, 2 and 3;
Rₐ and R_{b} are each independently selected from R, F, Cl, Br and I;
R_{c} is selected from H and CH₃;
each R_{d} is independently selected from F, Cl, Br, I, OCH₃, N(CH₃)₂ and morpholinyl.

2. The compound according to claim 1 or a pharmaceutically acceptable salt thereof, wherein structural moiety 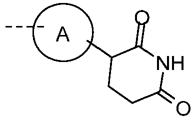 is selected from 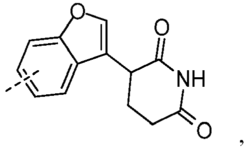 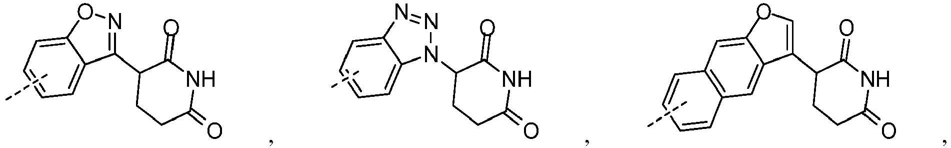 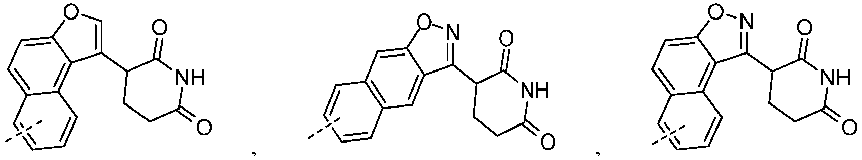 and

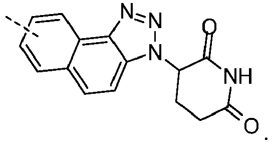

3. The compound according to claim 2 or a pharmaceutically acceptable salt thereof, wherein structural moiety 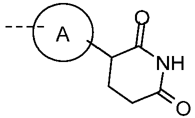 is selected from

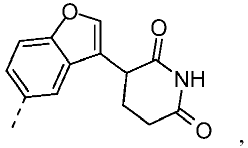

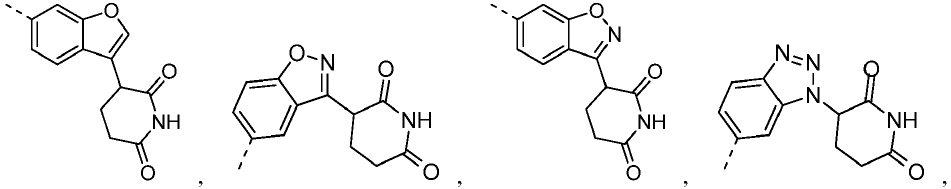

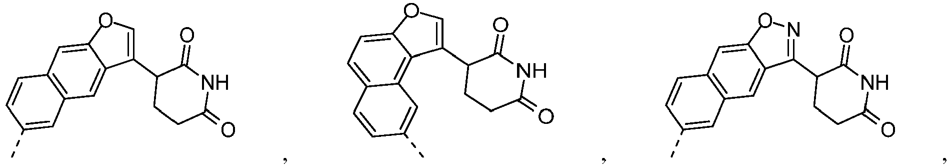

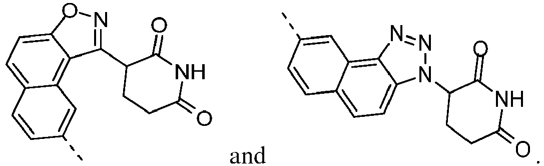

4. The compound according to claim 1 or a pharmaceutically acceptable salt thereof, wherein Ring A is selected from benzofuranyl and benzoisoxazolyl.

5. The compound according to claim 4 or a pharmaceutically acceptable salt thereof, wherein Ring A is selected from

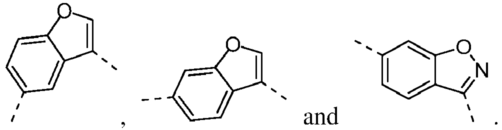

6. The compound according to any one of claims 1 to 3 or a pharmaceutically acceptable salt thereof, wherein structural moiety 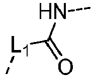 is selected from 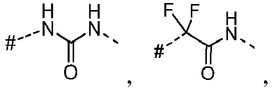 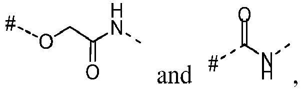 and the # end is connected to the phenyl group of formula (II).

7. The compound according to any one of claims 1 to 3 or a pharmaceutically acceptable salt thereof, wherein L₂ is selected from -CH₂-, -(CH₂)₆-C(=O)NH- and -CH₂CH₂O-.

8. The compound according to any one of claims 1 to 3 or a pharmaceutically acceptable salt thereof, wherein each R₁ is independently selected from F, Cl, Br, I, CH₃, OCH₃, OCH₂CH₃, thiazolyl and 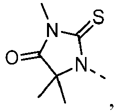 wherein the CH₃, OCH₃, OCH₂CH₃, thiazolyl and 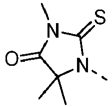 are optionally substituted by 1, 2 or 3 R_{d}.

9. The compound according to claim 8 or a pharmaceutically acceptable salt thereof, wherein each R₁ is independently selected from F, Cl, CH₃, -OCH₂CH₂OCH₃, -OCH₂CH₂N(CH₃)₂,

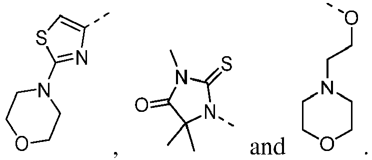

10. The compound according to any one of claims 1 to 3 or a pharmaceutically acceptable salt thereof, wherein structural moiety 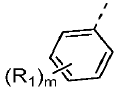 is selected from

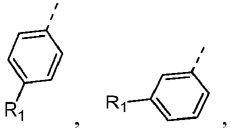

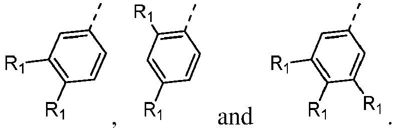

11. The compound according to any one of claims 1 to 3 or a pharmaceutically acceptable salt thereof, wherein structural moiety 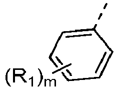 is selected from

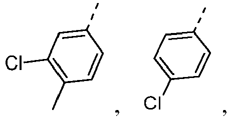

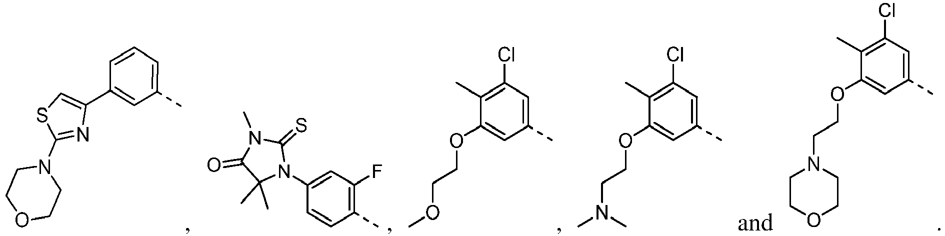

12. The compound according to any one of claims 1 to 11 or a pharmaceutically acceptable salt thereof, wherein the compound is selected from 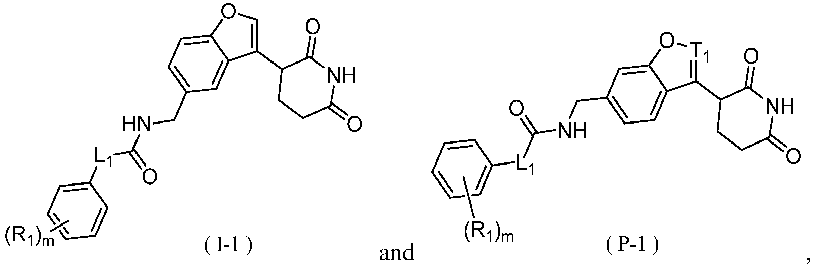 wherein
T₁ is selected from CH and N;
R₁, Li and m are as defined in any one of claims 1 to 11.

13. A compound, which is selected from 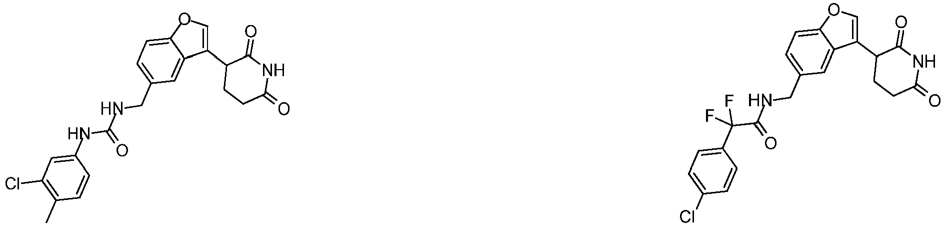 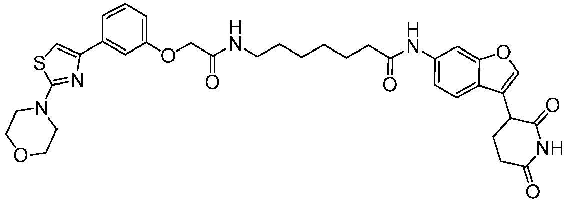 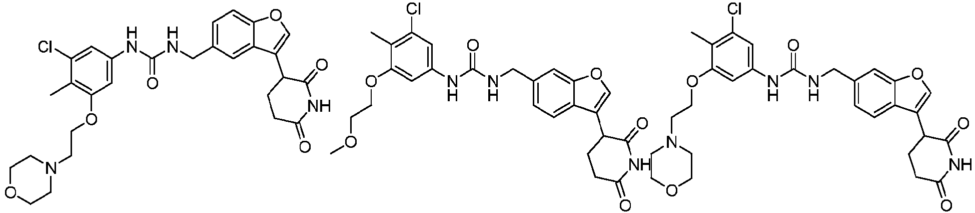 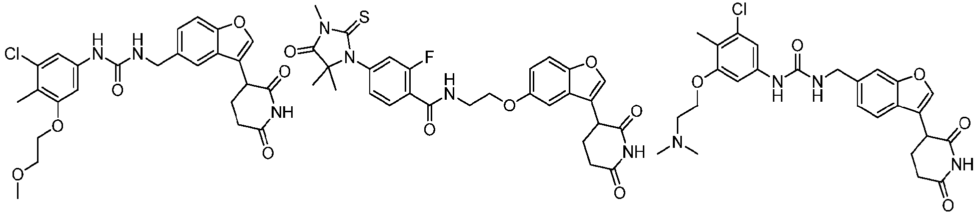 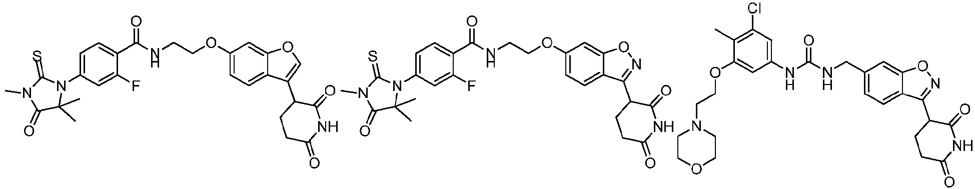 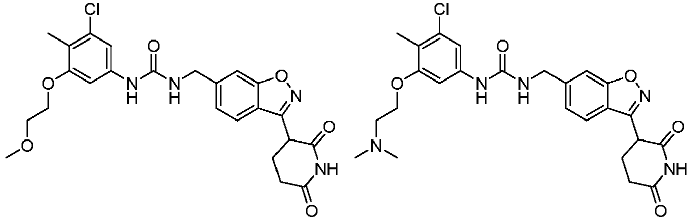 or a pharmaceutically acceptable salt thereof.

14. The compound according to claim 13 or a pharmaceutically acceptable salt thereof, wherein the compound is selected from
